(19) **Europäisches Patentamt / European Patent Office / Office européen des brevets**

(11) **EP 2 364 370 B1**

(12) **EUROPEAN PATENT SPECIFICATION**

(45) Date of publication and mention
of the grant of the patent:
**03.09.2014 Bulletin 2014/36**

(51) Int Cl.:
*C12Q 1/68* (2006.01)    *G01N 33/68* (2006.01)

(21) Application number: **09828325.2**

(22) Date of filing: **21.11.2009**

(86) International application number:
**PCT/US2009/065419**

(87) International publication number:
**WO 2010/059996 (27.05.2010 Gazette 2010/21)**

(54) **METHODS FOR PROGNOSIS OF ACUTE RENAL FAILURE**

VERFAHREN ZUR PROGNOSE VON AKUTER NIERENINSUFFIZIENZ

PROCÉDÉS POUR LE PRONOSTIC D'UNE INSUFFISANCE RÉNALE ACUTE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.11.2008  US 117137 P**
**22.11.2008  US 117159 P**
**22.11.2008  US 117142 P**
**22.11.2008  US 117164 P**
**22.11.2008  US 117174 P**
**22.11.2008  US 117170 P**
**22.11.2008  US 117166 P**
**22.11.2008  US 117171 P**
**22.11.2008  US 117165 P**
**22.11.2008  US 117147 P**

(43) Date of publication of application:
**14.09.2011  Bulletin 2011/37**

(73) Proprietor: **Astute Medical, Inc.**
**San Diego, CA 92121 (US)**

(72) Inventors:
• **ANDERBERG, Joseph**
**Encinitas**
**CA 92024 (US)**
• **GRAY, Jeff**
**Solana Beach**
**CA 92075 (US)**
• **McPHERSON, Paul**
**Encinitas**
**CA 92024 (US)**
• **NAKAMURA, Kevin**
**Cardiff by the Sea**
**CA 92007 (US)**

(74) Representative: **Schiweck, Weinzierl & Koch**
**European Patent Attorneys**
**Landsberger Straße 98**
**80339 München (DE)**

(56) References cited:
**WO-A2-2009/038742    US-A1- 2008 090 304**
**US-A1- 2008 153 092    US-A1- 2008 254 485**

• ABD EL LATIF M M S ET AL: "Urinary epidermal growth factor excretion: A useful prognostic marker for progression of renal damage in children", JOURNAL OF MEDICAL SCIENCES, ANSINET, vol. 7, no. 7, 1 October 2007 (2007-10-01) , pages 1171-1176, XP008148391, ISSN: 1682-4474, DOI: 10.3923/JMS. 2007.1171.1176

• SCHENA F P ET AL: "EGF and MCP-1 urinary excretion is a suitable prognostic marker in IgA nephropathy", JOURNAL OF THE AMERICAN SOCIETY OF NEPHROLOGY; MEETING OF THE AMERICAN SOCIETY OF NEPHROLOGY, WILLIAMS AND WILKINS, BALTIMORE, MD, US; PHILADELPHIA, PA, USA, vol. 13, no. Program and Abstracts Issue, 1 September 2002 (2002-09-01), page 458A, XP008148304, ISSN: 1046-6673

• TORRES D D ET AL: "The ratio of epidermal growth factor to monocyte chemotactic peptide-1 in the urine predicts renal prognosis in IgA nephropathy", KIDNEY INTERNATIONAL, NATURE PUBLISHING GROUP, LONDON, GB, vol. 73, no. 3, 1 February 2008 (2008-02-01), pages 327-333, XP008148303, ISSN: 0085-2538, DOI: 10.1038/SJ.KI.5002621 [retrieved on 2007-10-17]

- **KILEY S C ET AL: "Urinary biomarkers: The future looks promising", KIDNEY INTERNATIONAL, NATURE PUBLISHING GROUP, LONDON, GB, vol. 76, no. 2, 2 July 2009 (2009-07-02), pages 133-134, XP008148302, ISSN: 0085-2538, DOI: 10.1038/KI.2009.124**

**Description**

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830. Renal disease and/or injury may be acute or chronic. Acute and chronic kidney disease are described as follows (from Current Medical Diagnosis & Treatment 2008, 47th Ed, McGraw Hill, New York, pages 785-815): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222;

| Type | Risk Factors |
| --- | --- |
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; |
| **Type** | **Risk Factors** |
| | Immune-complex: Lupus glomerulonephritis, postinfectious glomerulonephritis, cryoglobulinemic glomerulonephritis |
| Acute tubulointerstitial nephritis | Drug reaction (eg, β-lactams, NSAIDs, sulfonamides, ciprofloxacin, thiazide diuretics, furosemide, phenytoin, allopurinol, pyelonephritis, papillary necrosis |

(continued)

| Type | Risk Factors |
|------|-------------|
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, proposes the following classifications for stratifying AKI patients:

"Risk": serun creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease—the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, each hereby incorporated by reference in its entirety, the RIFLE criteria provide a uniform definition of AKI which has been validated in numerous studies.

More recently, Mehta et al., Crit. Care 11:R31 (doi:10.1186.cc5713), 2007, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006;7(4):177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

US2008/0090304 discloses predictive markers for AKI but EGF is not mentioned as predictive marker.

Abd El Latif M M S (Journal of Medical Sciences, Vol. 7, No. 7, p. 1171-1176) disclose EGF as marker in patients that have already developed acute renal failure.

## Summary of the invention

The invention in its broadest scope is defined by independent claims 1 and 7:

1. A method for evaluating renal status in a subject, comprising:

performing one or more assays configured to detect a kidney injury marker on a body fluid sample obtained from the subject to provide one or more assay results, the kidney injury marker being Epidermal growth factor and optionally further Complement C3, Interleukin-4, Interleukin-1 alpha, Tubulointerstitial nephritis antigen, Transforming growth factor beta-i, Bone morphogenetic protein 7, Osteopontin, Netrin1, and Growth-regulated alpha protein; and

correlating the assay result(s) to one or more of risk stratification, staging, prognosis, classifying and monitoring of the renal status of the subject, wherein said correlating step comprises assigning a likelihood of future acute renal failure (ARF) suffered by the subject and optionally one or more future changes in renal status to the subject based on the assay result(s).

7. Use of a kidney injury markers for prognosis of future acute renal failure in vitro, wherein the kidney injury markers is Epidermal growth factor, and optionally further Complement C3, Interleukin-4, Interleukin-1 alpha, Tubulointerstitial nephritis antigen, Transforming growth factor beta-i, Bone morphogenetic protein 7, Osteopontin, Netrin-1, and Growth-regulated alpha protein.

Acute renal failure is also called acute kidney injury.

These kidney injury markers comprising at least EGF may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

The methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject.

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example a measured concentration of one or more markers selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

Disclosed herein are also embodiments where the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example a measured concentration of one or more markers selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein is/are correlated to a particular class and/or subclass.

Disclosed are furthermore classification embodiments where these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the $75^{th}$, $85^{th}$, $90^{th}$, $95^{th}$, or $99^{th}$ percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, *etc.*), by selecting a concentration representing the $75^{th}$, $85^{th}$, $90^{th}$, $95^{th}$, or $99^{th}$ percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present specification also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising: antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc.*) as well as molecules that may be indirectly detected by production of a detectable reaction product (*e.g.,* enzymes such as horseradish peroxidase, alkaline phosphatase, *etc.*) or through the use of a specific binding molecule which itself may be detectable (*e.g.,* a labeled antibody that binds to the second antibody, biotin, digoxigenin, maltose, oligohistidine, 2,4-dintrobenzene, phenylarsenate, ssDNA, dsDNA, *etc.*).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g.,* a diffraction grating, electrochemical sensor, etc) for generation of a signal, while in others, a signal is generated by a transducer that is spatially separate from the solid phase antibody (*e.g.,* a fluorometer that employs an excitation light source and an optical detector). This list is not meant to be limiting. Antibody-based biosensors may also be employed to determine the presence or amount of analytes that optionally eliminate the need for a labeled molecule.

BRIEF DESCRIPTION OF THE FIGURES

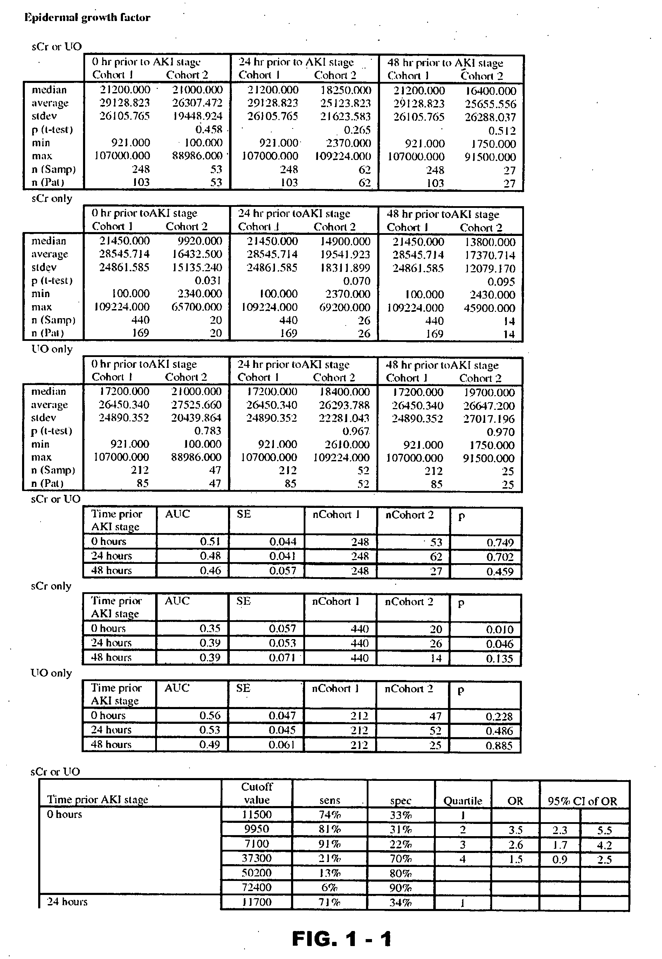

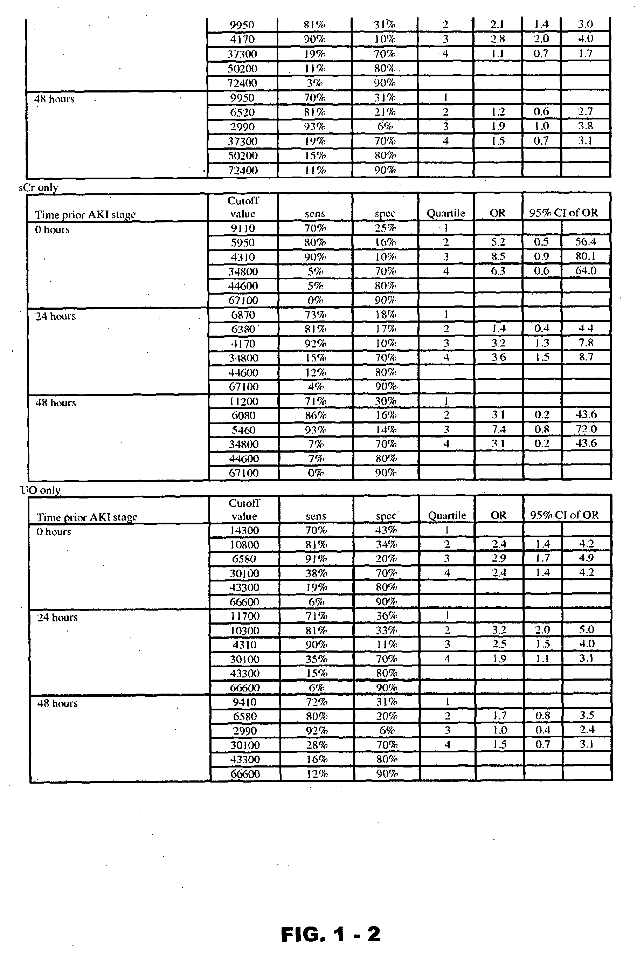

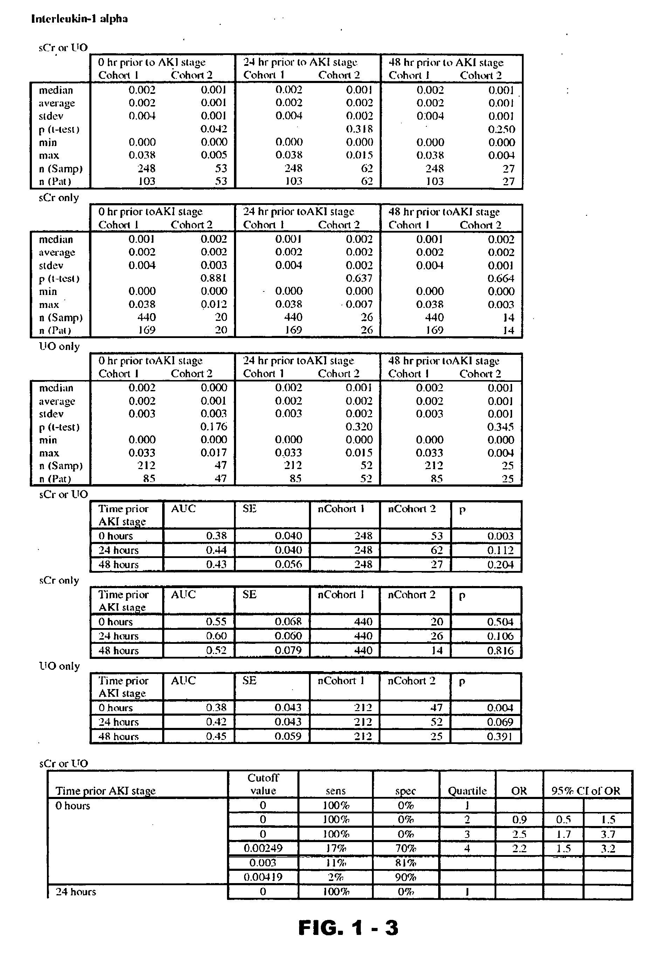

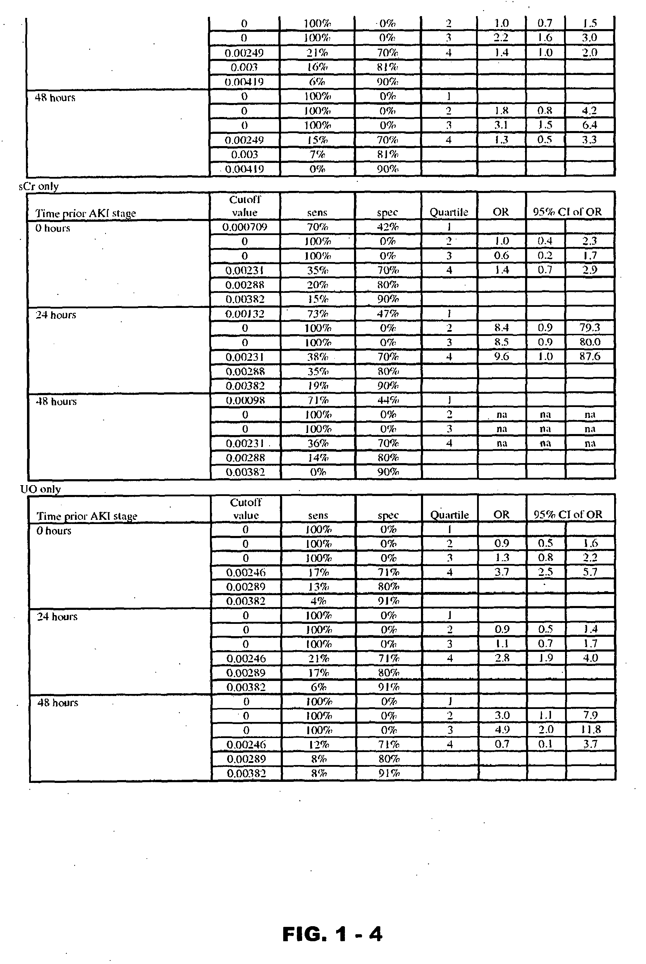

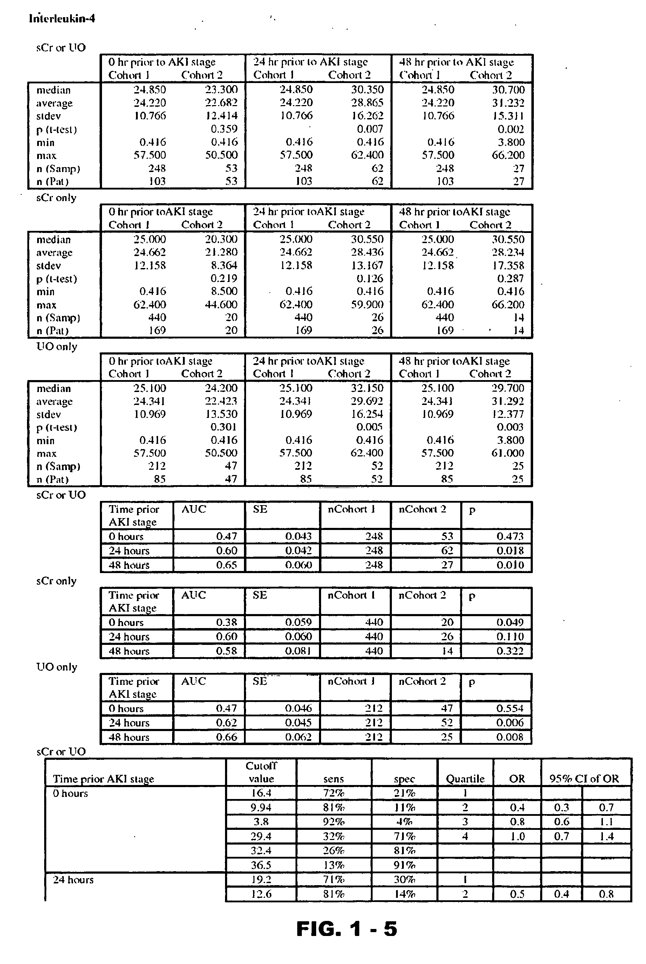

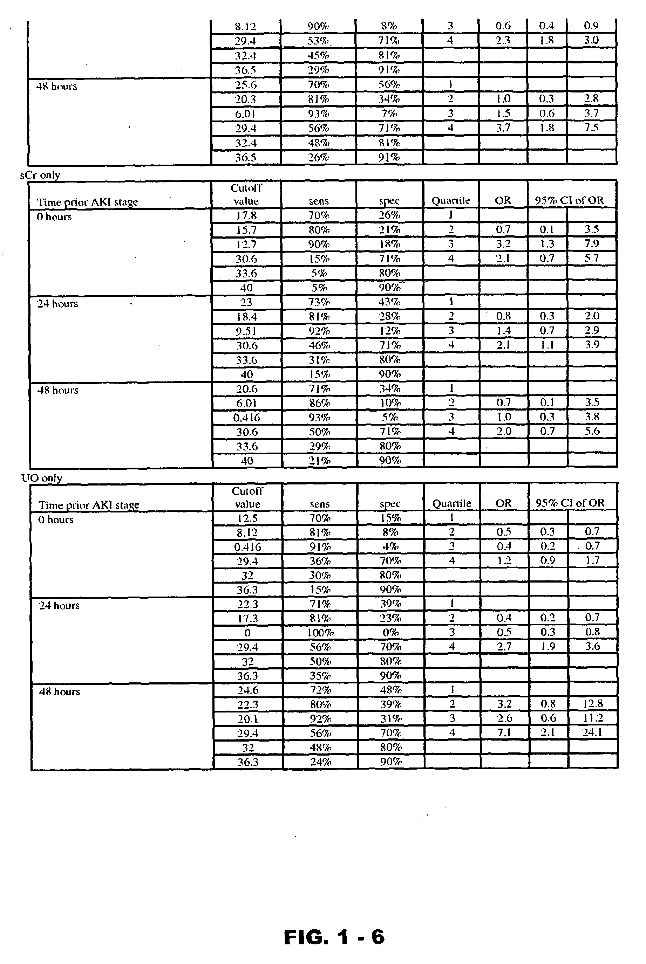

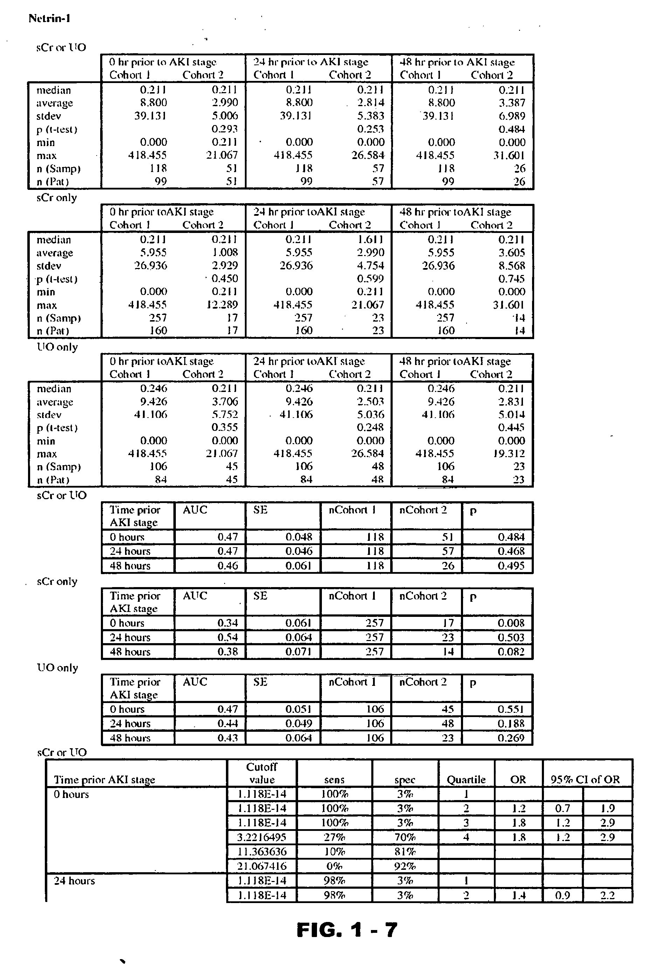

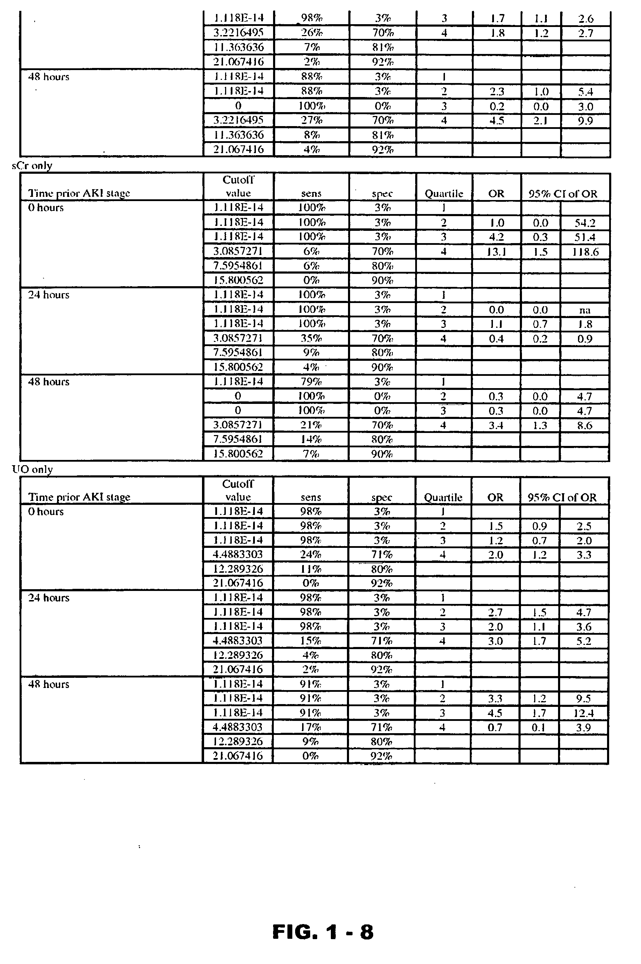

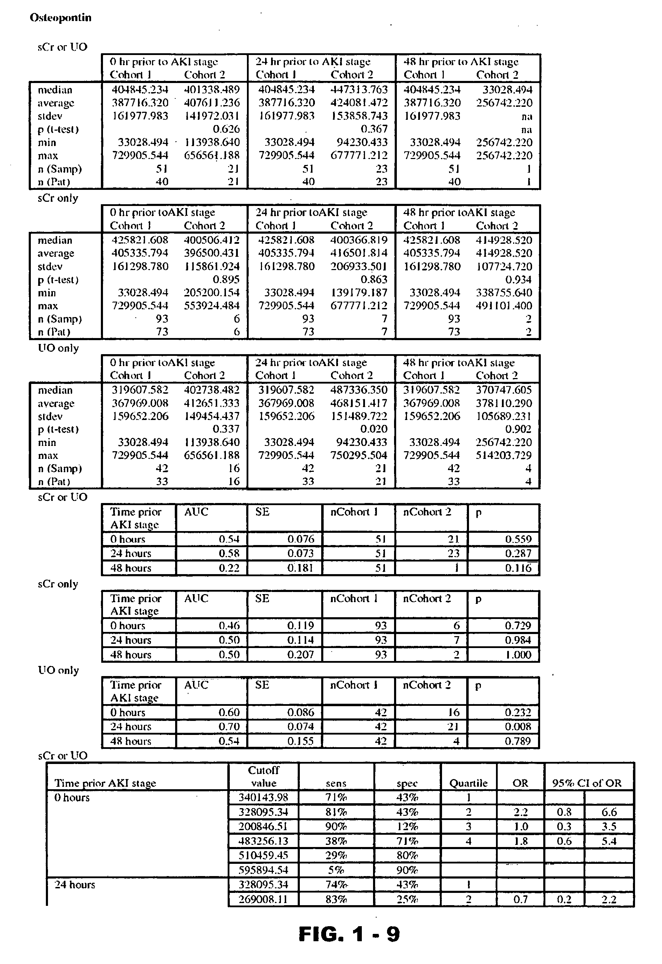

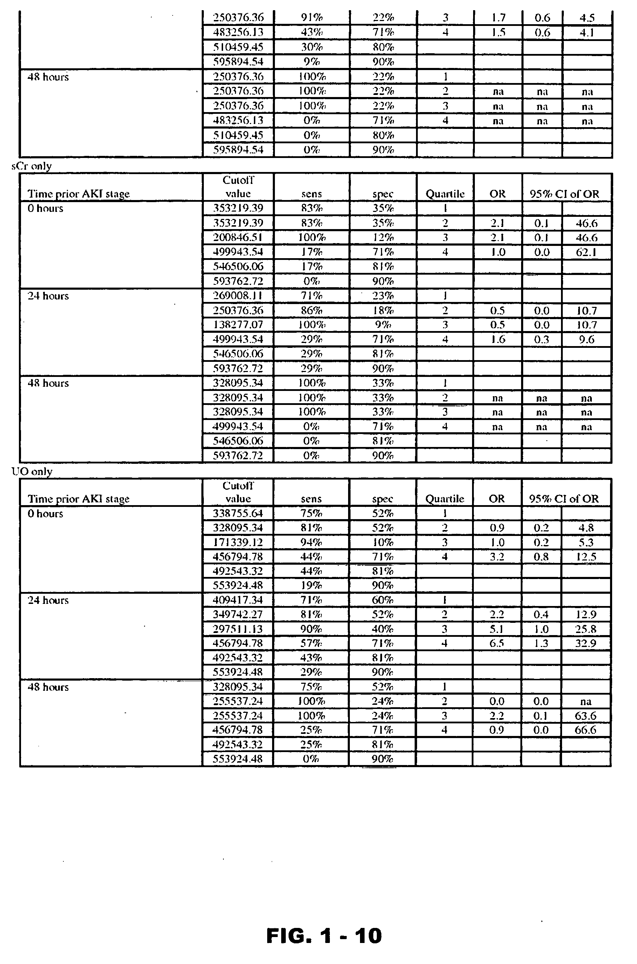

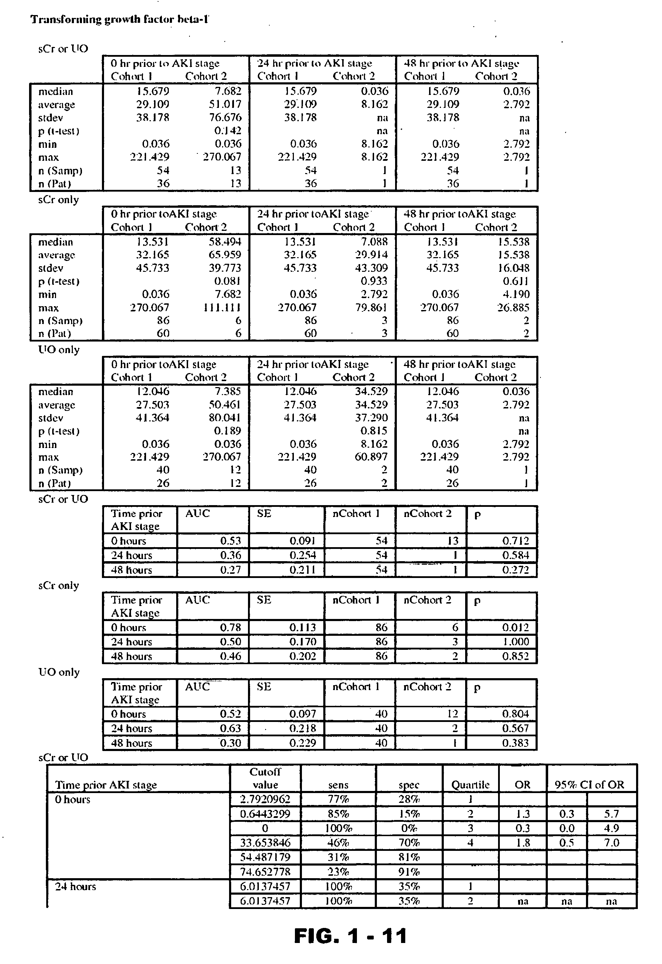

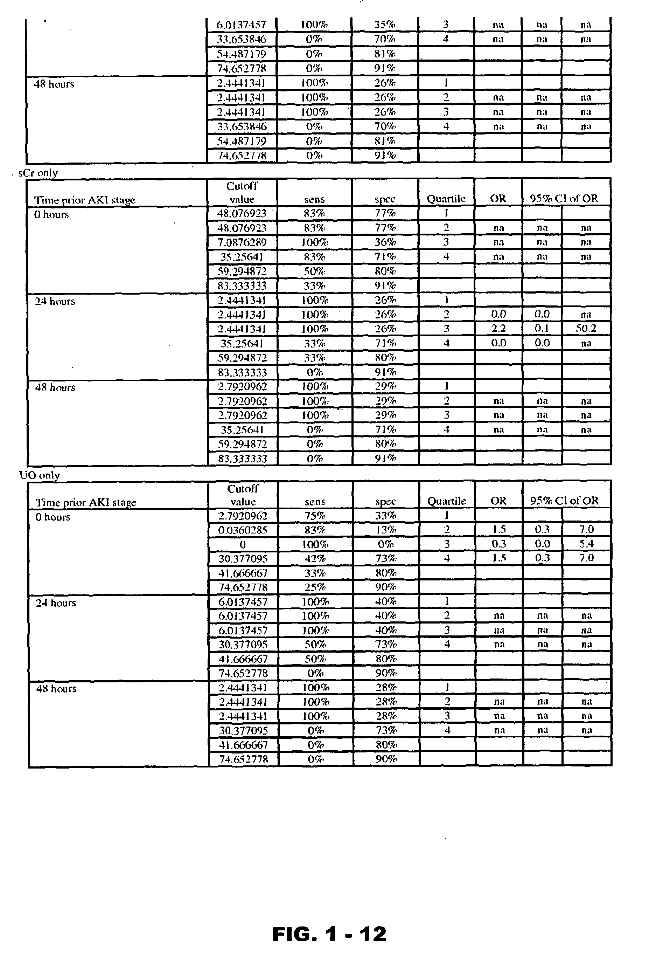

Fig. 1 provides data tables determined in accordance with Example 6 for the comparison of marker levels in urine samples collected for Cohort 1 (patients that did not progress beyond RIFLE stage 0) and in urine samples collected from subjects at 0, 24 hours, and 48 hours prior to reaching stage R, I or F in Cohort 2. Tables provide descriptive statistics, AUC analysis, and sensitivity, specificity and odds ratio calculations at various threshold (cutoff) levels for the various markers.

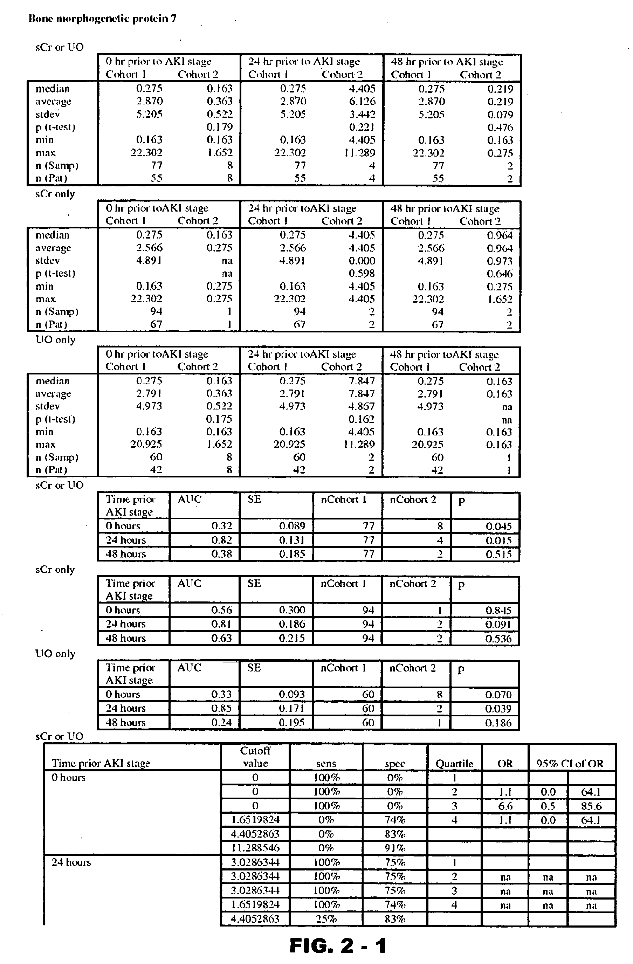

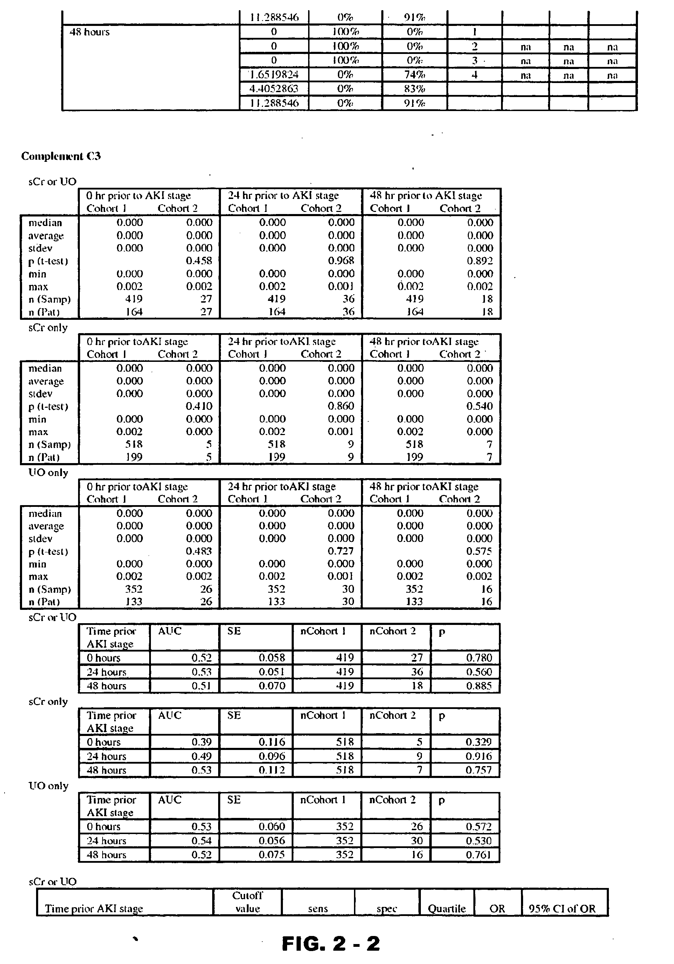

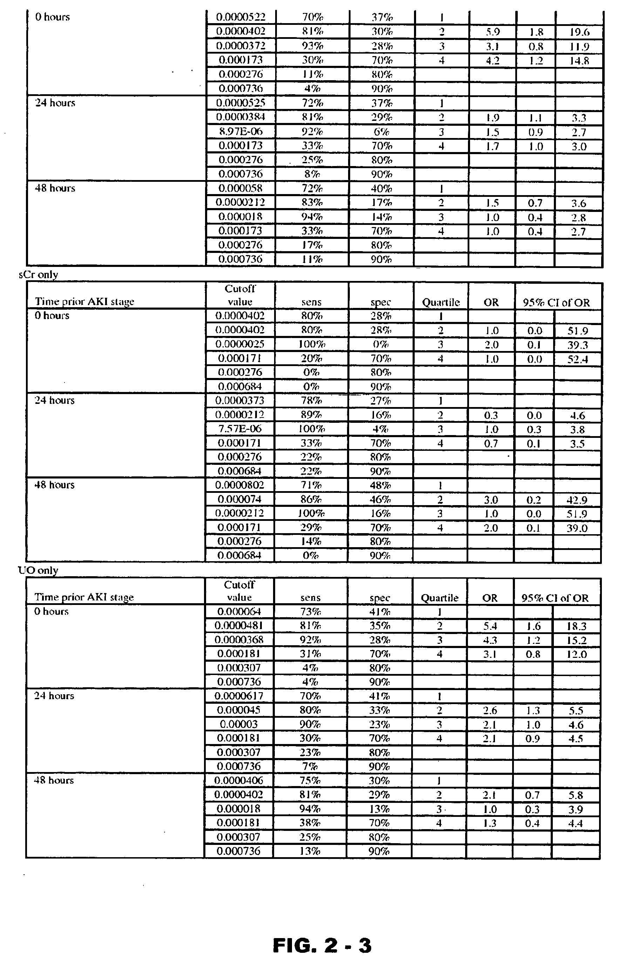

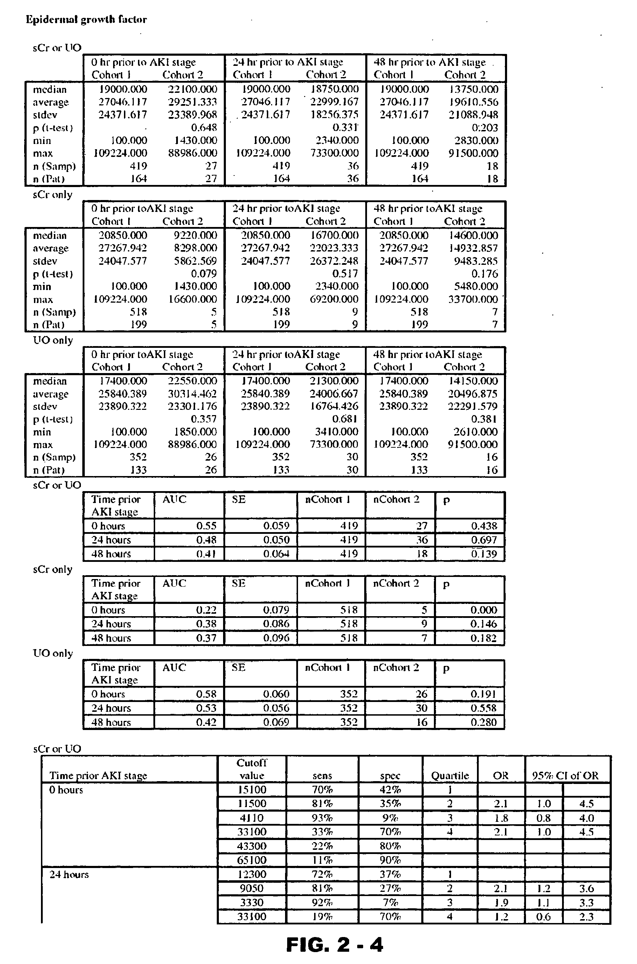

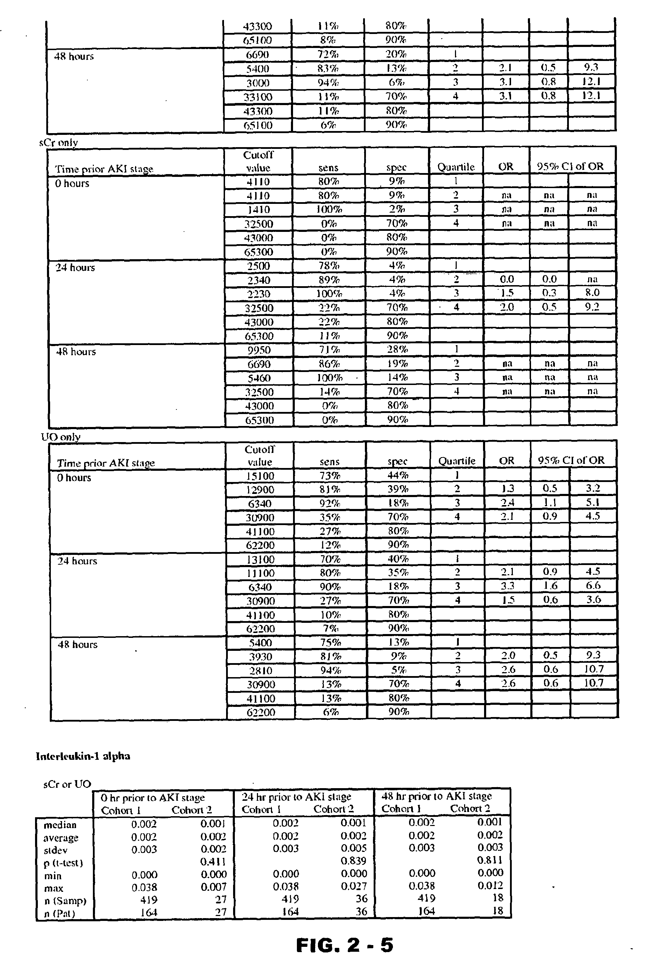

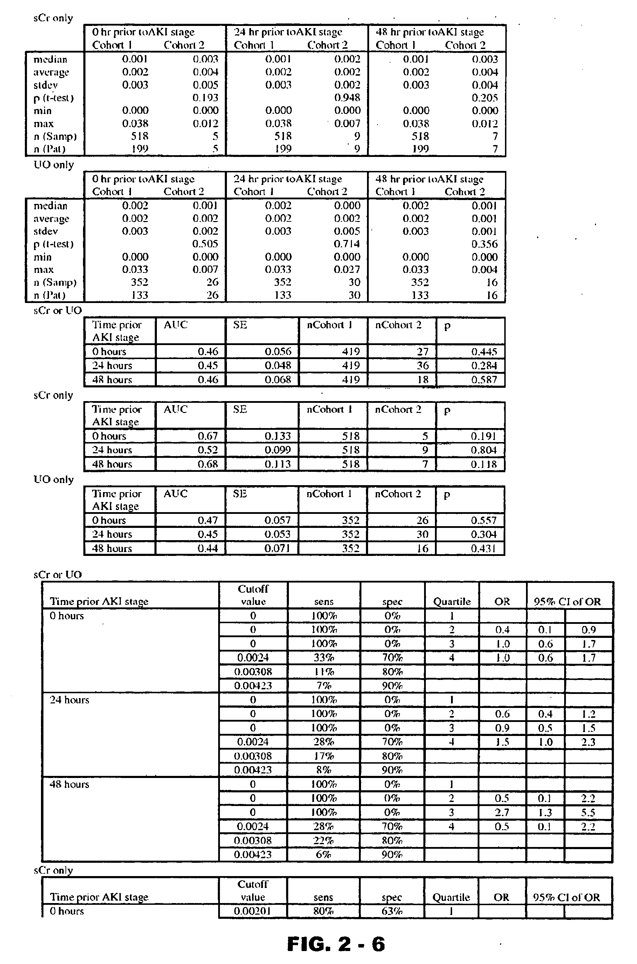

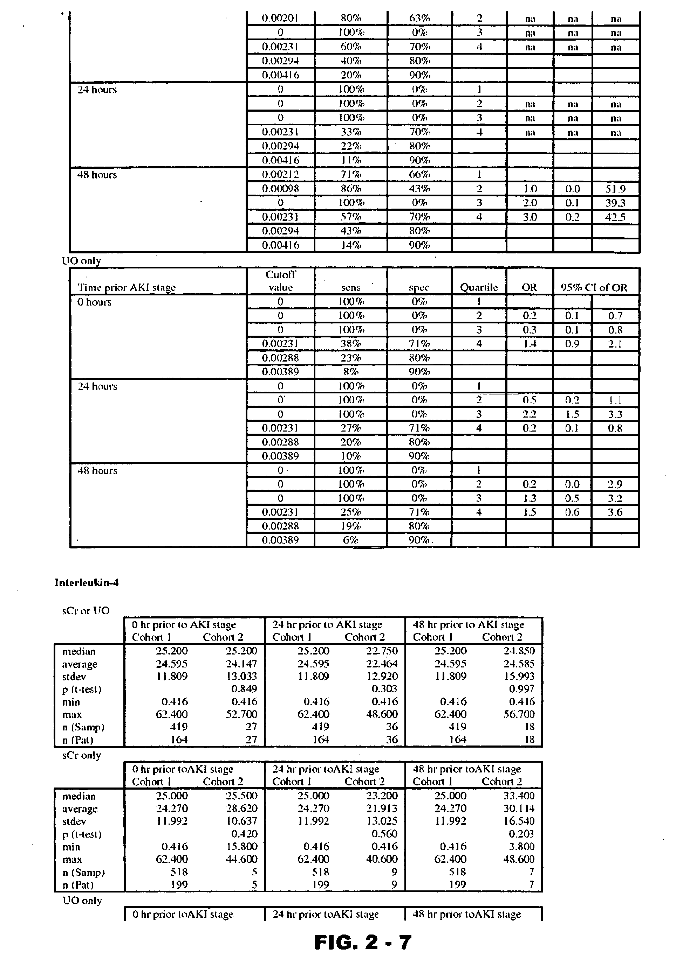

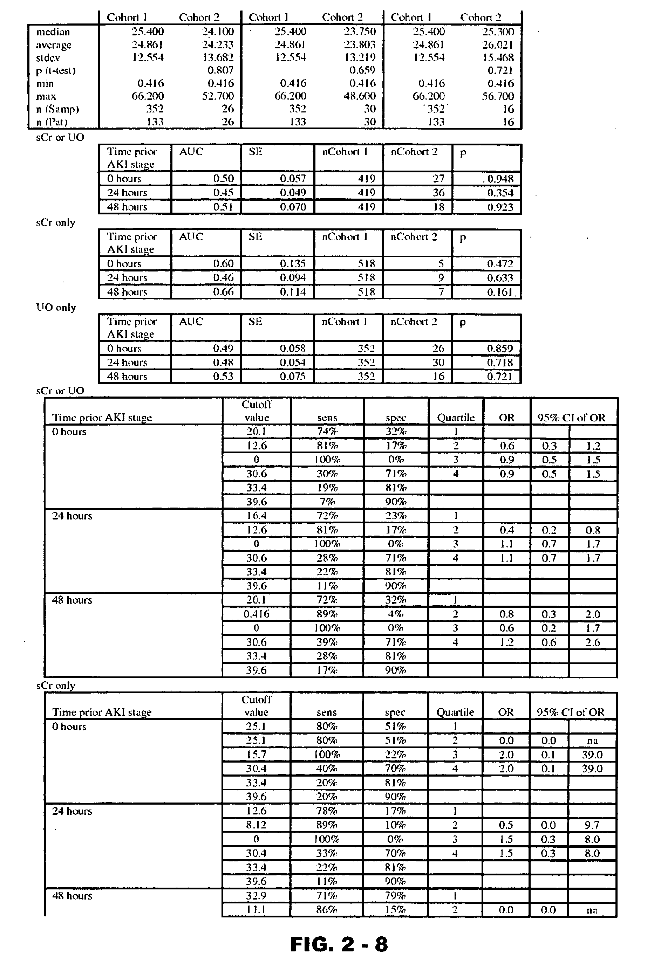

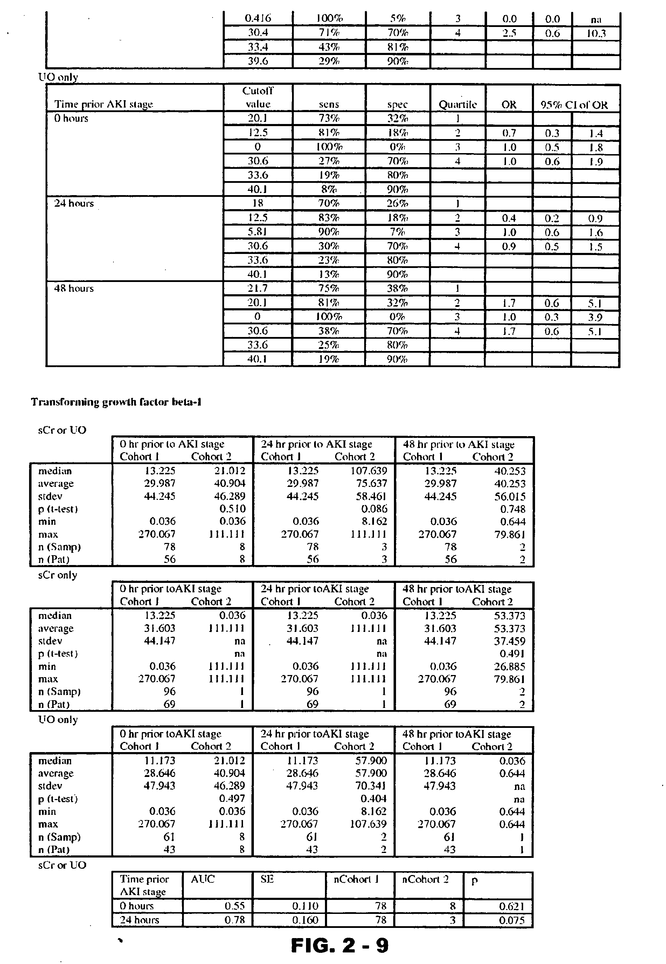

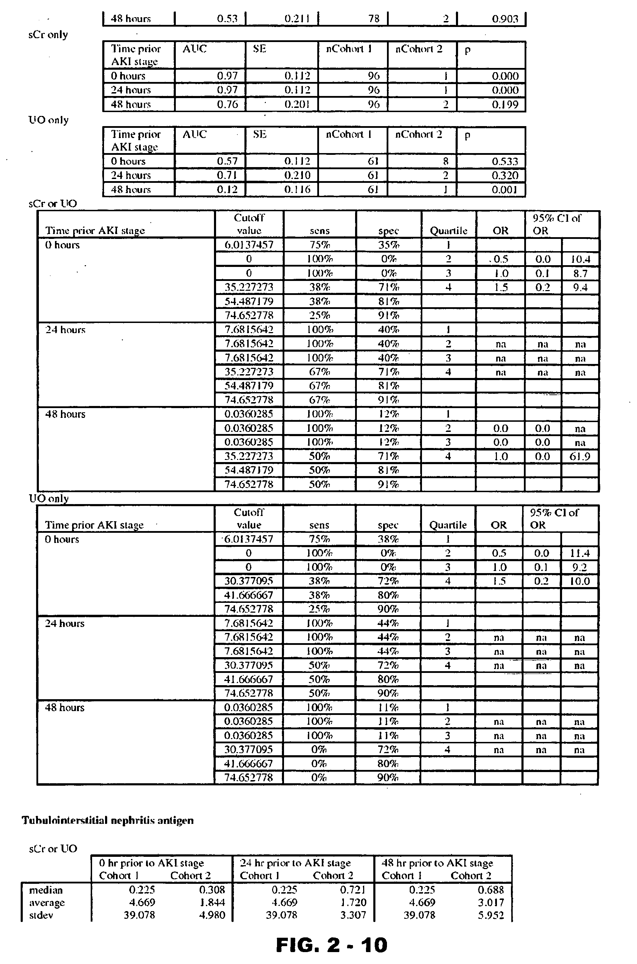

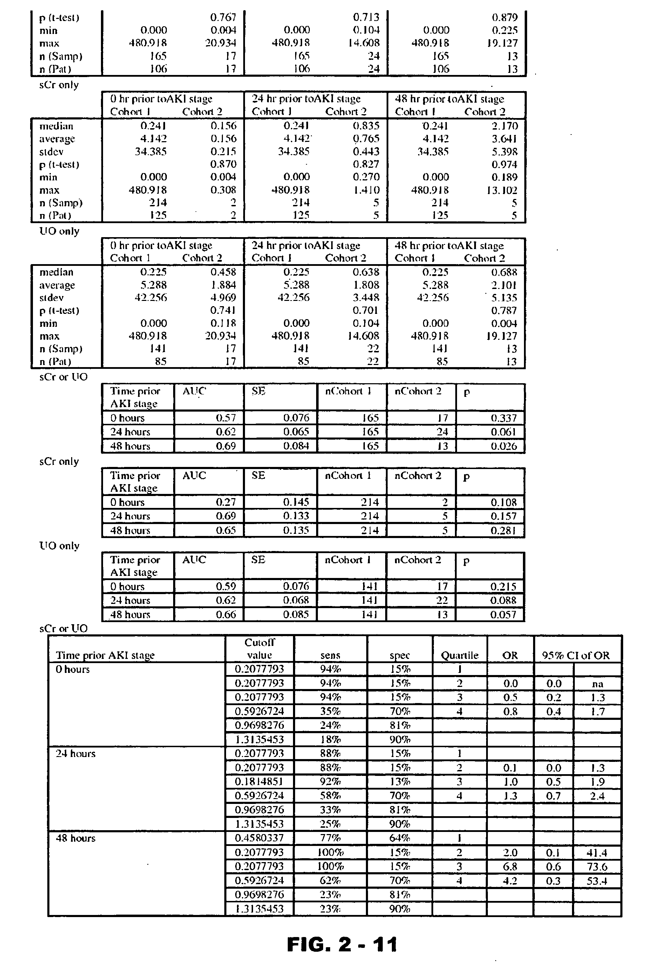

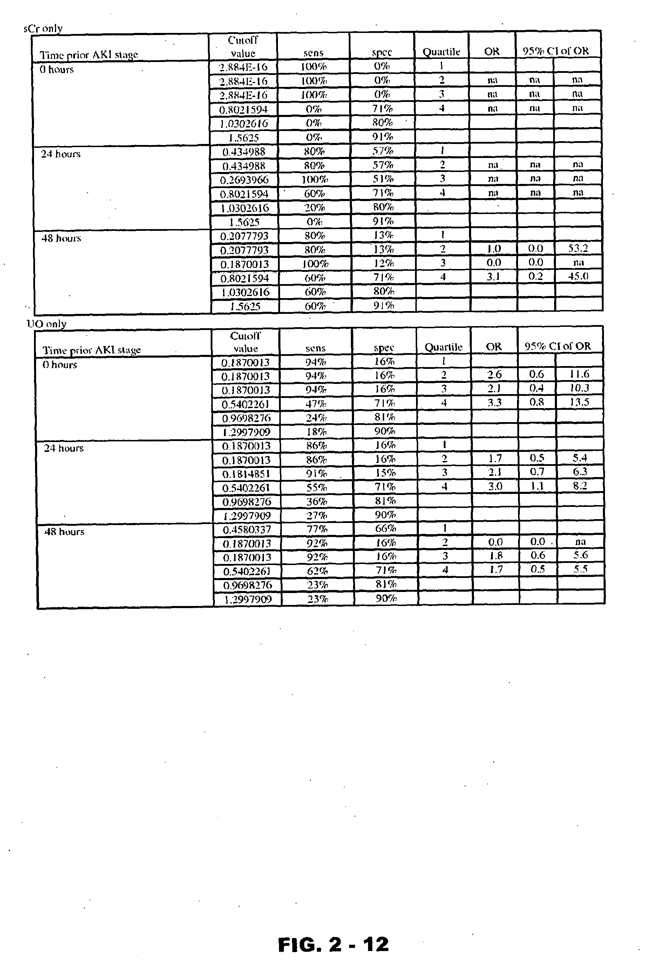

Fig. 2 provides data tables determined in accordance with Example 7 for the comparison of marker levels in urine samples collected for Cohort 1 (patients that did not progress beyond RIFLE stage 0 or R) and in urine samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

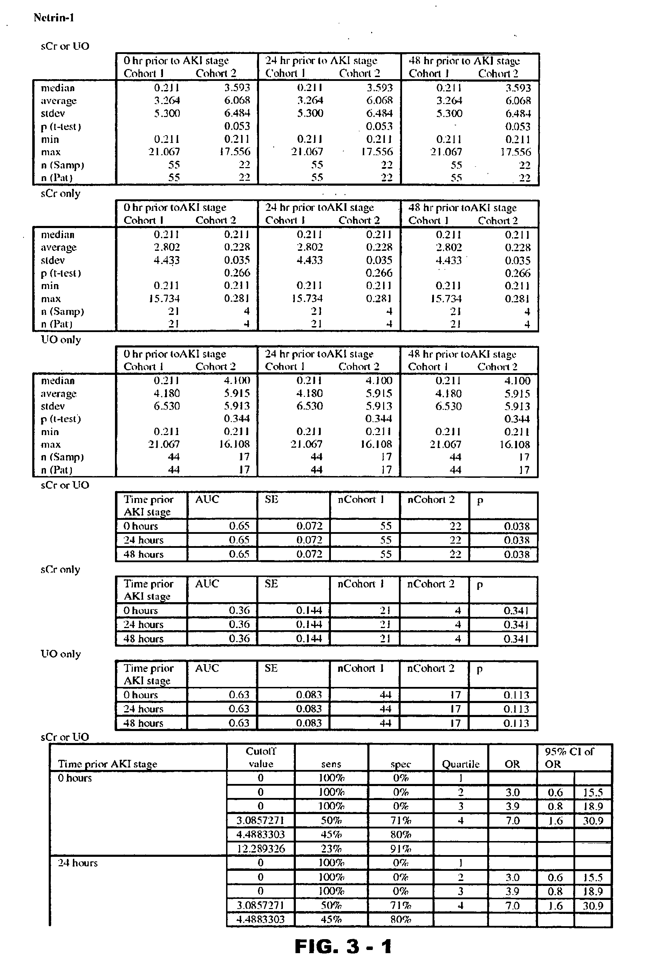

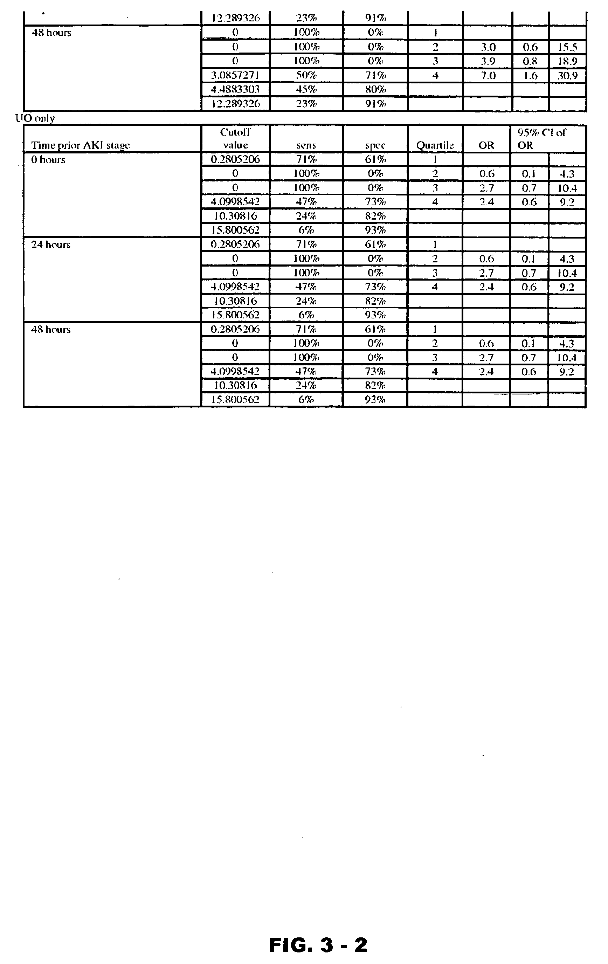

Fig. 3 provides data tables determined in accordance with Example 8 for the comparison of marker levels in urine samples collected for Cohort 1 (patients that reached, but did not progress beyond, RIFLE stage R) and in urine samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

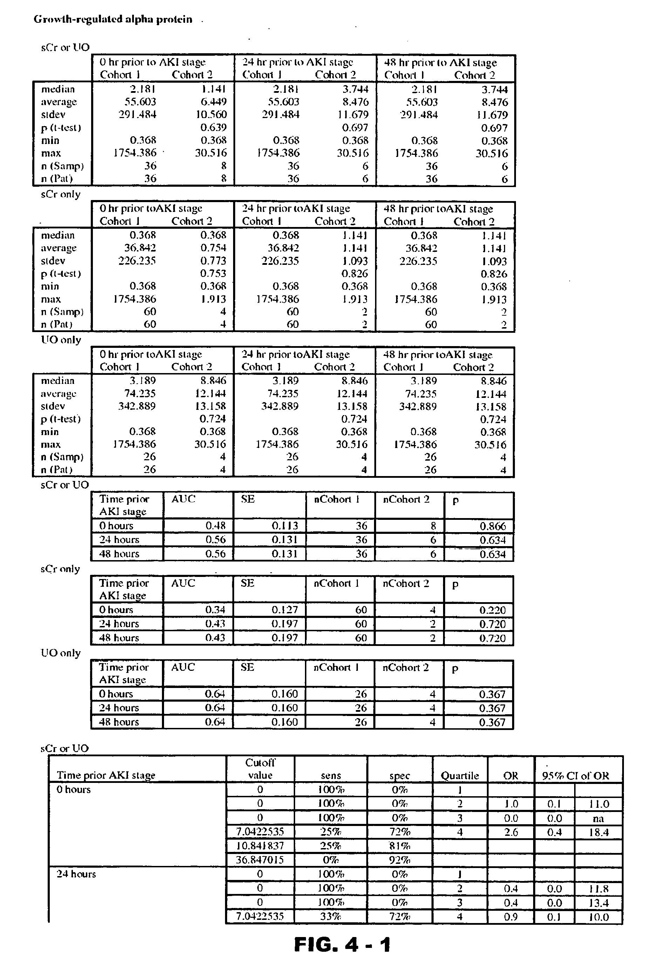

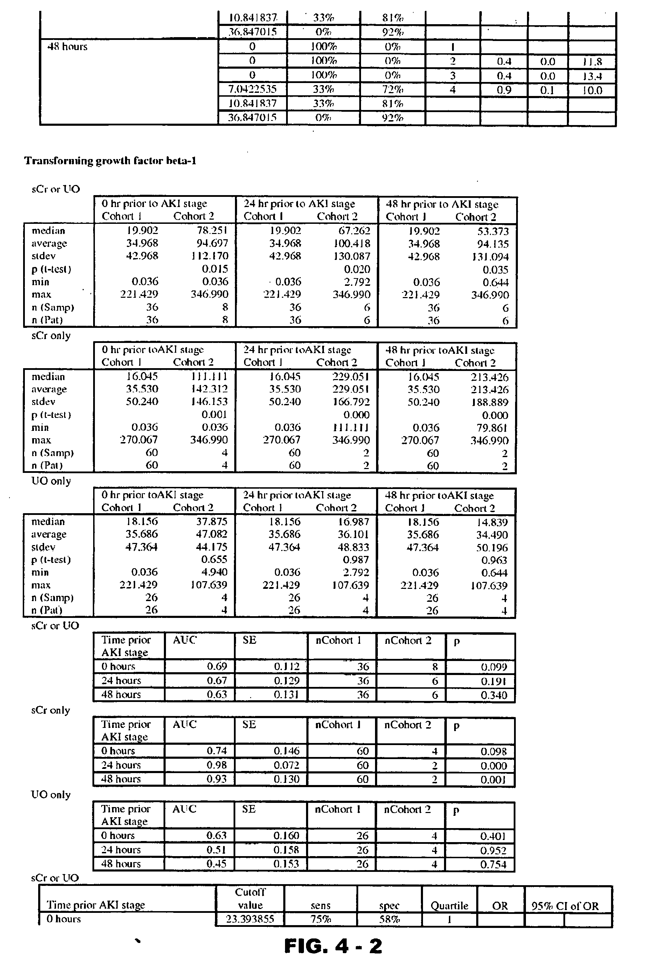

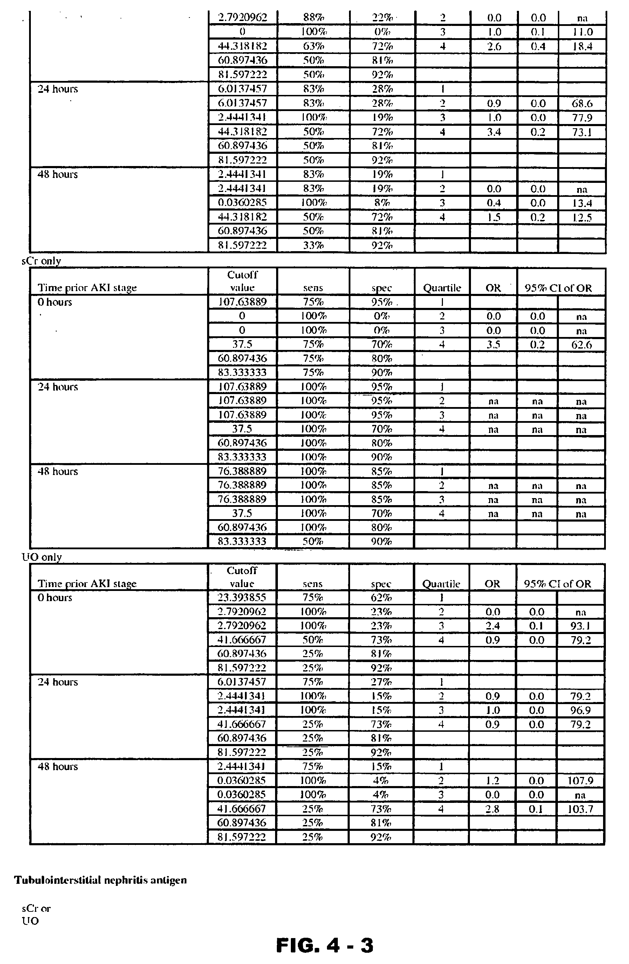

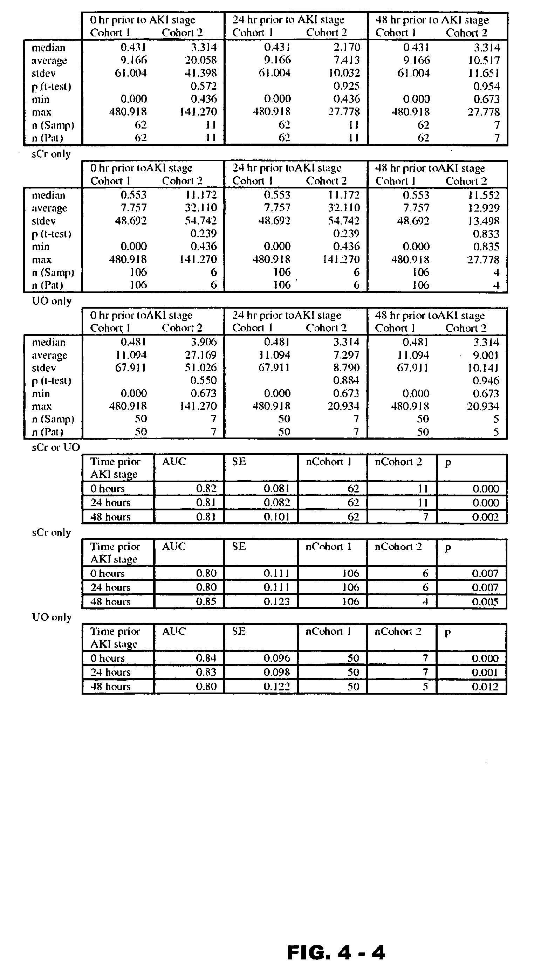

Fig. 4 provides data tables determined in accordance with Example 9 for the comparison of marker levels in urine samples collected for Cohort 1 (patients that did not progress beyond RIFLE stage 0) and in urine samples collected from subjects at 0, 24 hours, and 48 hours prior to reaching stage F in Cohort 2. Tables provide descriptive statistics, AUC analysis, and sensitivity, specificity and odds ratio calculations at various threshold (cutoff) levels for the various markers.

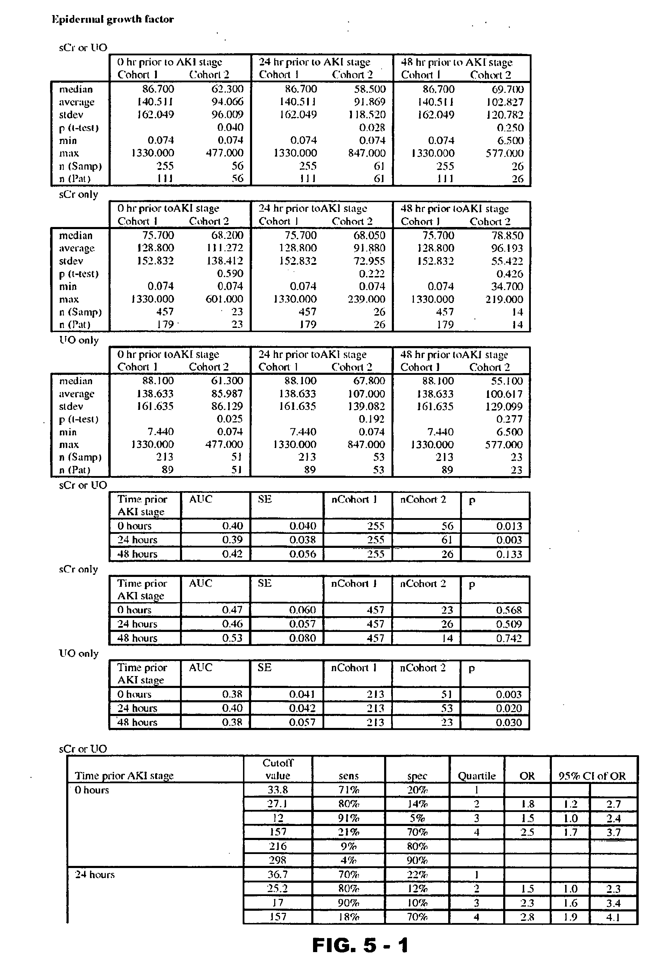

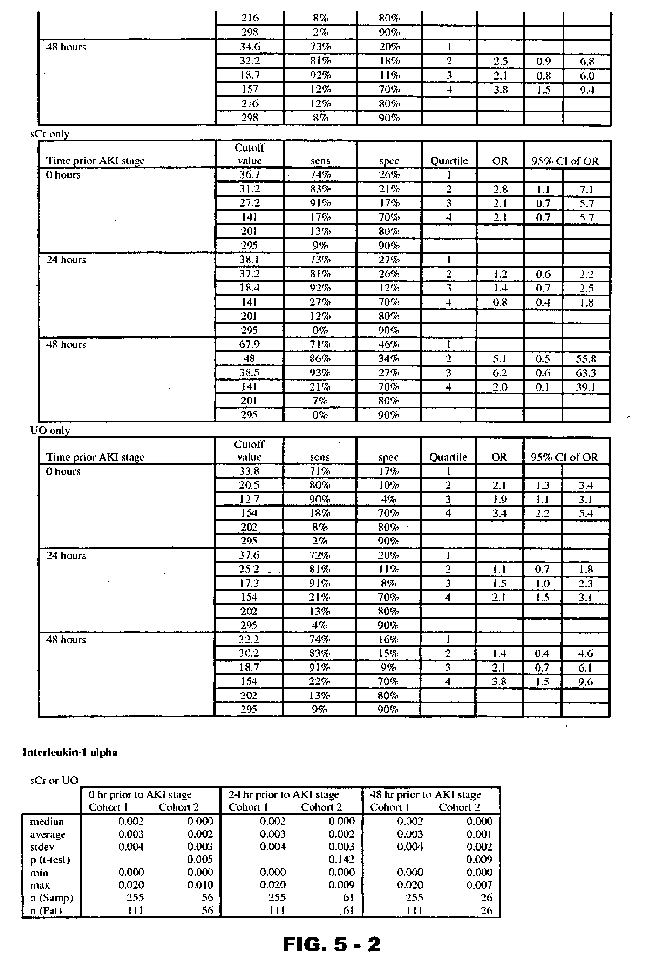

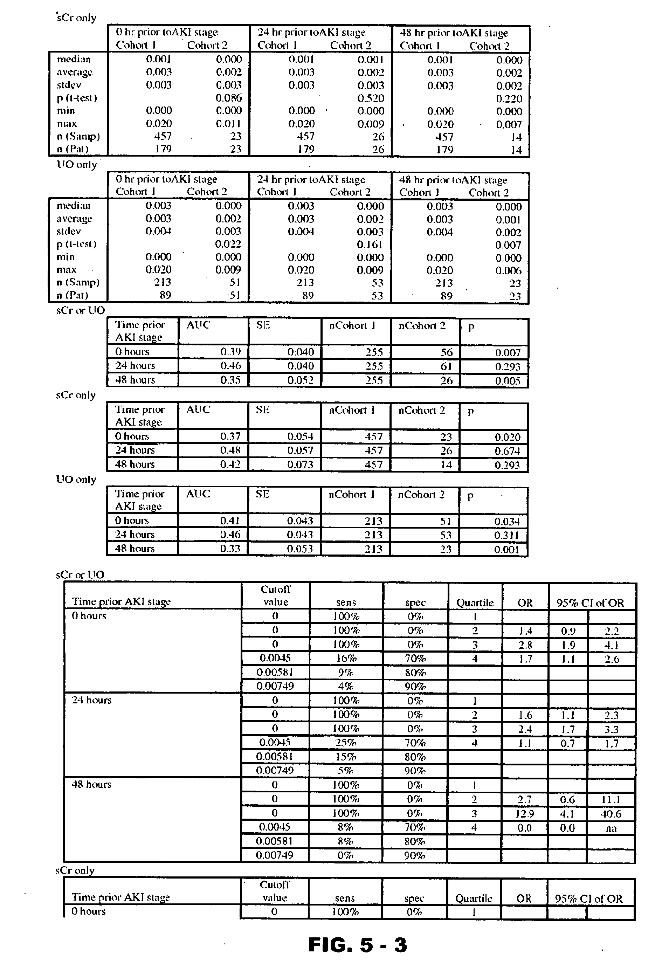

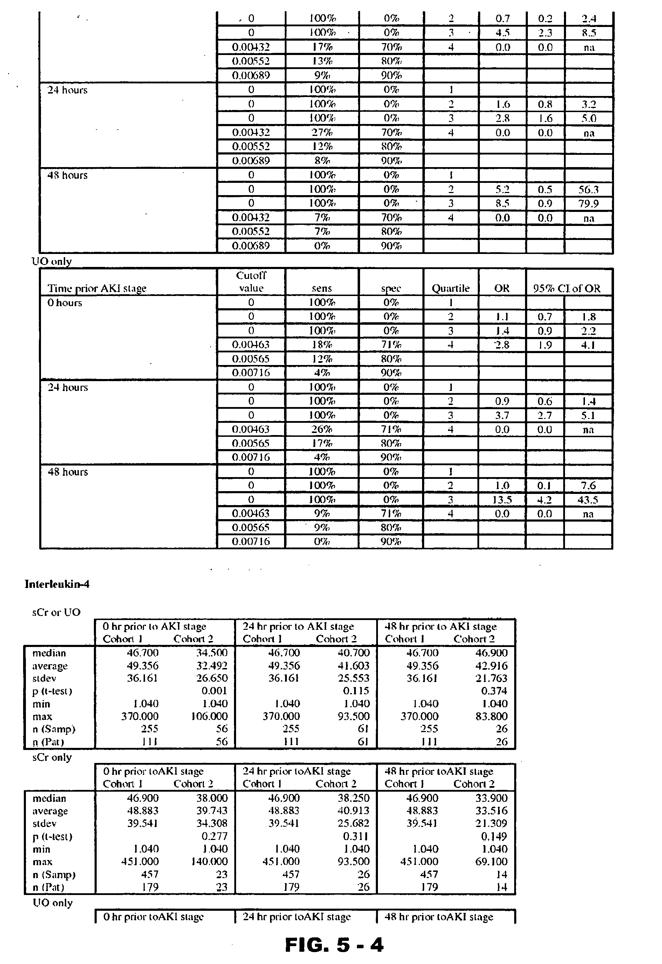

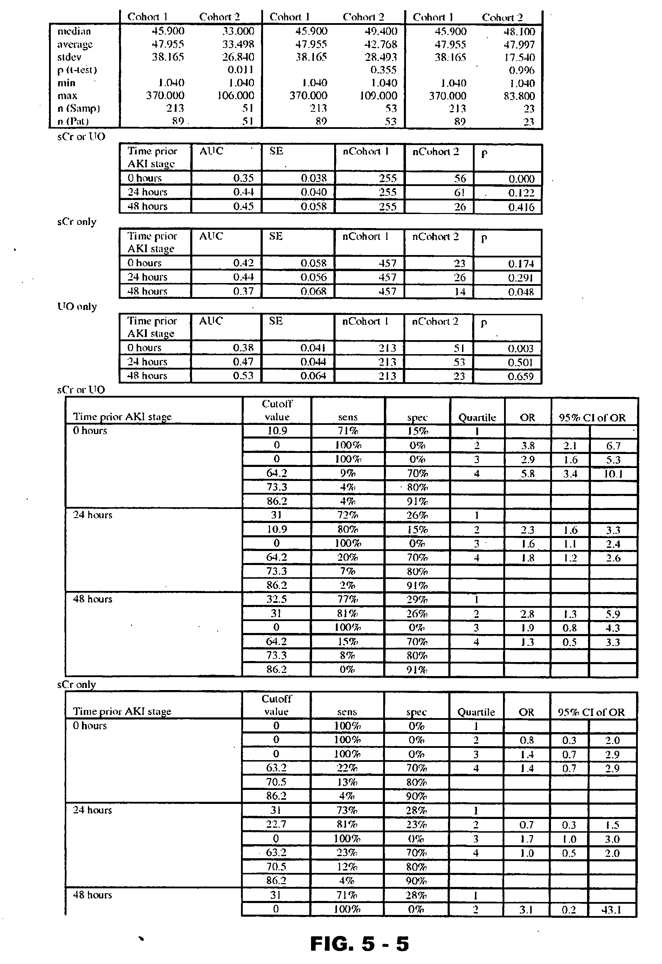

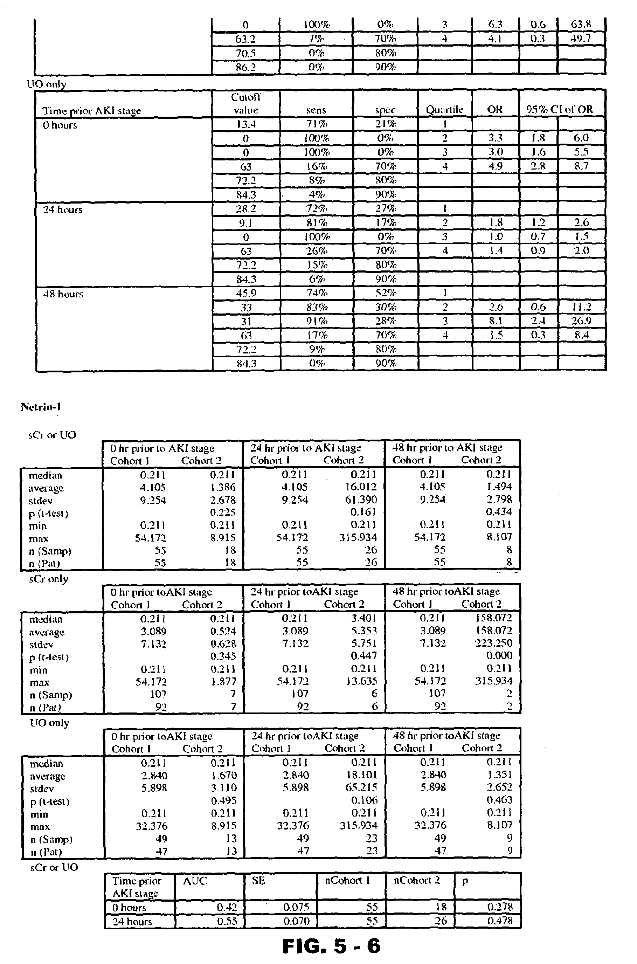

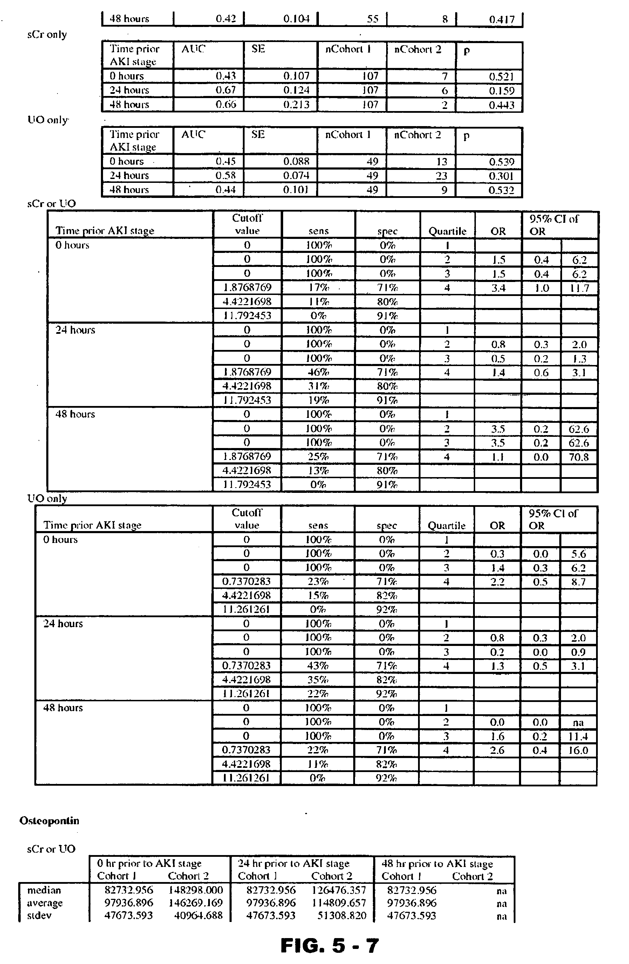

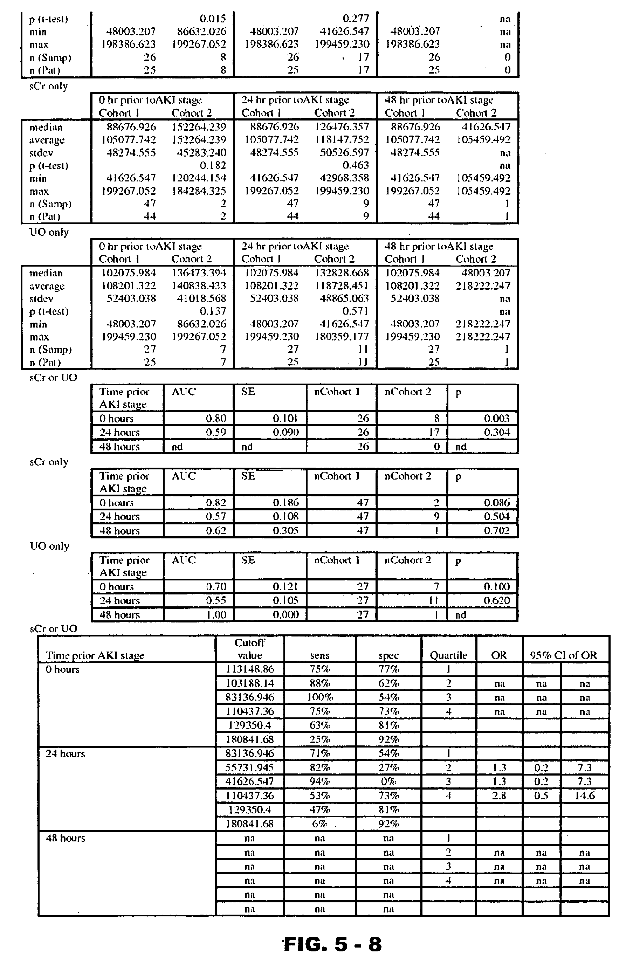

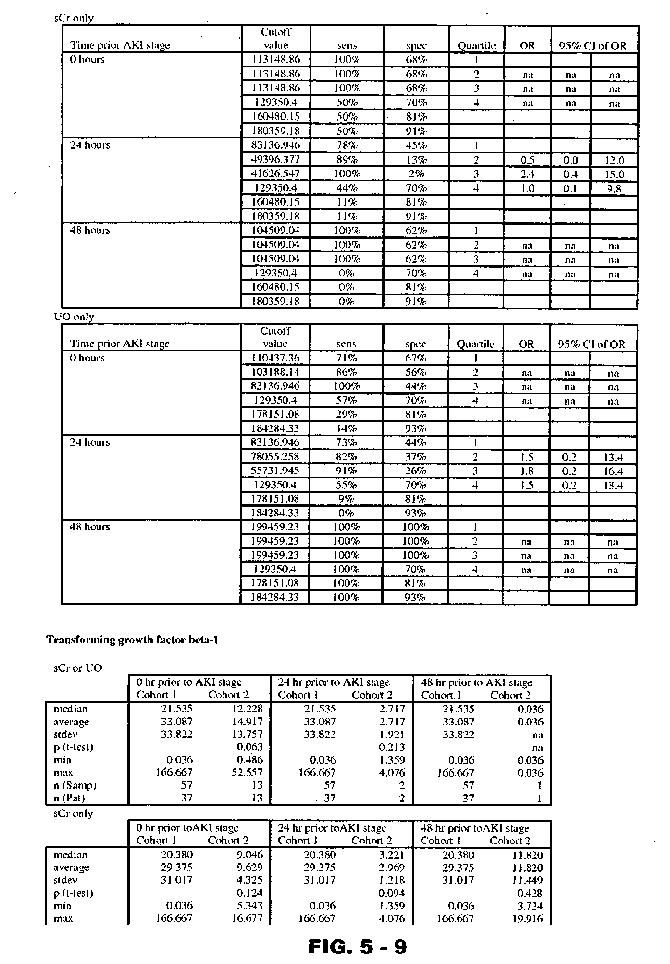

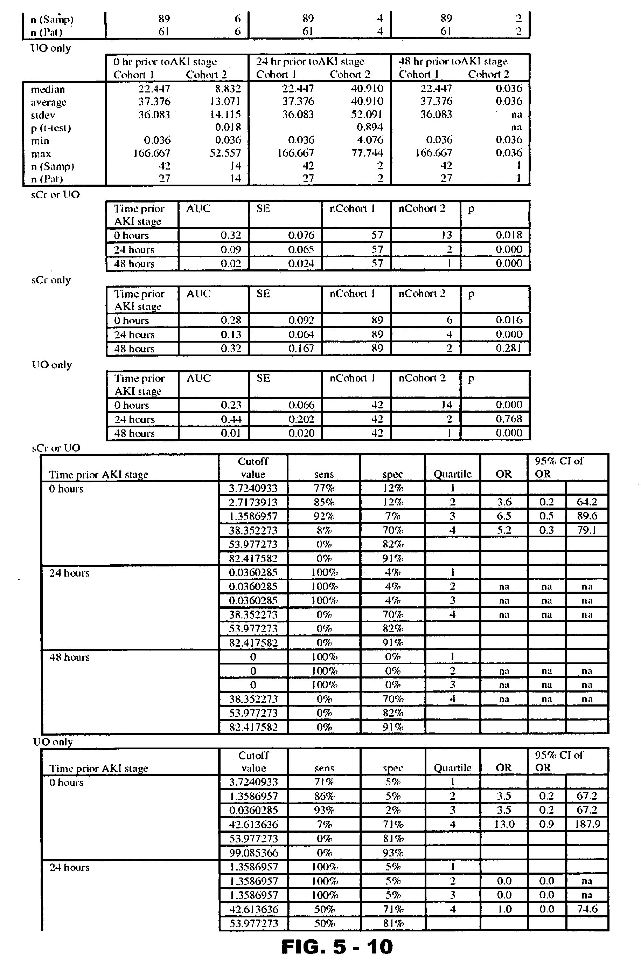

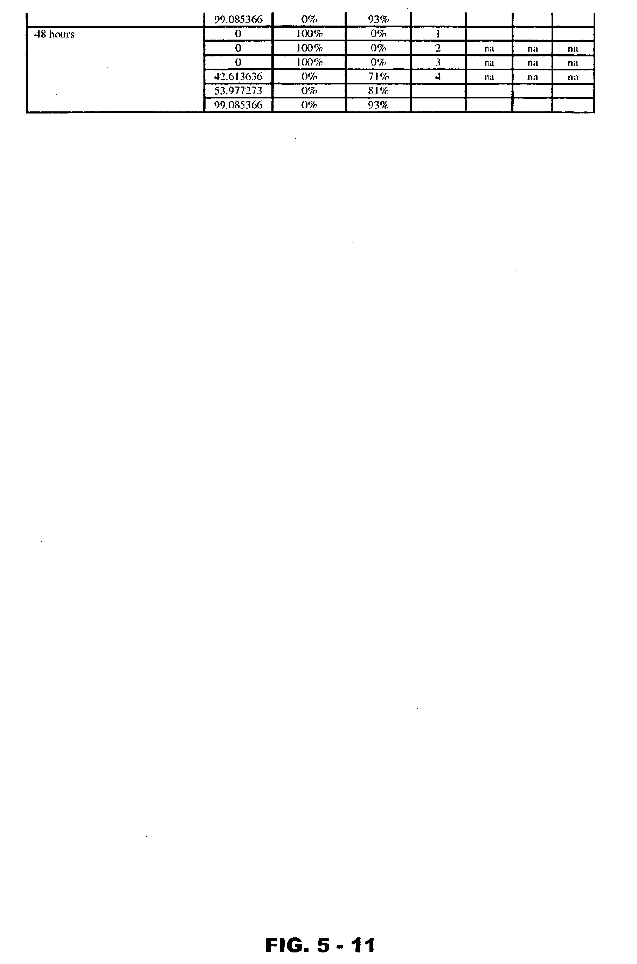

Fig. 5 provides data tables determined in accordance with Example 6 for the comparison of marker levels in plasma samples collected for Cohort 1 (patients that did not progress beyond RIFLE stage 0) and in plasma samples collected from subjects at 0, 24 hours, and 48 hours prior to reaching stage R, I or F in Cohort 2. Tables provide descriptive statistics, AUC analysis, and sensitivity, specificity and odds ratio calculations at various threshold (cutoff) levels for the various markers.

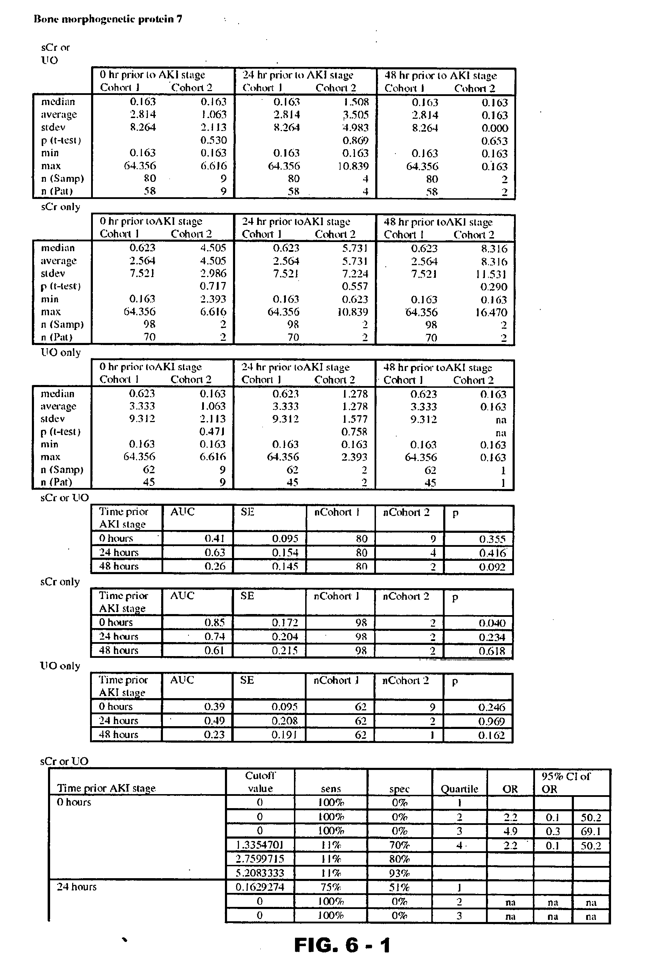

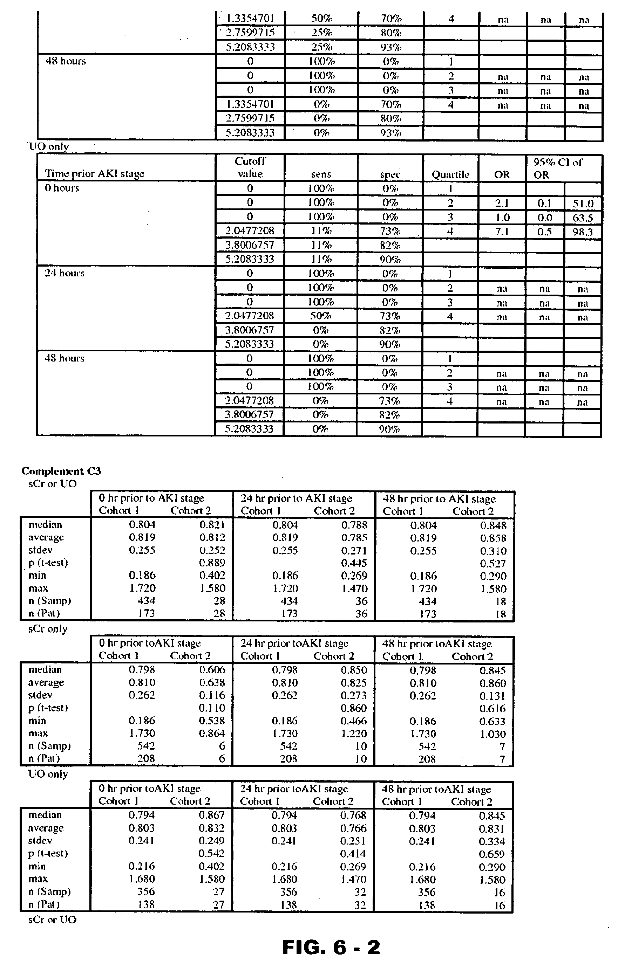

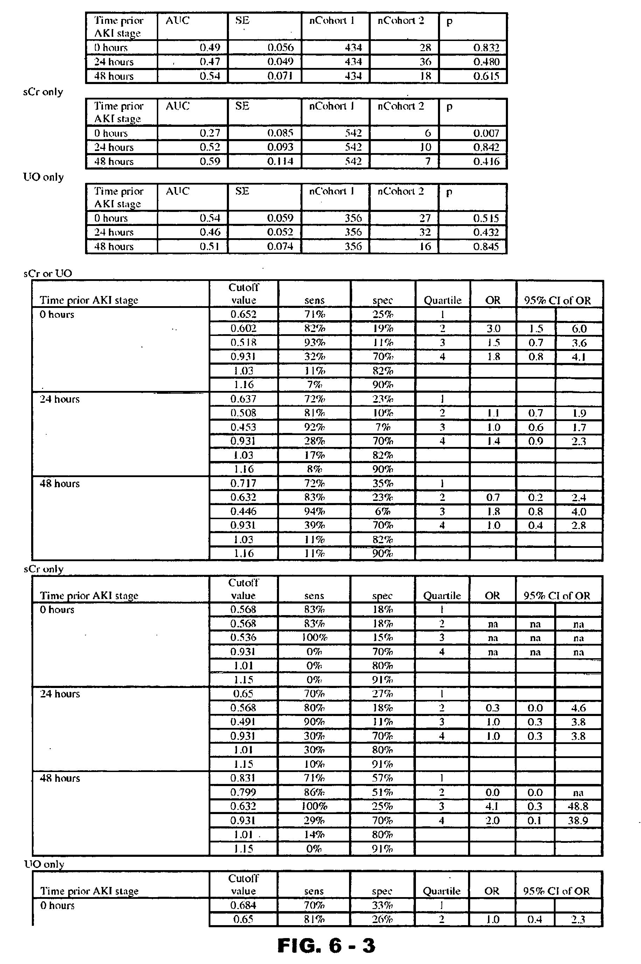

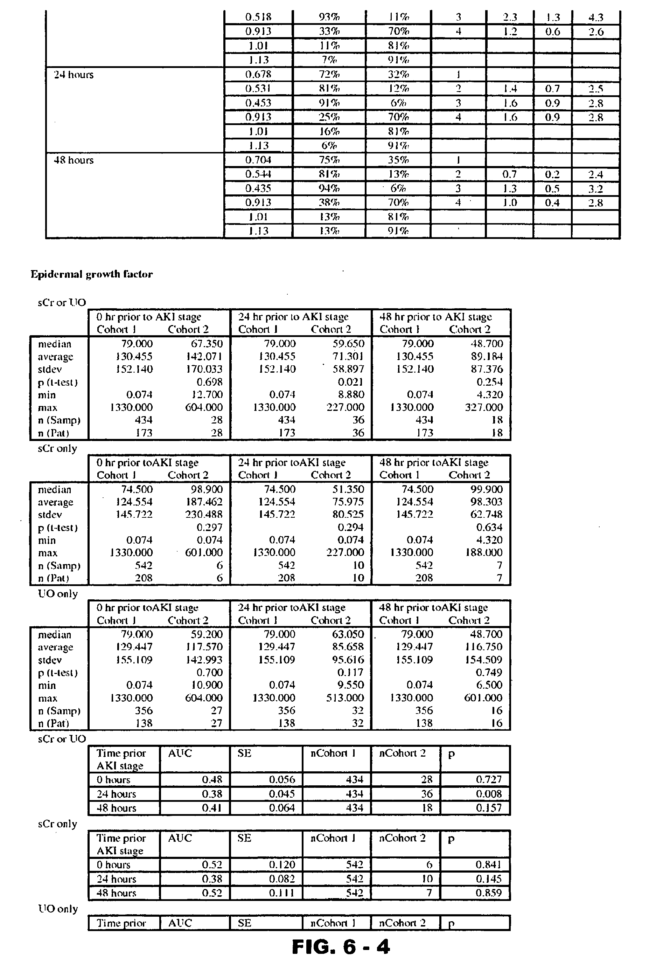

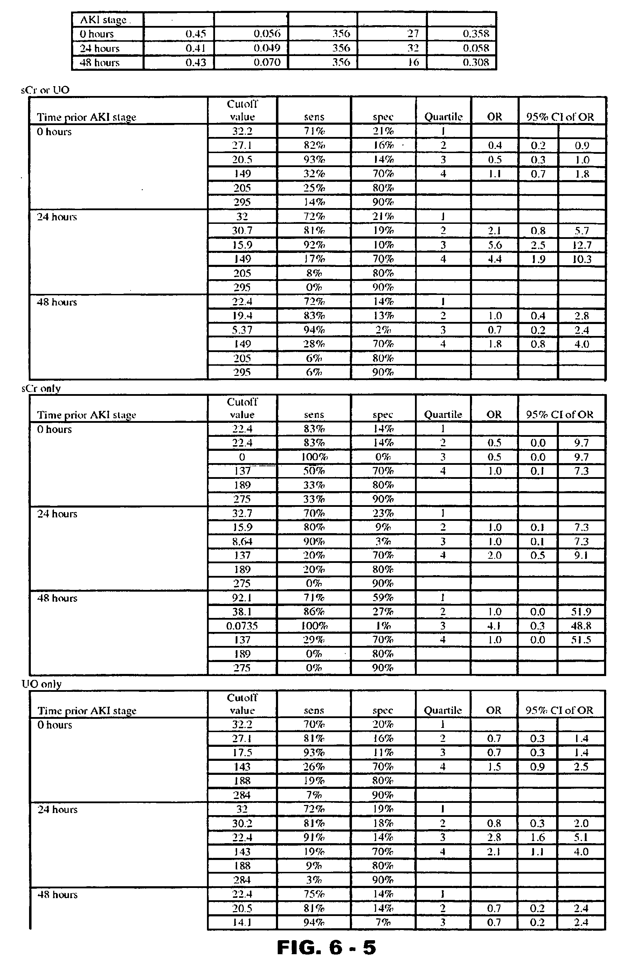

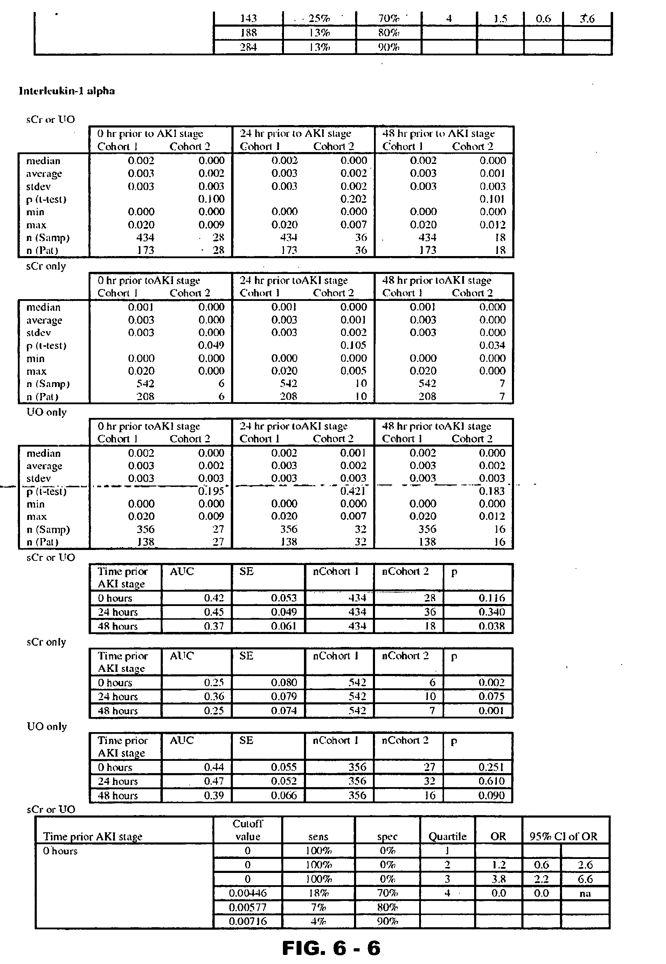

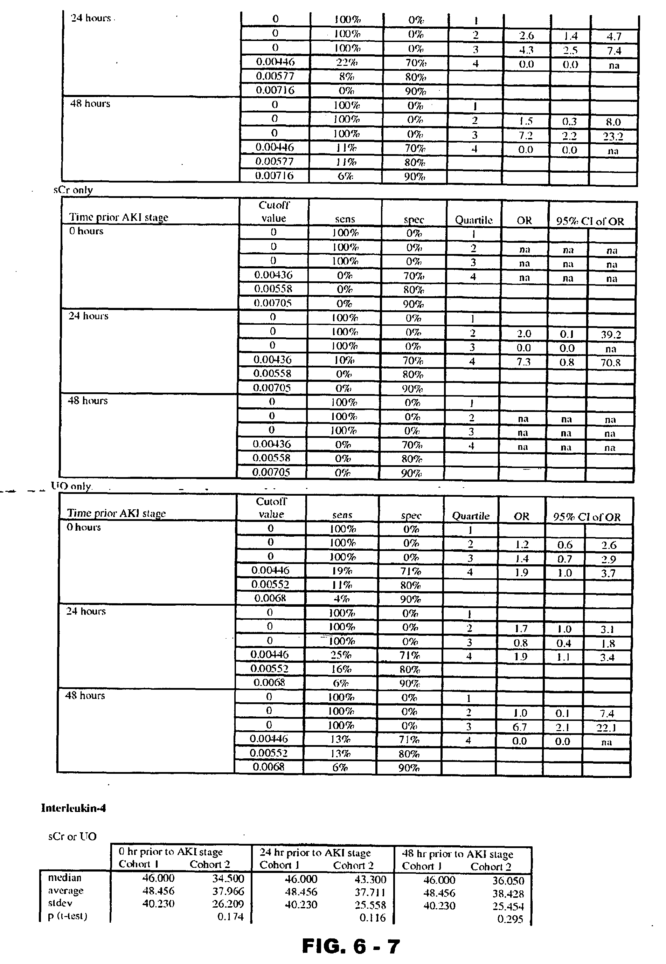

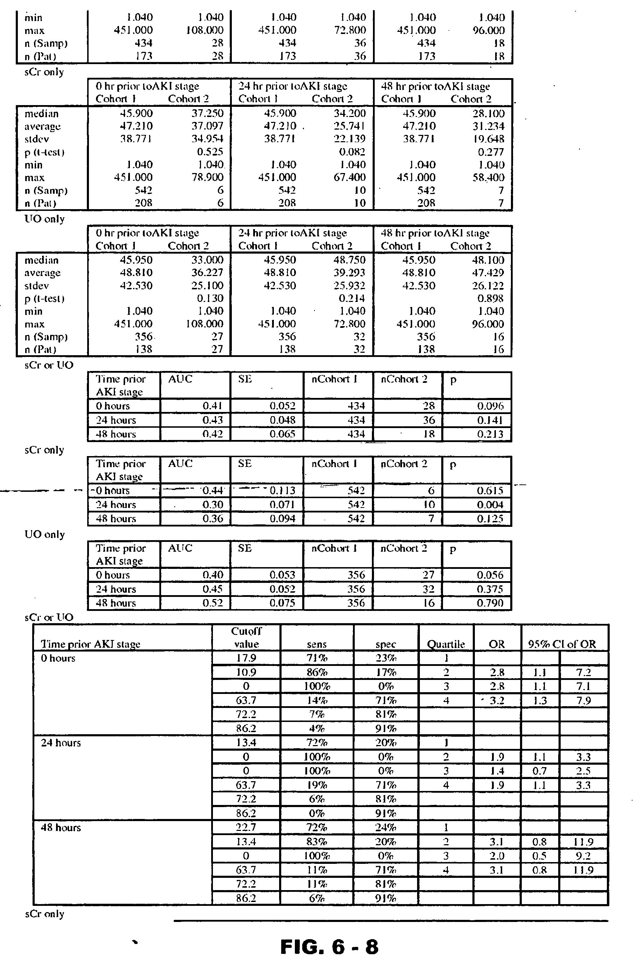

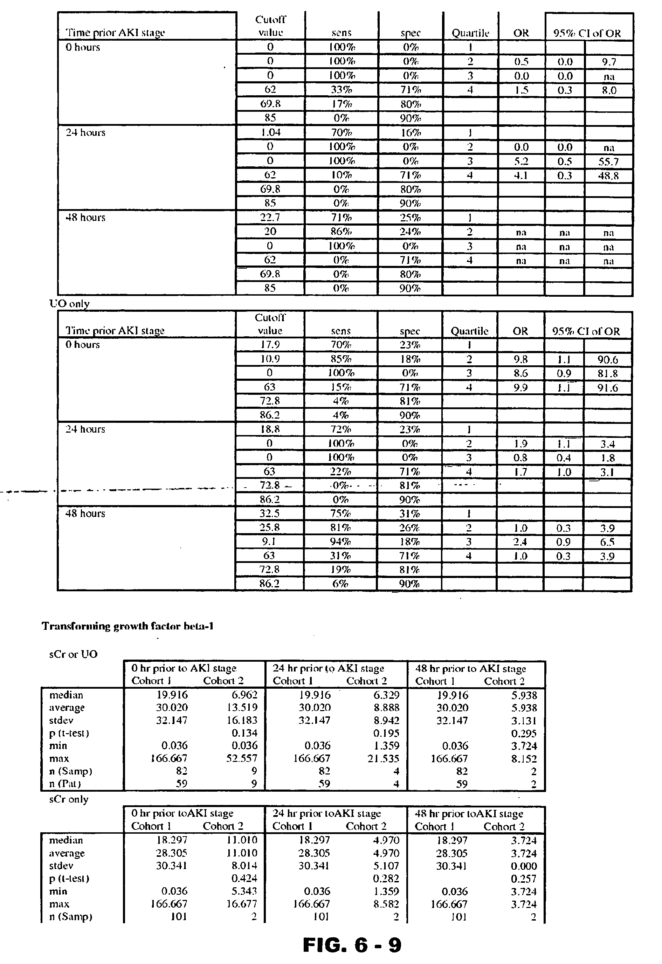

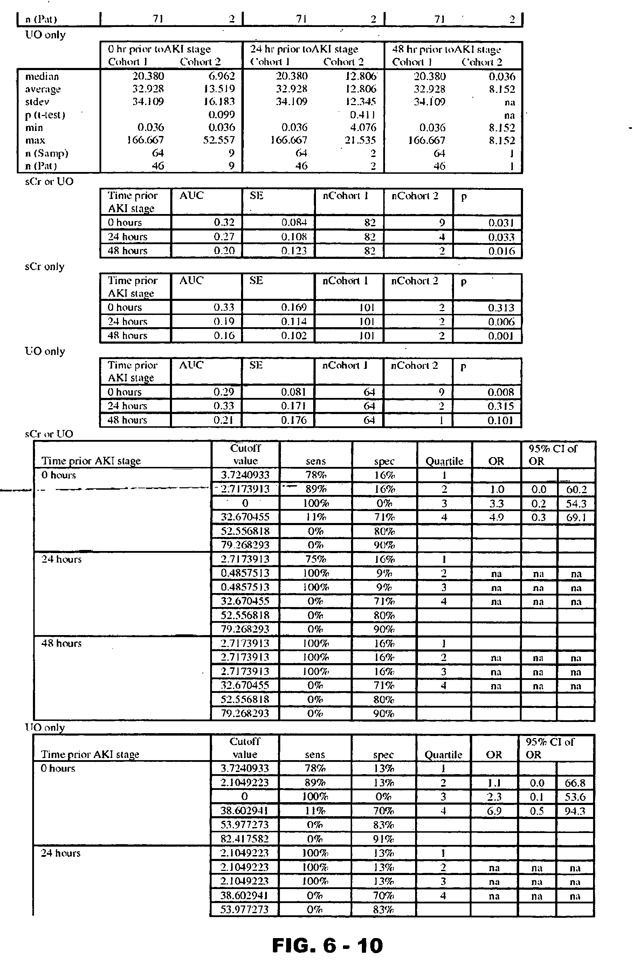

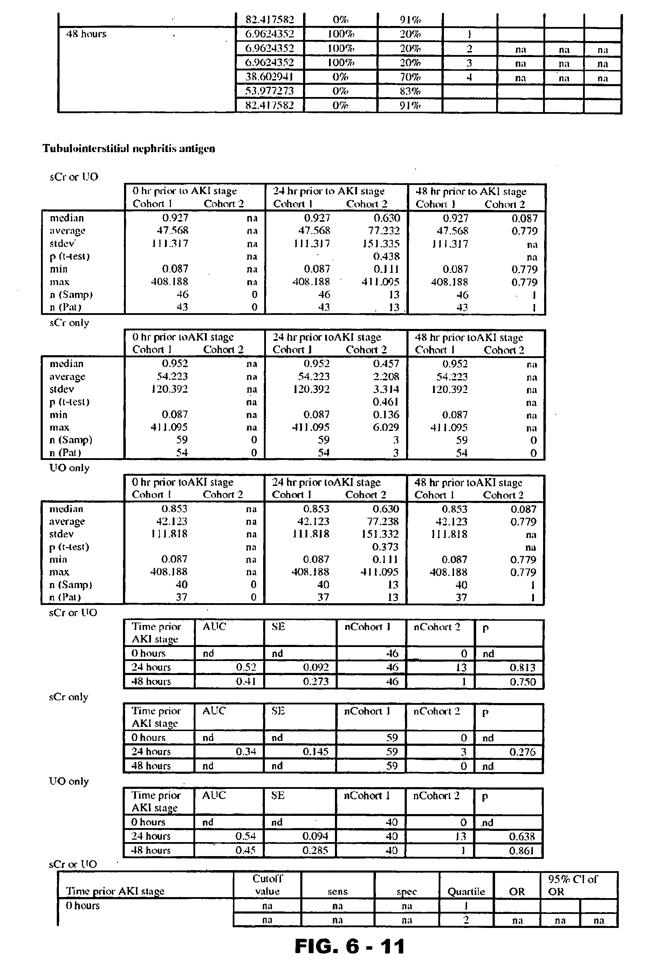

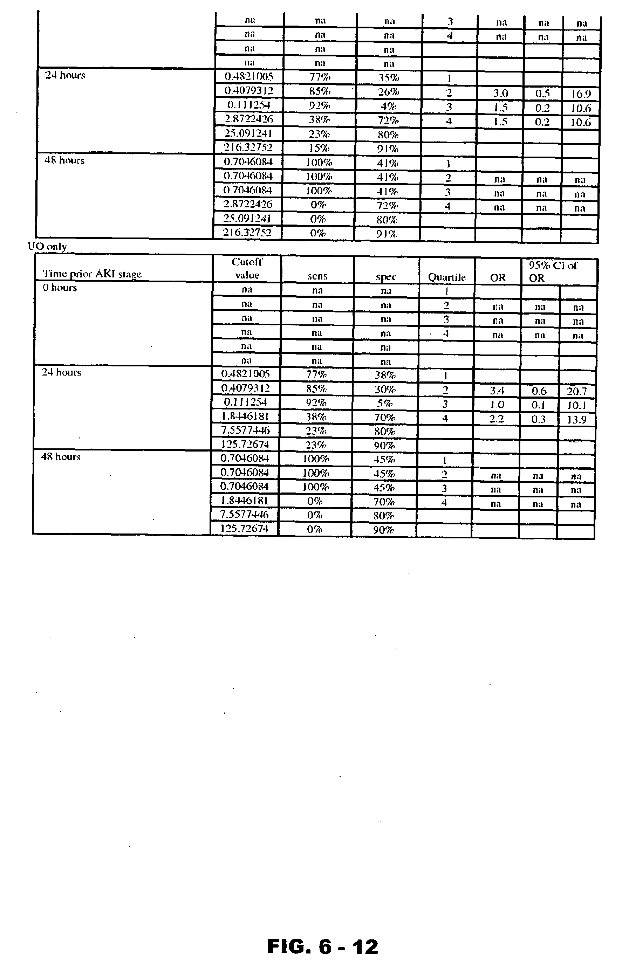

Fig. 6 provides data tables determined in accordance with Example 7 for the comparison of marker levels in plasma samples collected for Cohort 1 (patients that did not progress beyond RIFLE stage 0 or R) and in plasma samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

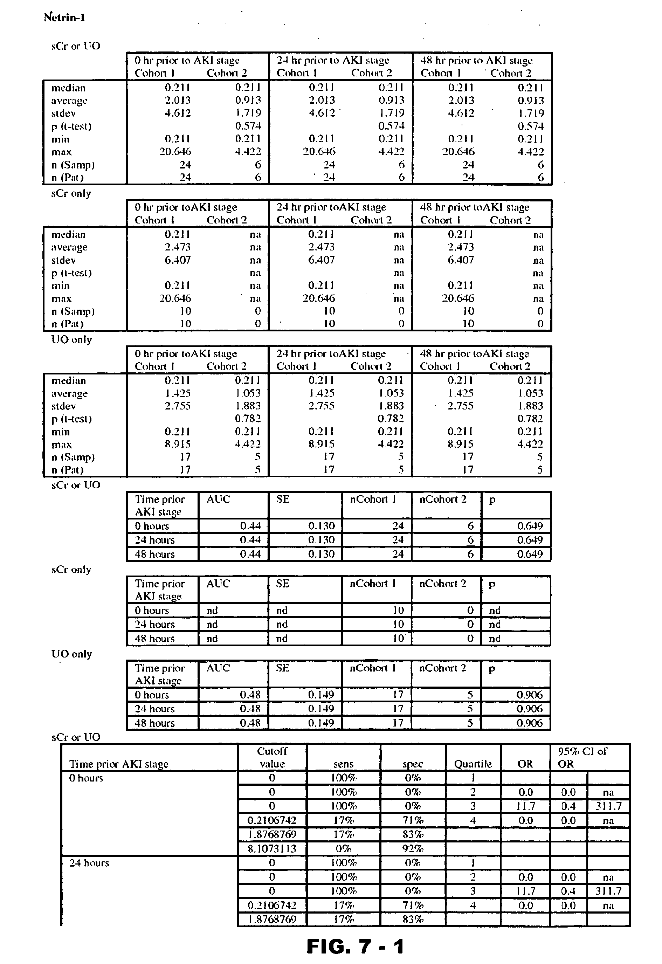

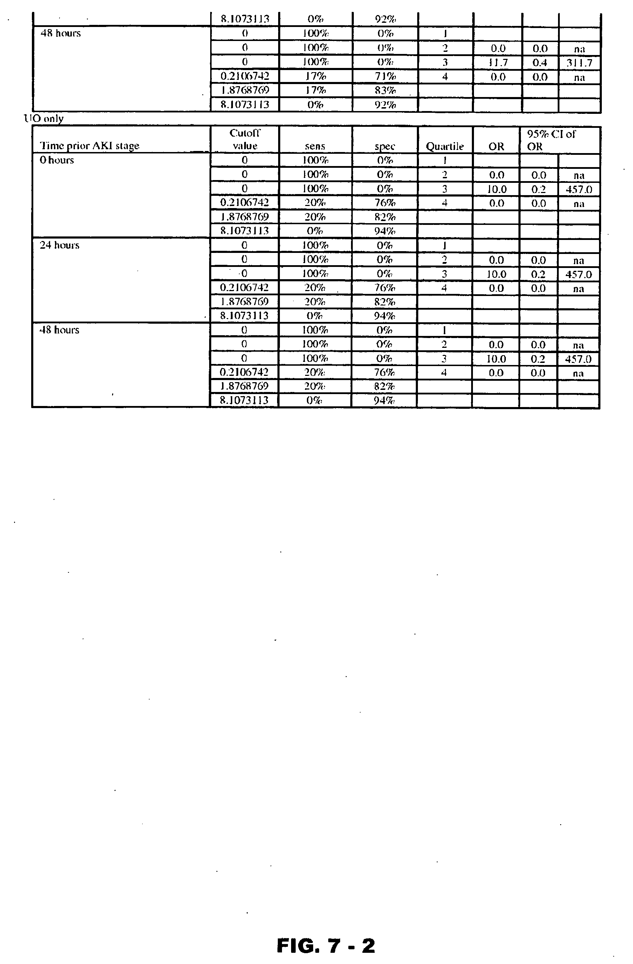

Fig. 7 provides data tables determined in accordance with Example 8 for the comparison of marker levels in plasma samples collected for Cohort 1 (patients that reached, but did not progress beyond, RIFLE stage R) and in plasma samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

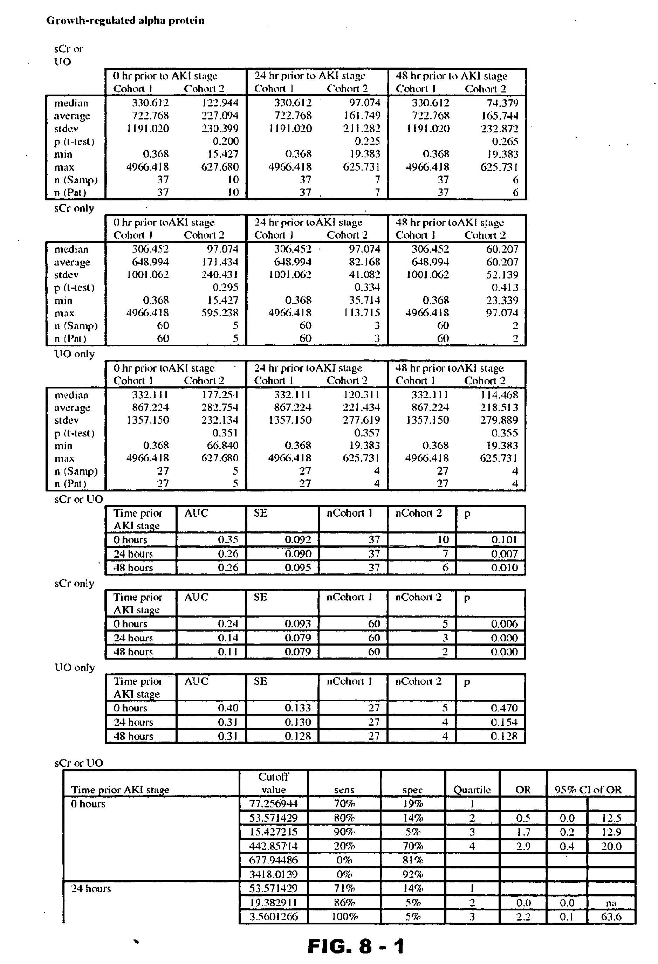

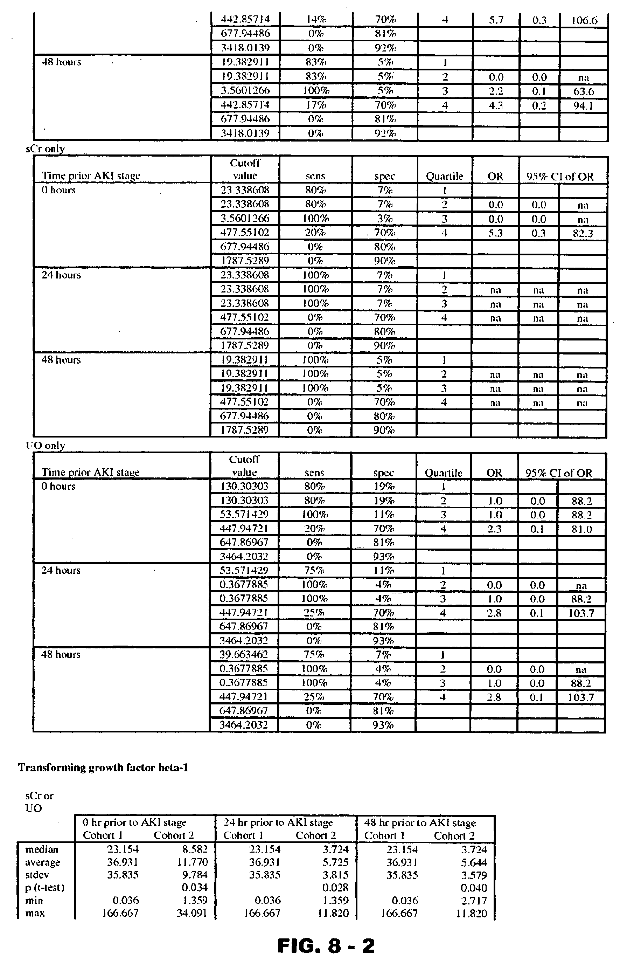

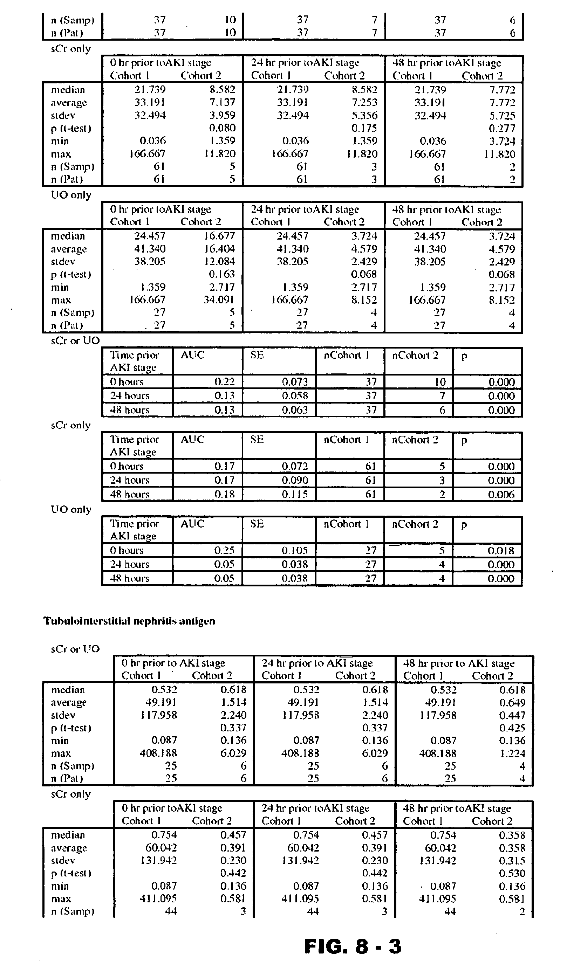

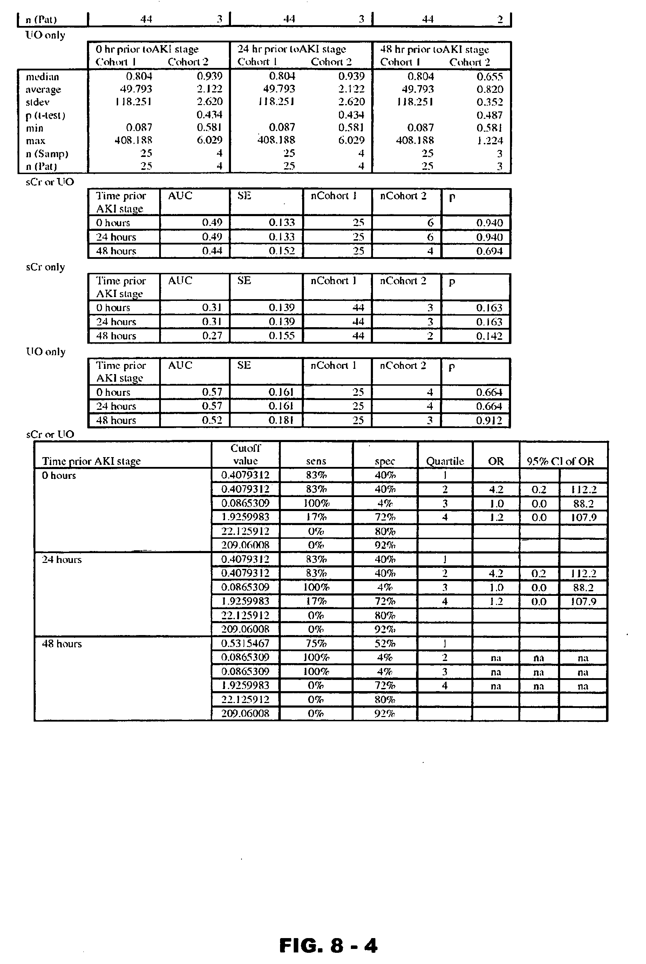

Fig. 8 provides data tables determined in accordance with Example 9 for the comparison of marker levels in plasma samples collected for Cohort 1 (patients that did not progress beyond RIFLE stage 0) and in plasma samples collected from subjects at 0, 24 hours, and 48 hours prior to reaching stage F in Cohort 2. Tables provide descriptive statistics, AUC analysis, and sensitivity, specificity and odds ratio calculations at various threshold (cutoff) levels for the various markers.

DETAILED DESCRIPTION OF THE INVENTION

The present invention relates to methods for evaluating renal status in a subject, comprising:

performing one or more assays configured to detect a kidney injury marker on a body fluid sample obtained from the subject to provide one or more assay results, the kidney injury marker being Epidermal growth factor and optionally further Complement C3, Interleukin-4, Interleukin-1 alpha, Tubulointerstitial nephritis antigen, Transforming growth factor beta-i, Bone morphogenetic protein 7, Osteopontin, Netrin1, and Growth-regulated alpha protein; and

correlating the assay result(s) to one or more of risk stratification, staging, prognosis, classifying and monitoring of the renal status of the subject, wherein said correlating step comprises assigning a likelihood of future acute renal failure (ARF) suffered by the subject and optionally one or more future changes in renal status to the subject based on the assay result(s) and the use of a kidney injury markers for prognosis of future acute renal failure in vitro, wherein the kidney injury marker is Epidermal growth factor, and optionally further Complement C3, Interleukin-4, Interleukin-1 alpha, Tubulointerstitial nephritis antigen, Transforming growth factor beta-i, Bone morphogenetic protein 7, Osteopontin, Netrin-1, and Growth-regulated alpha protein.

In the claimed invention at least Epidermal growth factor (EGF) is used as a marker.

For purposes of this document, the following definitions apply: As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq 8.8$ $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq 26.4$ $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

In this regard, the skilled artisan will understand that the signals obtained from an immunoassay are a direct result of complexes formed between one or more antibodies and the target biomolecule (*i.e.*, the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassay, including protein measurements (such as dot blots, western blots, chromatographic methods, mass spectrometry, *etc.*) and nucleic acid measurements (mRNA quatitation). This list is not meant to be limiting.

As used herein, the term "Epidermal growth factor" refers to one or more polypeptides present in a biological sample that are derived from the Epidermal growth factor precursor (Swiss-Prot P01 133 (SEQ ID NO: 1)).

```
          10         20         30         40         50         60
MLLTLIILLP VVSKFSFVSL SAPQHWSCPE GTLAGNGNST CVGPAPFLIF SHGNSIFRID

          70         80         90        100        110        120
TEGTNYEQLV VDAGVSVIMD FHYNEKRIYW VDLERQLLQR VFLNGSRQER VCNIEKNVSG

         130        140        150        160        170        180
MAINWINEEV IWSNQQEGII TVTDMKGNNS HILLSALKYP ANVAVDPVER FIFWSSEVAG

         190        200        210        220        230        240
SLYRADLDGV GVKALLETSE KITAVSLDVL DKRLFWIQYN REGSNSLICS CDYDGGSVHI

         250        260        270        280        290        300
SKHPTQHNLF AMSLFGDRIF YSTWKMKTIW IANKHTGKDM VRINLHSSFV PLGELKVVHP

         310        320        330        340        350        360
LAQPKAEDDT WEPEQKLCKL RKGNCSSTVC GQDLQSHLCM CAEGYALSRD RKYCEDVNEC

         370        380        390        400        410        420
AFWNHGCTLG CKNTPGSYYC TCPVGFVLLP DGKRCHQLVS CPRNVSECSH DCVLTSEGPL

         430        440        450        460        470        480
CFCPEGSVLE RDGKTCSGCS SPDNGGCSQL CVPLSPVSWE CDCFPGYDLQ LDEKSCAASG

         490        500        510        520        530        540
PQPFLLFANS QDIRHMHFDG TDYGTLLSQQ MGMVYALDHD PVENKIYFAH TALKWIERAN

         550        560        570        580        590        600
MDGSQRERLI EEGVDVPEGL AVDWIGRRFY WTDRGKSLIG RSDLNGKRSK IITKENISQP

         610        620        630        640        650        660
RGIAVHPMAK RLFWTDTGIN PRIESSSLQG LGRLVIASSD LIWPSGITID FLTDKLYWCD

         670        680        690        700        710        720
AKQSVIEMAN LDGSKRRRLT QNDVGHPFAV AVFEDYVWFS DWAMPSVIRV NKRTGKDRVR

         730        740        750        760        770        780
LQGSMLKPSS LVVVHPLAKP GADPCLYQNG GCEHICKKRL GTAWCSCREG FMKASDGKTC

         790        800        810        820        830        840
LALDGHQLLA GGEVDLKNQV TPLDILSKTR VSEDNITESQ HMLVAEIMVS DQDDCAPVGC

         850        860        870        880        890        900
```

```
SMYARCISEG EDATCQCLKG FAGDGKLCSD IDECEMGVPV CPPASSKCIN TEGGYVCRCS

       910        920        930        940        950        960
EGYQGDGIHC LDIDECQLGV HSCGENASCT NTEGGYTCMC AGRLSEPGLI CPDSTPPPHL

       970        980        990       1000       1010       1020
REDDHHYSVR NSDSECPLSH DGYCLHDGVC MYIEALDKYA CNCVVGYIGE RCQYRDLKWW

      1030       1040       1050       1060       1070       1080
ELRHAGHGQQ QKVIVVAVCV VVLVMLLLLS LWGAHYYRTQ KLLSKNPKNP YEESSRDVRS

      1090       1100       1110       1120       1130       1140
RRPADTEDGM SSCPQPWFVV IKEHQDLKNG GQPVAGEDGQ AADGSMQPTS WRQEPQLCGM

      1150       1160       1170       1180       1190       1200
GTEQGCWIPV SSDKGSCPQV MERSFHMPSY GTQTLEGGVE KPHSLLSANP LWQQRALDPP

HQMELTQ
```

Most preferably, the Epidermal growth factor assay detects one or more soluble forms of Epidermal growth factor. Epidermal growth factor is a single-pass type I membrane protein having a large extracellular domain, some or all of which is present in soluble forms of Epidermal growth factor generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Epidermal growth factor:

| Residues | Length | Domain ID |
|---|---|---|
| 1-22 | 22 | signal sequence |
| 23-1207 | 1185 | Pro-epidermal growth factor |
| 971-1023 | 53 | Epidermal growth factor |
| 23-1032 | 1010 | extracellular |
| 1033-1053 | 21 | transmembrane |
| 1054-1207 | 154 | cytoplasmic |

As used herein, the term "Complement C3" refers to one or more polypeptides present in a biological sample that are derived from the Complement C3 precursor (Swiss-Prot P01024 (SEQ ID NO: 2)).

```
         10         20         30         40         50         60
```

MGPTSGPSLL LLLLTHLPLA LGSPMYSIIT PNILRLESEE TMVLEAHDAQ GDVPVTVTVH

DFPGKKLVLS SEKTVLTPAT NHMGNVTFTI PANREFKSEK GRNKFVTVQA TFGTQVVEKV

VLVSLQSGYL FIQTDKTIYT PGSTVLYRIF TVNHKLLPVG RTVMVNIENP EGIPVKQDSL

SSQNQLGVLP LSWDIPELVN MGQWKIRAYY ENSPQQVFST EFEVKEYVLP SFEVIVEPTE

KFYYIYNEKG LEVTITARFL YGKKVEGTAF VIFGIQDGEQ RISLPESLKR IPIEDGSGEV

VLSRKVLLDG VQNPRAEDLV GKSLYVSATV ILHSGSDMVQ AERSGIPIVT SPYQIHFTKT

PKYFKPGMPF DLMVFVTNPD GSPAYRVPVA VQGEDTVQSL TQGDGVAKLS INTHPSQKPL

SITVRTKKQE LSEAEQATRT MQALPYSTVG NSNNYLHLSV LRTELRPGET LNVNFLLRMD

RAHEAKIRYY TYLIMNKGRL LKAGRQVREP GQDLVVLPLS ITTDFIPSFR LVAYYTLIGA

SGQREVVADS VWVDVKDSCV GSLVVKSGQS EDRQPVPGQQ MTLKIEGDHG ARVVLVAVDK

GVFVLNKKNK LTQSKIWDVV EKADIGCTPG SGKDYAGVFS DAGLTFTSSS GQQTAQRAEL

QCPQPAARRR RSVQLTEKRM DKVGKYPKEL RKCCEDGMRE NPMRFSCQRR TRFISLGEAC

KKVFLDCCNY ITELRRQHAR ASHLGLARSN LDEDIIAEEN IVSRSEFPES WLWNVEDLKE

PPKNGISTKL MNIFLKDSIT TWEILAVSMS DKKGICVADP FEVTVMQDFF IDLRLPYSVV

RNEQVEIRAV LYNYRQNQEL KVRVELLHNP AFCSLATTKR RHQQTVTIPP KSSLSVPYVI

VPLKTGLQEV EVKAAVYHHF ISDGVRKSLK VVPEGIRMNK TVAVRTLDPE RLGREGVQKE

DIPPADLSDQ VPDTESETRI LLQGTPVAQM TEDAVDAERL KHLIVTPSGC GEQNMIGMTP

TVIAVHYLDE TEQWEKFGLE KRQGALELIK KGYTQQLAFR QPSSAFAAFV KRAPSTWLTA

YVVKVFSLAV NLIAIDSQVL CGAVKWLILE KQKPDGVFQE DAPVIHQEMI GGLRNNNEKD

```
      1150       1160       1170       1180       1190       1200
MALTAFVLIS LQEAKDICEE QVNSLPGSIT KAGDFLEANY MNLQRSYTVA IAGYALAQMG

      1210       1220       1230       1240       1250       1260
RLKGPLLNKF LTTAKDKNRW EDPGKQLYNV EATSYALLAL LQLKDFDFVP PVVRWLNEQR

      1270       1280       1290       1300       1310       1320
YYGGGYGSTQ ATFMVFQALA QYQKDAPDHQ ELNLDVSLQL PSRSSKITHR IHWESASLLR

      1330       1340       1350       1360       1370       1380
SEETKENEGF TVTAEGKGQG TLSVVTMYHA KAKDQLTCNK FDLKVTIKPA PETEKRPQDA

      1390       1400       1410       1420       1430       1440
KNTMILEICT RYRGDQDATM SILDISMMTG FAPDTDDLKQ LANGVDRYIS KYELDKAFSD

      1450       1460       1470       1480       1490       1500
RNTLIIYLDK VSHSEDDCLA FKVHQYFNVE LIQPGAVKVY AYYNLEESCT RFYHPEKEDG

      1510       1520       1530       1540       1550       1560
KLNKLCRDEL CRCAEENCFI QKSDDKVTLE ERLDKACEPG VDYVYKTRLV KVQLSNDFDE

      1570       1580       1590       1600       1610       1620
YIMAIEQTIK SGSDEVQVGQ QRTFISPIKC REALKLEEKK HYLMWGLSSD FWGEKPNLSY

      1630       1640       1650       1660
IIGKDTWVEH WPEEDECQDE ENQKQCQDLG AFTESMVVFG CPN
```

The following domains have been identified in Complement C3:

| Residues | Length | Domain ID |
|---|---|---|
| 1-22 | 22 | Signal peptide |
| 23-1663 | 1641 | Complement C3 |
| 23-667 | 645 | Complement C3 beta chain |
| 672-1663 | 992 | Complement C3 alpha chain |
| 672-748 | 77 | Complement C3 anaphylatoxin |
| 749-1663 | 915 | Complement C3b alpha'chain |
| 749-954 | 206 | Complement C3c alpha' chain fragment 1 |
| 955-1303 | 349 | Complement C3dg fragment |
| 955-1001 | 47 | Complement C3g fragment |
| 1002-1303 | 302 | Complement C3d fragment |
| 1304-1320 | 17 | Complement C3f fragment |
| 1321-1663 | 343 | Complement C3c alpha' chain fragment 2 |

As used herein, the term "Interleukin-4" refers to one or more polypeptides present in a biological sample that are derived from the Interleukin-4 precursor (Swiss-Prot P05112 (SEQ ID NO: 3)).

```
            10         20         30         40         50         60
  MGLTSQLLPP LFFLLACAGN FVHGHKCDIT LQEIIKTLNS LTEQKTLCTE LTVTDIFAAS

            70         80         90        100        110        120
  KNTTEKETFC RAATVLRQFY SHHEKDTRCL GATAQQFHRH KQLIRFLKRL DRNLWGLAGL

           130        140        150
  NSCPVKEANQ STLENFLERL KTIMREKYSK CSS
```

The following domains have been identified in Interleukin-4:

| Residues | Length | Domain ID |
|----------|--------|-----------|
| 1-24 | 24 | Signal peptide |
| 25-153 | 129 | Interleukin-4 |

As used herein, the term "Interleukin-1 alpha" refers to one or more polypeptides present in a biological sample that are derived from the Interleukin-1 alpha precursor (Swiss-Prot P01583 (SEQ ID NO: 4)).

```
            10         20         30         40         50         60
  MAKVPDMFED LKNCYSENEE DSSSIDHLSL NQKSFYHVSY GPLHEGCMDQ SVSLSISETS

            70         80         90        100        110        120
  KTSKLTFKES MVVVATNGKV LKKRRLSLSQ SITDDDLEAI ANDSEEEIIK PRSAPFSFLS

           130        140        150        160        170        180
  NVKYNFMRII KYEFILNDAL NQSIIRANDQ YLTAAALHNL DEAVKFDMGA YKSSKDDAKI

           190        200        210        220        230        240
  TVILRISKTQ LYVTAQDEDQ PVLLKEMPEI PKTITGSETN LLFFWETHGT KNYFTSVAHP

           250        260        270
  NLFIATKQDY WVCLAGGPPS ITDFQILENQ A
```

The following domains have been identified in Interleukin-1 alpha:

| Residues | Length | Domain ID |
|----------|--------|-----------|
| 1-112 | 112 | Propeptide |
| 113-271 | 159 | Interleukin-1 alpha |

As used herein, the term "Tubulointerstitial nephritis antigen" refers to one or more polypeptides present in a biological sample that are derived from the Tubulointerstitial nephritis antigen precursor (Swiss-Prot Q9UJW2 (SEQ ID NO: 5)).

```
              10         20         30         40         50         60
         MWTGYKILIF SYLTTEIWME KQYLSQREVD LEAYFTRNHT VLQGTRFKRA IFQGQYCRNF

              70         80         90        100        110        120
         GCCEDRDDGC VTEFYAANAL CYCDKFCDRE NSDCCPDYKS FCREEKEWPP HTQPWYPEGC

             130        140        150        160        170        180
         FKDGQHYEEG SVIKENCNSC TCSGQQWKCS QHVCLVRPEL IEQVNKGDYG WTAQNYSQFW

             190        200        210        220        230        240
         GMTLEDGFKF RLGTLPPSPM LLSMNEMTAS LPATTDLPEF FVASYKWPGW THGPLDQKNC

             250        260        270        280        290        300
         AASWAFSTAS VAADRIAIQS KGRYTANLSP QNLISCCAKN RHGCNSGSID RAWWYLRKRG

             310        320        330        340        350        360
         LVSHACYPLF KDQNATNNGC AMASRSDGRG KRHATKPCPN NVEKSNRIYQ CSPPYRVSSN

             370        380        390        400        410        420
         ETEIMKEIMQ NGPVQAIMQV REDFFHYKTG IYRHVTSTNK ESEKYRKLQT HAVKLTGWGT

             430        440        450        460        470
         LRGAQGQKEK FWIAANSWGK SWGENGYFRI LRGVNESDIE KLIIAAWGQL TSSDEP
```

The following domains have been identified in Tubulointerstitial nephritis antigen:

| Residues | Length | Domain ID |
|----------|--------|-----------|
| 1-476 | 112 | Tubulointerstitial nephritis antigen |
| 59-107 | 49 | SMB |

As used herein, the term "Transforming growth factor beta-1" refers to one or more polypeptides present in a biological sample that are derived from the Transforming growth factor beta-1 precursor (Swiss-Prot P01137 (SEQ ID NO: 6)).

```
              10         20         30         40         50         60
         MPPSGLRLLL LLLPLLWLLV LTPGRPAAGL STCKTIDMEL VKRKRIEAIR GQILSKLRLA

              70         80         90        100        110        120
         SPPSQGEVPP GPLPEAVLAL YNSTRDRVAG ESAEPEPEPE ADYYAKEVTR VLMVETHNEI

             130        140        150        160        170        180
         YDKFKQSTHS IYMFFNTSEL REAVPEPVLL SRAELRLLRL KLKVEQHVEL YQKYSNNSWR

             190        200        210        220        230        240
         YLSNRLLAPS DSPEWLSFDV TGVVRQWLSR GGEIEGFRLS AHCSCDSRDN TLQVDINGFT

             250        260        270        280        290        300
         TGRRGDLATI HGMNRPFLLL MATPLERAQH LQSSRHRRAL DTNYCFSSTE KNCCVRQLYI

             310        320        330        340        350        360
         DFRKDLGWKW IHEPKGYHAN FCLGPCPYIW SLDTQYSKVL ALYNQHNPGA SAAPCCVPQA

             370        380        390
         LEPLPIVYYV GRKPKVEQLS NMIVRSCKCS
```

The following domains have been identified in Transforming growth factor beta-1:

| Residues | Length | Domain ID |
|---|---|---|
| 1-29 | 29 | Signal peptide |
| 30-278 | 249 | Latency-associated peptide |
| 279-390 | 112 | Transforming growth factor beta-1 |

As used herein, the term "Bone morphogenetic protein 7" refers to one or more polypeptides present in a biological sample that are derived from the Bone morphogenetic protein 7 precursor (Swiss-Prot P18075 (SEQ ID NO: 7)).

```
            10         20         30         40         50         60
    MHVRSLRAAA PHSFVALWAP LFLLRSALAD FSLDNEVHSS FIHRRLRSQE RREMQREILS

            70         80         90        100        110        120
    ILGLPHRPRP HLQGKHNSAP MFMLDLYNAM AVEEGGGPGG QGFSYPYKAV FSTQGPPLAS

           130        140        150        160        170        180
    LQDSHFLTDA DMVMSFVNLV EHDKEFFHPR YHHREFRFDL SKIPEGEAVT AAEFRIYKDY

           190        200        210        220        230        240
    IRERFDNETF RISVYQVLQE HLGRESDLFL LDSRTLWASE EGWLVFDITA TSNHWVVNPR

           250        260        270        280        290        300
    HNLGLQLSVE TLDGQSINPK LAGLIGRHGP QNKQPFMVAF FKATEVHFRS IRSTGSKQRS

           310        320        330        340        350        360
    QNRSKTPKNQ EALRMANVAE NSSSDQRQAC KKHELYVSFR DLGWQDWIIA PEGYAAYYCE

           370        380        390        400        410        420
    GECAFPLNSY MNATNHAIVQ TLVHFINPET VPKPCCAPTQ LNAISVLYFD DSSNVILKKY

           430
    RNMVVRACGC H
```

The following domains have been identified in Bone morphogenetic protein 7:

| Residues | Length | Domain ID |
|---|---|---|
| 1-29 | 29 | Signal peptide |
| 30-292 | 263 | Propeptide |
| 293-431 | 139 | Bone morphogenetic protein 7 |

As used herein, the term "Osteopontin" refers to one or more polypeptides present in a biological sample that are derived from the Osteopontin precursor (Swiss-Prot P10451 (SEQ ID NO: 8)).

```
          10         20         30         40         50         60
    MRIAVICFCL LGITCAIPVK QADSGSSEEK QLYNKYPDAV ATWLNPDPSQ KQNLLAPQNA

          70         80         90        100        110        120
    VSSEETNDFK QETLPSKSNE SHDHMDDMDD EDDDDHVDSQ DSIDSNDSDD VDDTDDSHQS

         130        140        150        160        170        180
    DESHHSDESD ELVTDFPTDL PATEVFTPVV PTVDTYDGRG DSVVYGLRSK SKKFRRPDIQ

         190        200        210        220        230        240
    YPDATDEDIT SHMESEELNG AYKAIPVAQD LNAPSDWDSR GKDSYETSQL DDQSAETHSH

         250        260        270        280        290        300
    KQSRLYKRKA NDESNEHSDV IDSQELSKVS REFHSHEFHS HEDMLVVDPK SKEEDKHLKF

         310
    RISHELDSAS SEVN
```

The following domains have been identified in Osteopontin:

| Residues | Length | Domain ID |
|----------|--------|-----------|
| 1-16 | 16 | Signal peptide |
| 17-314 | 427 | Osteopontin |

As used herein, the term "Growth-regulated alpha protein" refers to one or more polypeptides present in a biological sample that are derived from the Growth-regulated alpha protein precursor (Swiss-Prot P09341 (SEQ ID NO: 9)).

```
          10         20         30         40         50         60
    MARAALSAAP SNPRLLRVAL LLLLLVAAGR RAAGASVATE LRCQCLQTLQ GIHPKNIQSV

          70         80         90        100
    NVKSPGPHCA QTEVIATLKN GRKACLNPAS PIVKKIIEKM LNSDKSN
```

The following domains have been identified in Growth-regulated alpha protein:

| Residues | Length | Domain ID |
|----------|--------|-----------|
| 1-34 | 34 | Signal peptide |
| 35-107 | 73 | Growth-regulated alpha protein |
| 38-107 | 70 | Growth-regulated alpha protein (4-73) |
| 39-107 | 69 | Growth-regulated alpha protein (5-73) |
| 40-107 | 68 | Growth-regulated alpha protein (6-73) |

As used herein, the term "Netrin-1" refers to one or more polypeptides present in a biological sample that are derived from the Netrin-1 precursor (Swiss-Prot 095631 (SEQ ID NO: 10)).

```
          10          20          30          40          50          60
MMRAVWEALA  ALAAVACLVG  AVRGGPGLSM  FAGQAAQPDP  CSDENGHPRR  CIPDFVNAAF

          70          80          90         100         110         120
GKDVRVSSTC  GRPPARYCVV  SERGEERLRS  CHLCNASDPK  KAHPPAFLTD  LNNPHNLTCW

         130         140         150         160         170         180
QSENYLQFPH  NVTLTLSLGK  KFEVTYVSLQ  FCSPRPESMA  IYKSMDYGRT  WVPFQFYSTQ

         190         200         210         220         230         240
CRKMYNRPHR  APITKQNEQE  AVCTDSHTDM  RPLSGGLIAF  STLDGRPSAH  DFDNSPVLQD

         250         260         270         280         290         300
WVTATDIRVA  FSRLHTFGDE  NEDDSELARD  SYFYAVSDLQ  VGGRCKCNGH  AARCVRDRTD

         310         320         330         340         350         360
SLVCDCRHNT  AGPECDRCKP  FHYDRPWQRA  TAREANECVA  CNCNLHARRC  RFNMELYKLS

         370         380         390         400         410         420
GRKSGGVCLN  CRHNTAGRHC  HYCKEGYYRD  MGKPITHRKA  CKACDCHPVG  AAGKTCNQTT

         430         440         450         460         470         480
GQCPCKDGVT  GITCNRCAKG  YQQSRSPIAP  CIKIPVAPPT  TAASSVEEPE  DCDSYCKASK

         490         500         510         520         530         540
GKLKINMKKY  CKKDYAVQIH  ILKADKAGDW  WKFTVNIISV  YKQGTSRIRR  GDQSLWIRSR

         550         560         570         580         590         600
DIACKCPKIK  PLKKYLLLGN  AEDSPDQSGI  VADKSSLVIQ  WRDTWARRLR  KFQQREKKGK
```

CKKA

The following domains have been identified in Netrin-1:

| Residues | Length | Domain ID |
|----------|--------|-----------|
| 1-24 | 24 | Signal peptide |
| 25-604 | 580 | Netrin-1 |

As used herein, the term "relating a signal to the presence or amount" of an analyte reflects this understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.*, fluorescent moieties, electrochemical labels, metal chelates, *etc.*) as well as molecules that may be indirectly detected by production of a detectable reaction product (*e.g.,* enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present specification provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writhing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See*, *e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecules. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See*, *e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.,* in sandwich assays) may interfere with one another sterically, *etc.,* assay performance of an antibody may be a more important measure than absolute affinity and specificity of an antibody.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5$^{th}$ percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, *etc.*) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Cathepsin B (P07858); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1 alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltrans-

ferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of F1F0 ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (O14788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, *etc.* The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points =risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.

Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria

males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay formal

Analytes are is measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Concentrations are expressed in the following examples as follows: Epidermal growth factor - pg/mL, Complement C3 - mg/mL, Interleukin-4 - pg/mL, Interleukin-1 alpha - ng/mL, Tubulointerstitial nephritis antigen - $\mu$g/mL, Transforming growth factor beta-1 - pg/mL, Bone morphogenetic protein 7 - pg/mL, Osteopontin - pg/mL, Netrin-1 - ng/mL, and Growth-regulated alpha protein - pg/mL.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Kidney injury markers for evaluating renal status in patients at RIFLE Stage 0

Patients from the intensive care unit (ICU) were classified by kidney status as non-injury (0), risk of injury (R), injury (I), and failure (F) according to the maximum stage reached within 7 days of enrollment as determined by the RIFLE criteria.

Two cohorts were defined as (Cohort 1) patients that did not progress beyond stage 0, and (Cohort 2) patients that reached stage R, I, or F within 10 days. To address normal marker fluctuations that occur within patients at the ICU and thereby assess utility for monitoring AKI status, marker levels were measured in urine samples collected for Cohort 1. Marker concentrations were measured in urine samples collected from a subject at 0, 24 hours, and 48 hours prior to reaching stage R, I or F in Cohort 2. In the following tables, the time "prior max stage" represents the time at which a sample is collected, relative to the time a particular patient reaches the lowest disease stage as defined for that cohort, binned into three groups which are +/- 12 hours. For example, 24 hr prior for this example (0 vs R, I, F) would mean 24 hr (+/- 12 hours) prior to reaching stage R (or I if no sample at R, or F if no sample at R or I).

Each marker was measured by standard immunoassay methods using commercially available assay reagents. A receiver operating characteristic (ROC) curve was generated for each marker and the area under each ROC curve (AUC) was determined. Patients in Cohort 2 were also separated according to the reason for adjudication to stage R, I, or F as being based on serum creatinine measurements (sCr), being based on urine output (UO), or being based on either serum creatinine measurements or urine output. That is, for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, for those patients adjudicated to stage R, I, or F on the basis of serum creatinine measurements or urine output, the adjudication method which yielded the most severe RIFLE stage was used.

The ability to distinguish cohort 1 (subjects remaining in RIFLE 0) from Cohort 2 (subjects progressing to RIFLE R, I or F) was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 1.

Example 7. Kidney injury markers for evaluating renal status in patients at RIFLE Stages 0 and R

Patients were classified and analyzed as described in Example 6. However, patients that reached stage R but did not progress to stage I or F were grouped with patients from non-injury stage 0 in Cohort 1. Cohort 2 in this example included only patients that progressed to stage I or F. Marker concentrations in urine samples were included for Cohort 1. Marker concentrations in urine samples collected within 0, 24, and 48 hours of reaching stage I or F were included for Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 2.

Example 8. Kidney injury markers for evaluating renal status in patients progressing from Stage R to Stages I and F

Patients were classified and analyzed as described in Example 6, but only those patients that reached Stage R were included in this example. Cohort 1 contained patients that reached stage R but did not progress to stage I or F within 10 days, and Cohort 2 included only patients that progressed to stage I or F. Marker concentrations in urine samples collected within 12 hours of reaching stage R were included in the analysis for both Cohort 1 and 2.

The ability to distinguish cohort 1 (subjects remaining in RIFLE R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 3.

Example 9. Kidney injury markers for evaluating renal status in patients at RIFLE Stage 0

Patients were classified and analyzed as described in Example 6. However, patients that reached stage R or I but did not progress to stage F were eliminated from the analysis. Patients from non-injury stage 0 are included in Cohort 1. Cohort 2 in this example included only patients that progressed to stage F. The maximum marker concentrations in urine samples were included for each patient in Cohort 1. The maximum marker concentrations in urine samples collected within 0, 24, and 48 hours of reaching stage F were included for each patient in Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 4.

Example 10. Kidney injury markers for evaluating renal status in patients at RIFLE Stage 0

Patients from the intensive care unit (ICU) were classified by kidney status as non-injury (0), risk of injury (R), injury (I), and failure (F) according to the maximum stage reached within 7 days of enrollment as determined by the RIFLE criteria.

Two cohorts were defined as (Cohort 1) patients that did not progress beyond stage 0, and (Cohort 2) patients that reached stage R, I, or F within 10 days. To address normal marker fluctuations that occur within patients at the ICU and thereby assess utility for monitoring AKI status, marker levels were measured in the plasma component of blood samples collected for Cohort 1. Marker concentrations were measured in the plasma component of blood samples collected from a subject at 0, 24 hours, and 48 hours prior to reaching stage R, I or F in Cohort 2. In the following tables, the time "prior max stage" represents the time at which a sample is collected, relative to the time a particular patient reaches the lowest disease stage as defined for that cohort, binned into three groups which are +/- 12 hours. For example, 24 hr prior for this example (0 vs R, I, F) would mean 24 hr (+/- 12 hours) prior to reaching stage R (or I if no sample at R, or F if no sample at R or I).

Each marker was measured by standard immunoassay methods using commercially available assay reagents. A receiver operating characteristic (ROC) curve was generated for each marker and the area under each ROC curve (AUC) was determined. Patients in Cohort 2 were also separated according to the reason for adjudication to stage R, I, or F as being based on serum creatinine measurements (sCr), being based on urine output (UO), or being based on either serum creatinine measurements or urine output. That is, for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, for those patients adjudicated to stage R, I, or F on the basis of serum creatinine measurements or urine output, the adjudication method which yielded the most severe RIFLE stage was used.

The ability to distinguish cohort 1 (subjects remaining in RIFLE 0) from Cohort 2 (subjects progressing to RIFLE R, I or F) was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 5.

Example 11. Kidney injury markers for evaluating renal status in patients at RIFLE Stages 0 and R

Patients were classified and analyzed as described in Example 10. However, patients that reached stage R but did not progress to stage I or F were grouped with patients from non-injury stage 0 in Cohort 1. Cohort 2 in this example included only patients that progressed to stage I or F. Marker concentrations in the plasma component of blood samples were included for Cohort 1. Marker concentrations in the plasma component of blood samples collected within 0, 24, and 48 hours of reaching stage I or F were included for Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 6.

Example 12. Kidney injury markers for evaluating renal status in patients progressing from Stage R to Stages I and F

Patients were classified and analyzed as described in Example 10, but only those patients that reached Stage R were included in this example. Cohort 1 contained patients that reached stage R but did not progress to stage I or F within 10 days, and Cohort 2 included only patients that progressed to stage 1 or F. Marker concentrations in the plasma component of blood samples collected within 12 hours of reaching stage R were included in the analysis for both Cohort 1 and 2.

The ability to distinguish cohort 1 (subjects remaining in RIFLE R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 7.

Example 13. Kidney injury markers for evaluating renal status in patients at RIFLE Stage 0

Patients were classified and analyzed as described in Example 10. However, patients that reached stage R or I but did not progress to stage F were eliminated from the analysis. Patients from non-injury stage 0 are included in Cohort 1. Cohort 2 in this example included only patients that progressed to stage F. The maximum marker concentrations in the plasma component of blood samples were included from each patient in Cohort 1. The maximum marker concentrations in the plasma component of blood samples collected within 0, 24, and 48 hours of reaching stage F were included from each patient in Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 8.

The invention illustratively described herein suitably may be practiced in the absence of any element or elements, limitation or limitations which is not specifically disclosed herein. Thus, for example, in each instance herein any of the terms "comprising" and "consisting essentially of" may be replaced with either of the other two terms.

**Claims**

**1.** A method for evaluating renal status in a subject, comprising:

performing one or more assays configured to detect a kidney injury marker on a body fluid sample obtained from the subject to provide one or more assay results, the kidney injury marker being Epidermal growth factor and optionally further one or more of Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein; and

correlating the assay result(s) to one or more of risk stratification, staging, prognosis, classifying and monitoring of the renal status of the subject, wherein said correlating step comprises assigning a likelihood of future acute renal failure (ARF) suffered by the subject, and optionally one or more future changes in renal status to the subject, based on the assay result(s).

2. A method according to claim 1, wherein said assay result(s) comprise:

(i) a measured concentration of Epidermal growth factor, and optionally further one or more of:
(ii) a measured concentration of Complement C3,
(iii) a measured concentration of Interleukin-4,
(iv) a measured concentration of Interleukin-1 alpha,
(v) a measured concentration of Tubulointerstitial nephritis antigen,
(vi) a measured concentration of Transforming growth factor beta-1,
(vii) a measured concentration of Bone morphogenetic protein 7,
(viii) a measured concentration of Osteopontin,
(ix) a measured concentration of Netrin-1, and
(x) a measured concentration of Growth-regulated alpha protein,
and wherein said correlation step comprises, for each assay result, comparing said measure concentration to a threshold concentration, and

for a positive going marker, assigning an increased likelihood of suffering future ARF, to the subject when the measured concentration is above the threshold, relative to a likelihood assigned when the measured concentration is below the threshold or assigning a decreased likelihood of suffering future ARF to the subject when the measured concentration is below the threshold, relative to a likelihood assigned when the measured concentration is above the threshold, or

for a negative going marker, assigning an increased likelihood of suffering future ARF to the subject when the measured concentration is below the threshold, relative to a likelihood assigned when the measured concentration is above the threshold or assigning a decreased likelihood of suffering future ARF to the subject when the measured concentration is above the threshold, relative to a likelihood assigned when the measured concentration is below the threshold.

3. A method according to claim 1, wherein the likelihood of future ARF and optionally one or more future changes in renal status is that an event of interest is more or less likely to occur within a period selected from the group consisting of 30 days, 21 days, 14 days, 7 days, 5 days, 96 hours, 72 hours, 48 hours, 36 hours, 24 hours, and 12 hours.

4. A method according to claim 1, wherein the subject is selected for evaluation of renal status based on (i) the pre-existence in the subject of one or more known risk factors for prerenal, intrinsic renal, or postrenal ARF or (ii) an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

5. A method according to claim 1, wherein said method is a method of assigning a risk of

(i) the future occurrence or nonoccurrence of acute renal failure in said subject,
(ii) the future occurrence or nonoccurrence of a need for renal replacement therapy in said subject or
(iii) the future occurrence or nonoccurrence of a need for renal transplantation in said subject.

6. The method according to claim 2, wherein said one or more future changes in renal status comprise future acute renal failure (ARF) within 72 hours, within 48 hours or within 24 hours of the time at which the body fluid sample is obtained.

7. Use of a kidney injury marker for prognosis of future acute renal failure in vitro, wherein the kidney injury marker is Epidermal growth factor, and optionally further one or more of Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein.

**Patentansprüche**

1. Verfahren zum Evaluieren des Nierenstatus eines Subjekts, aufweisend:

ein oder mehrere Untersuchungstestverfahren durchführen, die dazu konfiguriert sind, in einer Körperflüssigkeitsprobe, die dem Subjekt entstammt, einen Nierenschädigungs-Marker zu detektieren, um ein oder mehrere Untersuchungs-Ergebnisse bereit zu stellen, wobei der Nierenschädigungs-Marker der Epidermale Wachstumsfaktor ist und optional weiterhin einer oder mehrere von Complement C3, Interleukin-4, Interleukin-1 alpha, Tubointerstitielles Nephritis-Antigen, Transformierender Wachstumsfaktor beta-1, knochenmorphogenetisches Protein 7, Osteopontin, Netrin-1, und Wachstumsreguliertes Alpha-Protein und das/die Untersuchungs-Ergebnis(se) mit einem oder mehreren von Risikostratifizierung, Einstufen des Erkrankungsstadiums (Staging), Prognose, Klassifizierung und Überwachen des Nierenstatus des Subjekts zu korrelieren, wobei besagter Korrelierungs-Schritt aufweist, auf der Grundlage des/der Untersuchungs-Ergebnisse(s) eine Wahrscheinlichkeit von durch das Subjekt erlittener zukünftiger akuter Niereninsuffizienz (ARF) umfassen/ umfasst, sowie optional eine oder mehrere zukünftige(n) Veränderung(en) im Nierenstatus des Subjekts.

2. Verfahren nach Anspruch 1, wobei das/die Untersuchungs-Ergebnis(se) umfasst/umfassen:

i) eine gemessene Konzentration des Epidermale Wachstumsfaktors und optional weiterhin einen oder mehrere von:
ii) eine gemessene Konzentration von Complement C3, , und
iii) eine gemessene Konzentration von Interleukin-4,
iv) eine gemessene Konzentration von Interleukin-1 alpha,
v) eine gemessene Konzentration von Tubointerstitielles Nephritis-Antigen,
vi) eine gemessene Konzentration von Transformierender Wachstumsfaktor beta-1,
vii) eine gemessene Konzentration von knochenmorphogenetisches Protein 7,
viii) eine gemessene Konzentration von Osteopontin,
ix) eine gemessene Konzentration von Netrin-1,
x) eine gemessene Konzentration von Wachstumsreguliertes Alpha-Protein und besagter Korrelierungs-Schritt aufweist, für jedes Untersuchungs-Ergebnis die gemessene Konzentration mit einer Schwellenkonzentration zu vergleichen und
bei einem positiver werdenden Marker, wenn die gemessene Konzentration über dem Schwellenwert liegt, eine erhöhte Wahrscheinlichkeit bestimmen, dass das Subjekt in der Zukunft ARF erleiden wird, im Vergleich zu einer Wahrscheinlichkeit, die bestimmt wird, wenn die gemessene Konzentration unter dem Schwellenwert liegt, oder wenn die gemessene Konzentration unter dem Schwellenwert liegt, eine verringerte Wahrscheinlichkeit bestimmen, dass das Subjekt in der Zukunft ARF erleiden wird, im Vergleich zu einer Wahrscheinlichkeit, die bestimmt wird, wenn die gemessene Konzentration über dem Schwellenwert liegt, oder bei einem negativer werdenden Marker, wenn die gemessene Konzentration unter dem Schwellenwert liegt, eine erhöhte Wahrscheinlichkeit bestimmen, dass das Subjekt in der Zukunft ARF erleiden wird, im Vergleich zu einer Wahrscheinlichkeit, die bestimmt wird, wenn die gemessene Konzentration über dem Schwellenwert liegt, oder wenn die gemessene Konzentration über dem Schwellenwert liegt, eine verringerte Wahrscheinlichkeit bestimmen, dass das Subjekt in der Zukunft ARF erleiden wird, im Vergleich zu einer Wahrscheinlichkeit, die bestimmt wird, wenn die gemessene Konzentration unter dem Schwellenwert liegt.

3. Verfahren nach Anspruch 1, wobei die Wahrscheinlichkeit für zukünftiges ARF und optional einer oder mehrere zukünftige Veränderungen im Nierenstatus ist, dass ein Vorfall von Interesse mehr oder weniger wahrscheinlich innerhalb eines Zeitraums auftreten wird, der aus der Gruppe ausgewählt ist, die aus 30 Tagen, 21 Tagen, 7 Tagen, 5 Tagen, 96 Stunden, 72 Stunden, 48 Stunden, 36 Stunden, 24 Stunden und 12 Stunden besteht.

4. Verfahren nach Anspruch 1, wobei das Subjekt zum Evaluieren des Nierenstatus ausgewählt wird auf der Grundlage von (i) dem Bestehen von einem oder mehreren bereits bestehenden bekannten Risikofaktoren des Subjekts für prärenales, immanentrenales, oder postrenales ARF, oder ii) einer bestehenden Diagnose von einem oder mehreren von kongestiver Herzinsuffizienz, Präeklampsie, Eklampsie, Diabetes mellitus, Hypertonie, Koronar-Arterienerkrankung, Proteinurie, Niereninsuffizienz, einer glomerulären Filtration unterhalb des normalen Bereichs, Zirrhose, Serum-Kreatinin oberhalb des normalen Bereichs, Sepsis, Störung der Nierenfunktion, verminderte Nierenfunktion oder ARF, oder auf der Grundlage eines laufenden oder vorangegangenen größeren gefäßchirurgischen Eingriffs, einer laufenden oder vorangegangenen Koronararterien-Bypassoperation oder einer sonstigen laufenden oder vorangegangenen Herzchirurgie, oder auf der auf der Grundlage einer Exposition zu NSAIDs, Cyclosporinen, Tacrolimus, Aminoglycosiden, Foscarnet, Ethylenglykol, Hämoglobin, Myoglobin, Ifosfamid, Schwermetallen, Methotrexat, von röntgendichten Kontrastmitteln oder Streptozotocin.

5. Verfahren nach Anspruch 1, wobei besagtes Verfahren ein Verfahren zum Bestimmen des Risikos

i) des zukünftigen Auftretens oder Nichtauftretens von akuter Niereninsuffizienz beim besagten Subjekt ist,
ii) des zukünftigen Auftretens oder Nichtauftretens eines Bedarfs einer Nierenersatztherapie beim besagten Subjekt ist, oder
iii) eines zukünftigen Auftretens oder Nichtauftretens eines Bedarfs einer Nierentransplantation beim besagten Subjekt ist.

6. Verfahren nach Anspruch 2, wobei besagte eine oder mehrere zukünftige Veränderung(en) im Nierenstatus aufweisen: zukünftige akute Niereninsuffizienz (ARF) innerhalb von 72 Stunden, innerhalb von 48 Stunden oder innerhalb von 24 Stunden ab dem Zeitpunkt zu dem die Körperflüssigkeitsprobe vom Subjekt gewonnen wird.

7. Verwendung eines Nierenschädigungs-Markers zur Prognose von zukünftiger akuter Niereninsuffizienz in vitro, wobei der Nierenschädigungs-Marker der Epidermale Wachstumsfaktor ist und optional weiterhin einer oder mehrere von Complement C3, Interleukin-4, Interleukin-1 alpha, Tubointerstitielles Nephritis-Antigen, Transformierender Wachstumsfaktor beta-1, knochenmorphogenetisches Protein 7, Osteopontin, Netrin-1, und Wachstumsreguliertes Alpha-Protein.

**Revendications**

1. Procédé d'évaluation de l'état des reins chez un sujet, comprenant :

la réalisation d'un ou de plusieurs dosages configurés pour détecter un marqueur de lésion rénale sur un échantillon de fluide corporel obtenu du sujet pour fournir un ou plusieurs résultats de dosage, le marqueur de lésion rénale étant un facteur de croissance épidermique et
éventuellement encore un ou plusieurs parmi le complément C3, l'interleukine-4, l'interleukine-1 alpha, un antigène de néphrite tubulo-interstitielle, le facteur de croissance transformant bêta-1, la protéine morphogénétique osseuse 7, l'ostéopontine, la nétrine-1, et la protéine alpha régulée par la croissance ; et
la corrélation du/des résultat(s) de dosage à un(e) ou plusieurs parmi une stratification des risques, une stadification, un pronostic, une classification et une surveillance de l'état des reins du sujet, dans lequel ladite étape de corrélation comprend d'assigner une probabilité d'insuffisance rénale aiguë (IRA) future soufferte par le sujet, et éventuellement d'un ou de plusieurs changements futurs de l'état des reins au sujet, en fonction du/des résultat(s) de dosage.

2. Procédé selon la revendication 1, dans lequel ledit/lesdits résultat(s) de dosage comprennent :

(i) une concentration mesurée de facteur de croissance épidermique, et éventuellement encore un ou plusieurs parmi :
(ii) une concentration mesurée de complément C3,
(iii) une concentration mesurée d'interleukine-4,
(iv) une concentration mesurée d'interleukine-1 alpha,
(v) une concentration mesurée d'antigène de néphrite tubulo-interstitielle,
(vi) une concentration mesurée de facteur de croissance transformant bêta-1,
(vii) une concentration mesurée de protéine morphogénétique osseuse 7,
(viii) une concentration mesurée d'ostéopontine,
(ix) une concentration mesurée de nétrine-1, et
(x) une concentration mesurée de protéine alpha régulée par la croissance,
et dans lequel ladite étape de corrélation comprend, pour chaque résultat de dosage, de comparer ladite concentration de mesure à une concentration seuil, et

pour un marqueur montant, d'assigner au sujet une probabilité augmentée de souffrir d'une IRA future quand la concentration mesurée est supérieure au seuil, par rapport à une probabilité assignée quand la concentration mesurée est inférieure au seuil ou d'assigner au sujet une probabilité diminuée de souffrir d'une IRA future quand la concentration mesurée est inférieure au seuil, par rapport à une probabilité assignée quand la concentration mesurée est supérieure au seuil, ou

pour un marqueur descendant, d'assigner au sujet une probabilité augmentée de souffrir d'une IRA future quand la concentration mesurée est inférieure au seuil, par rapport à une probabilité assignée quand la concentration mesurée est supérieure au seuil ou d'assigner au sujet une probabilité diminuée de souffrir d'une IRA future quand la concentration mesurée est supérieure au seuil, par rapport à une probabilité assignée quand la concentration mesurée est inférieure au seuil.

3. Procédé selon la revendication 1, dans lequel la probabilité d'IRA future et éventuellement d'un ou de plusieurs changements futurs dans l'état des reins est qu'un événement d'intérêt est plus ou moins à même de se produire dans une période sélectionnée parmi le groupe constitué de 30 jours, 21 jours, 14 jours, 7 jours, 5 jours, 96 heures, 72 heures, 48 heures, 36 heures, 24 heures, et 12 heures.

4. Procédé selon la revendication 1, dans lequel le sujet est sélectionné pour une évaluation de l'état des reins en fonction de (i) la préexistence chez le sujet d'un ou de plusieurs facteurs de risque connus pour une IRA pré-rénale, rénale intrinsèque, ou post-rénale, ou (ii) d'un diagnostic existant d'un(e) ou de plusieurs parmi une insuffisance cardiaque congestive, une pré-éclampsie, une éclampsie, un diabète sucré, une hypertension, une coronaropathie, une protéinurie, une insuffisance rénale, une filtration glomérulaire inférieure à la plage normale, une cirrhose, de la créatinine sérique supérieure à la plage normale, une sepsie, une lésion de la fonction rénale, une fonction rénale réduite, ou une IRA, ou en fonction du fait de subir ou d'avoir subi une intervention chirurgicale vasculaire majeure, un pontage aorto-coronarien, ou une autre intervention cardiaque, ou en fonction de l'exposition à des AINS, à des cyclosporines, au tacrolimus, à des aminoglycosides, au foscarnet, à de l'éthylèneglycol, à l'hémoglobine, à la myoglobine, à l'ifosfamide, à des métaux lourds, au méthotrexate, à des agents de contraste radio-opaque, ou à de la streptozotocine.

5. Procédé selon la revendication 1, dans lequel ledit procédé est un procédé d'assignation d'un risque de

(i) survenue ou de non survenue future d'une insuffisance rénale aiguë chez ledit sujet,
(ii) survenue ou de non survenue future d'un besoin pour une thérapie de remplacement rénal chez ledit sujet ou
(iii) survenue ou de non survenue future d'un besoin pour une transplantation rénale chez ledit sujet.

6. Procédé selon la revendication 2, dans lequel lesdits un ou plusieurs changements futurs dans l'état des reins comprennent une insuffisance rénale aiguë (IRA) future dans les 72 heures, dans les 48 heures ou dans les 24 heures suivant l'heure à laquelle l'échantillon de fluide corporel est obtenu.

7. Utilisation d'un marqueur de lésion rénale pour le pronostic d'une insuffisance rénale aiguë future in vitro, dans laquelle le marqueur de lésion rénale est le facteur de croissance épidermique, et éventuellement encore un ou plusieurs parmi le complément C3, l'interleukine-4, l'interleukine-1 alpha, un antigène de néphrite tubulo-interstitielle, le facteur de croissance transformant bêta-1, la protéine morphogénétique osseuse 7, l'ostéopontine, la nétrine-1, et la protéine alpha régulée par la croissance.

**Epidermal growth factor**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 21200.000 | 21000.000 | 21200.000 | 18250.000 | 21200.000 | 16400.000 |
| average | 29128.823 | 26307.472 | 29128.823 | 25123.823 | 29128.823 | 25655.556 |
| stdev | 26105.765 | 19448.924 | 26105.765 | 21623.583 | 26105.765 | 26288.037 |
| p (t-test) | | 0.458 | | 0.265 | | 0.512 |
| min | 921.000 | 100.000 | 921.000 | 2370.000 | 921.000 | 1750.000 |
| max | 107000.000 | 88986.000 | 107000.000 | 109224.000 | 107000.000 | 91500.000 |
| n (Samp) | 248 | 53 | 248 | 62 | 248 | 27 |
| n (Pat) | 103 | 53 | 103 | 62 | 103 | 27 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 21450.000 | 9920.000 | 21450.000 | 14900.000 | 21450.000 | 13800.000 |
| average | 28545.714 | 16432.500 | 28545.714 | 19541.923 | 28545.714 | 17370.714 |
| stdev | 24861.585 | 15135.240 | 24861.585 | 18311.899 | 24861.585 | 12079.170 |
| p (t-test) | | 0.031 | | 0.070 | | 0.095 |
| min | 100.000 | 2340.000 | 100.000 | 2370.000 | 100.000 | 2430.000 |
| max | 109224.000 | 65700.000 | 109224.000 | 69200.000 | 109224.000 | 45900.000 |
| n (Samp) | 440 | 20 | 440 | 26 | 440 | 14 |
| n (Pat) | 169 | 20 | 169 | 26 | 169 | 14 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 17200.000 | 21000.000 | 17200.000 | 18400.000 | 17200.000 | 19700.000 |
| average | 26450.340 | 27525.660 | 26450.340 | 26293.788 | 26450.340 | 26647.200 |
| stdev | 24890.352 | 20439.864 | 24890.352 | 22281.043 | 24890.352 | 27017.196 |
| p (t-test) | | 0.783 | | 0.967 | | 0.970 |
| min | 921.000 | 100.000 | 921.000 | 2610.000 | 921.000 | 1750.000 |
| max | 107000.000 | 88986.000 | 107000.000 | 109224.000 | 107000.000 | 91500.000 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.51 | 0.044 | 248 | 53 | 0.749 |
| 24 hours | 0.48 | 0.041 | 248 | 62 | 0.702 |
| 48 hours | 0.46 | 0.057 | 248 | 27 | 0.459 |

sCr only

| Time prior AKI stage | AUC | SE | nCohort 1 | nCohort 2 | p |
|---|---|---|---|---|---|
| 0 hours | 0.35 | 0.057 | 440 | 20 | 0.010 |
| 24 hours | 0.39 | 0.053 | 440 | 26 | 0.046 |
| 48 hours | 0.39 | 0.071 | 440 | 14 | 0.135 |

UO only

| Time prior AKI stage | AUC | SE | nCohort 1 | nCohort 2 | p |
|---|---|---|---|---|---|
| 0 hours | 0.56 | 0.047 | 212 | 47 | 0.228 |
| 24 hours | 0.53 | 0.045 | 212 | 52 | 0.486 |
| 48 hours | 0.49 | 0.061 | 212 | 25 | 0.885 |

sCr or UO

| Time prior AKI stage | Cutoff value | sens | spec | Quartile | OR | 95% CI of OR | |
|---|---|---|---|---|---|---|---|
| 0 hours | 11500 | 74% | 33% | 1 | | | |
| | 9950 | 81% | 31% | 2 | 3.5 | 2.3 | 5.5 |
| | 7100 | 91% | 22% | 3 | 2.6 | 1.7 | 4.2 |
| | 37300 | 21% | 70% | 4 | 1.5 | 0.9 | 2.5 |
| | 50200 | 13% | 80% | | | | |
| | 72400 | 6% | 90% | | | | |
| 24 hours | 11700 | 71% | 34% | 1 | | | |

## FIG. 1 - 1

| Time prior AKI stage | Cutoff value | sens | spec | Quartile | OR | 95% CI of OR | |
|---|---|---|---|---|---|---|---|
| | 9950 | 81% | 31% | 2 | 2.1 | 1.4 | 3.0 |
| | 4170 | 90% | 10% | 3 | 2.8 | 2.0 | 4.0 |
| | 37300 | 19% | 70% | 4 | 1.1 | 0.7 | 1.7 |
| | 50200 | 11% | 80% | | | | |
| | 72400 | 3% | 90% | | | | |
| 48 hours | 9950 | 70% | 31% | 1 | | | |
| | 6520 | 81% | 21% | 2 | 1.2 | 0.6 | 2.7 |
| | 2990 | 93% | 6% | 3 | 1.9 | 1.0 | 3.8 |
| | 37300 | 19% | 70% | 4 | 1.5 | 0.7 | 3.1 |
| | 50200 | 15% | 80% | | | | |
| | 72400 | 11% | 90% | | | | |

sCr only

| Time prior AKI stage | Cutoff value | sens | spec | Quartile | OR | 95% CI of OR | |
|---|---|---|---|---|---|---|---|
| 0 hours | 9110 | 70% | 25% | 1 | | | |
| | 5950 | 80% | 16% | 2 | 5.2 | 0.5 | 56.4 |
| | 4310 | 90% | 10% | 3 | 8.5 | 0.9 | 80.1 |
| | 34800 | 5% | 70% | 4 | 6.3 | 0.6 | 64.0 |
| | 44600 | 5% | 80% | | | | |
| | 67100 | 0% | 90% | | | | |
| 24 hours | 6870 | 73% | 18% | 1 | | | |
| | 6380 | 81% | 17% | 2 | 1.4 | 0.4 | 4.4 |
| | 4170 | 92% | 10% | 3 | 3.2 | 1.3 | 7.8 |
| | 34800 | 15% | 70% | 4 | 3.6 | 1.5 | 8.7 |
| | 44600 | 12% | 80% | | | | |
| | 67100 | 4% | 90% | | | | |
| 48 hours | 11200 | 71% | 30% | 1 | | | |
| | 6080 | 86% | 16% | 2 | 3.1 | 0.2 | 43.6 |
| | 5460 | 93% | 14% | 3 | 7.4 | 0.8 | 72.0 |
| | 34800 | 7% | 70% | 4 | 3.1 | 0.2 | 43.6 |
| | 44600 | 7% | 80% | | | | |
| | 67100 | 0% | 90% | | | | |

UO only

| Time prior AKI stage | Cutoff value | sens | spec | Quartile | OR | 95% CI of OR | |
|---|---|---|---|---|---|---|---|
| 0 hours | 14300 | 70% | 43% | 1 | | | |
| | 10800 | 81% | 34% | 2 | 2.4 | 1.4 | 4.2 |
| | 6580 | 91% | 20% | 3 | 2.9 | 1.7 | 4.9 |
| | 30100 | 38% | 70% | 4 | 2.4 | 1.4 | 4.2 |
| | 43300 | 19% | 80% | | | | |
| | 66600 | 6% | 90% | | | | |
| 24 hours | 11700 | 71% | 36% | 1 | | | |
| | 10300 | 81% | 33% | 2 | 3.2 | 2.0 | 5.0 |
| | 4310 | 90% | 11% | 3 | 2.5 | 1.5 | 4.0 |
| | 30100 | 35% | 70% | 4 | 1.9 | 1.1 | 3.1 |
| | 43300 | 15% | 80% | | | | |
| | 66600 | 6% | 90% | | | | |
| 48 hours | 9410 | 72% | 31% | 1 | | | |
| | 6580 | 80% | 20% | 2 | 1.7 | 0.8 | 3.5 |
| | 2990 | 92% | 6% | 3 | 1.0 | 0.4 | 2.4 |
| | 30100 | 28% | 70% | 4 | 1.5 | 0.7 | 3.1 |
| | 43300 | 16% | 80% | | | | |
| | 66600 | 12% | 90% | | | | |

FIG. 1 - 2

**Interleukin-1 alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.002 | 0.001 | 0.002 | 0.001 | 0.002 | 0.001 |
| average | 0.002 | 0.001 | 0.002 | 0.002 | 0.002 | 0.001 |
| stdev | 0.004 | 0.001 | 0.004 | 0.002 | 0.004 | 0.001 |
| p (t-test) | | 0.042 | | 0.318 | | 0.250 |
| min | 0.000 | 0.000 | 0.000 | 0.000 | 0.000 | 0.000 |
| max | 0.038 | 0.005 | 0.038 | 0.015 | 0.038 | 0.004 |
| n (Samp) | 248 | 53 | 248 | 62 | 248 | 27 |
| n (Pat) | 103 | 53 | 103 | 62 | 103 | 27 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.001 | 0.002 | 0.001 | 0.002 | 0.001 | 0.002 |
| average | 0.002 | 0.002 | 0.002 | 0.002 | 0.002 | 0.002 |
| stdev | 0.004 | 0.003 | 0.004 | 0.002 | 0.004 | 0.001 |
| p (t-test) | | 0.881 | | 0.637 | | 0.664 |
| min | 0.000 | 0.000 | 0.000 | 0.000 | 0.000 | 0.000 |
| max | 0.038 | 0.012 | 0.038 | 0.007 | 0.038 | 0.003 |
| n (Samp) | 440 | 20 | 440 | 26 | 440 | 14 |
| n (Pat) | 169 | 20 | 169 | 26 | 169 | 14 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.002 | 0.000 | 0.002 | 0.001 | 0.002 | 0.001 |
| average | 0.002 | 0.001 | 0.002 | 0.002 | 0.002 | 0.001 |
| stdev | 0.003 | 0.003 | 0.003 | 0.002 | 0.003 | 0.001 |
| p (t-test) | | 0.176 | | 0.320 | | 0.345 |
| min | 0.000 | 0.000 | 0.000 | 0.000 | 0.000 | 0.000 |
| max | 0.033 | 0.017 | 0.033 | 0.015 | 0.033 | 0.004 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.38 | 0.040 | 248 | 53 | 0.003 |
| 24 hours | 0.44 | 0.040 | 248 | 62 | 0.112 |
| 48 hours | 0.43 | 0.056 | 248 | 27 | 0.204 |

sCr only

| Time prior AKI stage | AUC | SE | nCohort 1 | nCohort 2 | p |
|---|---|---|---|---|---|
| 0 hours | 0.55 | 0.068 | 440 | 20 | 0.504 |
| 24 hours | 0.60 | 0.060 | 440 | 26 | 0.106 |
| 48 hours | 0.52 | 0.079 | 440 | 14 | 0.816 |

UO only

| Time prior AKI stage | AUC | SE | nCohort 1 | nCohort 2 | p |
|---|---|---|---|---|---|
| 0 hours | 0.38 | 0.043 | 212 | 47 | 0.004 |
| 24 hours | 0.42 | 0.043 | 212 | 52 | 0.069 |
| 48 hours | 0.45 | 0.059 | 212 | 25 | 0.391 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.9 | 0.5 | 1.5 |
| | 0 | 100% | 0% | 3 | 2.5 | 1.7 | 3.7 |
| | 0.00249 | 17% | 70% | 4 | 2.2 | 1.5 | 3.2 |
| | 0.003 | 11% | 81% | | | | |
| | 0.00419 | 2% | 90% | | | | |
| 24 hours | 0 | 100% | 0% | 1 | | | |

# FIG. 1 - 3

| | Cutoff value | sens | spec | Quartile | OR | 95% CI of OR | |
|---|---|---|---|---|---|---|---|
| | 0 | 100% | 0% | 2 | 1.0 | 0.7 | 1.5 |
| | 0 | 100% | 0% | 3 | 2.2 | 1.6 | 3.0 |
| | 0.00249 | 21% | 70% | 4 | 1.4 | 1.0 | 2.0 |
| | 0.003 | 16% | 81% | | | | |
| | 0.00419 | 6% | 90% | | | | |
| 48 hours | 0 | 100% | 0% | 1 | | | |
| | 0 | 100% | 0% | 2 | 1.8 | 0.8 | 4.2 |
| | 0 | 100% | 0% | 3 | 3.1 | 1.5 | 6.4 |
| | 0.00249 | 15% | 70% | 4 | 1.3 | 0.5 | 3.3 |
| | 0.003 | 7% | 81% | | | | |
| | 0.00419 | 0% | 90% | | | | |

sCr only

| Time prior AKI stage | Cutoff value | sens | spec | Quartile | OR | 95% CI of OR | |
|---|---|---|---|---|---|---|---|
| 0 hours | 0.000709 | 70% | 42% | 1 | | | |
| | 0 | 100% | 0% | 2 | 1.0 | 0.4 | 2.3 |
| | 0 | 100% | 0% | 3 | 0.6 | 0.2 | 1.7 |
| | 0.00231 | 35% | 70% | 4 | 1.4 | 0.7 | 2.9 |
| | 0.00288 | 20% | 80% | | | | |
| | 0.00382 | 15% | 90% | | | | |
| 24 hours | 0.00132 | 73% | 47% | 1 | | | |
| | 0 | 100% | 0% | 2 | 8.4 | 0.9 | 79.3 |
| | 0 | 100% | 0% | 3 | 8.5 | 0.9 | 80.0 |
| | 0.00231 | 38% | 70% | 4 | 9.6 | 1.0 | 87.6 |
| | 0.00288 | 35% | 80% | | | | |
| | 0.00382 | 19% | 90% | | | | |
| 48 hours | 0.00098 | 71% | 44% | 1 | | | |
| | 0 | 100% | 0% | 2 | na | na | na |
| | 0 | 100% | 0% | 3 | na | na | na |
| | 0.00231 | 36% | 70% | 4 | na | na | na |
| | 0.00288 | 14% | 80% | | | | |
| | 0.00382 | 0% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.9 | 0.5 | 1.6 |
| | 0 | 100% | 0% | 3 | 1.3 | 0.8 | 2.2 |
| | 0.00246 | 17% | 71% | 4 | 3.7 | 2.5 | 5.7 |
| | 0.00289 | 13% | 80% | | | | |
| | 0.00382 | 4% | 91% | | | | |
| 24 hours | 0 | 100% | 0% | 1 | | | |
| | 0 | 100% | 0% | 2 | 0.9 | 0.5 | 1.4 |
| | 0 | 100% | 0% | 3 | 1.1 | 0.7 | 1.7 |
| | 0.00246 | 21% | 71% | 4 | 2.8 | 1.9 | 4.0 |
| | 0.00289 | 17% | 80% | | | | |
| | 0.00382 | 6% | 91% | | | | |
| 48 hours | 0 | 100% | 0% | 1 | | | |
| | 0 | 100% | 0% | 2 | 3.0 | 1.1 | 7.9 |
| | 0 | 100% | 0% | 3 | 4.9 | 2.0 | 11.8 |
| | 0.00246 | 12% | 71% | 4 | 0.7 | 0.1 | 3.7 |
| | 0.00289 | 8% | 80% | | | | |
| | 0.00382 | 8% | 91% | | | | |

**FIG. 1 - 4**

**Interleukin-4**

sCr or UO

| | 0 hr prior to AKI stage Cohort 1 | Cohort 2 | 24 hr prior to AKI stage Cohort 1 | Cohort 2 | 48 hr prior to AKI stage Cohort 1 | Cohort 2 |
|---|---|---|---|---|---|---|
| median | 24.850 | 23.300 | 24.850 | 30.350 | 24.850 | 30.700 |
| average | 24.220 | 22.682 | 24.220 | 28.865 | 24.220 | 31.232 |
| stdev | 10.766 | 12.414 | 10.766 | 16.262 | 10.766 | 15.311 |
| p (t-test) | | 0.359 | | 0.007 | | 0.002 |
| min | 0.416 | 0.416 | 0.416 | 0.416 | 0.416 | 3.800 |
| max | 57.500 | 50.500 | 57.500 | 62.400 | 57.500 | 66.200 |
| n (Samp) | 248 | 53 | 248 | 62 | 248 | 27 |
| n (Pat) | 103 | 53 | 103 | 62 | 103 | 27 |

sCr only

| | 0 hr prior toAKI stage Cohort 1 | Cohort 2 | 24 hr prior toAKI stage Cohort 1 | Cohort 2 | 48 hr prior toAKI stage Cohort 1 | Cohort 2 |
|---|---|---|---|---|---|---|
| median | 25.000 | 20.300 | 25.000 | 30.550 | 25.000 | 30.550 |
| average | 24.662 | 21.280 | 24.662 | 28.436 | 24.662 | 28.234 |
| stdev | 12.158 | 8.364 | 12.158 | 13.167 | 12.158 | 17.358 |
| p (t-test) | | 0.219 | | 0.126 | | 0.287 |
| min | 0.416 | 8.500 | 0.416 | 0.416 | 0.416 | 0.416 |
| max | 62.400 | 44.600 | 62.400 | 59.900 | 62.400 | 66.200 |
| n (Samp) | 440 | 20 | 440 | 26 | 440 | 14 |
| n (Pat) | 169 | 20 | 169 | 26 | 169 | 14 |

UO only

| | 0 hr prior toAKI stage Cohort 1 | Cohort 2 | 24 hr prior toAKI stage Cohort 1 | Cohort 2 | 48 hr prior toAKI stage Cohort 1 | Cohort 2 |
|---|---|---|---|---|---|---|
| median | 25.100 | 24.200 | 25.100 | 32.150 | 25.100 | 29.700 |
| average | 24.341 | 22.423 | 24.341 | 29.692 | 24.341 | 31.292 |
| stdev | 10.969 | 13.530 | 10.969 | 16.254 | 10.969 | 12.377 |
| p (t-test) | | 0.301 | | 0.005 | | 0.003 |
| min | 0.416 | 0.416 | 0.416 | 0.416 | 0.416 | 3.800 |
| max | 57.500 | 50.500 | 57.500 | 62.400 | 57.500 | 61.000 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.47 | 0.043 | 248 | 53 | 0.473 |
| 24 hours | 0.60 | 0.042 | 248 | 62 | 0.018 |
| 48 hours | 0.65 | 0.060 | 248 | 27 | 0.010 |

sCr only

| Time prior AKI stage | AUC | SE | nCohort 1 | nCohort 2 | p |
|---|---|---|---|---|---|
| 0 hours | 0.38 | 0.059 | 440 | 20 | 0.049 |
| 24 hours | 0.60 | 0.060 | 440 | 26 | 0.110 |
| 48 hours | 0.58 | 0.081 | 440 | 14 | 0.322 |

UO only

| Time prior AKI stage | AUC | SE | nCohort 1 | nCohort 2 | p |
|---|---|---|---|---|---|
| 0 hours | 0.47 | 0.046 | 212 | 47 | 0.554 |
| 24 hours | 0.62 | 0.045 | 212 | 52 | 0.006 |
| 48 hours | 0.66 | 0.062 | 212 | 25 | 0.008 |

sCr or UO

| Time prior AKI stage | Cutoff value | sens | spec | Quartile | OR | 95% CI of OR | |
|---|---|---|---|---|---|---|---|
| 0 hours | 16.4 | 72% | 21% | 1 | | | |
| | 9.94 | 81% | 11% | 2 | 0.4 | 0.3 | 0.7 |
| | 3.8 | 92% | 4% | 3 | 0.8 | 0.6 | 1.1 |
| | 29.4 | 32% | 71% | 4 | 1.0 | 0.7 | 1.4 |
| | 32.4 | 26% | 81% | | | | |
| | 36.5 | 13% | 91% | | | | |
| 24 hours | 19.2 | 71% | 30% | 1 | | | |
| | 12.6 | 81% | 14% | 2 | 0.5 | 0.4 | 0.8 |

## FIG. 1 - 5

| Time prior AKI stage | Cutoff value | sens | spec | Quartile | OR | 95% CI of OR | |
|---|---|---|---|---|---|---|---|
| | 8.12 | 90% | 8% | 3 | 0.6 | 0.4 | 0.9 |
| | 29.4 | 53% | 71% | 4 | 2.3 | 1.8 | 3.0 |
| | 32.4 | 45% | 81% | | | | |
| | 36.5 | 29% | 91% | | | | |
| 48 hours | 25.6 | 70% | 56% | 1 | | | |
| | 20.3 | 81% | 34% | 2 | 1.0 | 0.3 | 2.8 |
| | 6.01 | 93% | 7% | 3 | 1.5 | 0.6 | 3.7 |
| | 29.4 | 56% | 71% | 4 | 3.7 | 1.8 | 7.5 |
| | 32.4 | 48% | 81% | | | | |
| | 36.5 | 26% | 91% | | | | |

sCr only

| Time prior AKI stage | Cutoff value | sens | spec | Quartile | OR | 95% CI of OR | |
|---|---|---|---|---|---|---|---|
| 0 hours | 17.8 | 70% | 26% | 1 | | | |
| | 15.7 | 80% | 21% | 2 | 0.7 | 0.1 | 3.5 |
| | 12.7 | 90% | 18% | 3 | 3.2 | 1.3 | 7.9 |
| | 30.6 | 15% | 71% | 4 | 2.1 | 0.7 | 5.7 |
| | 33.6 | 5% | 80% | | | | |
| | 40 | 5% | 90% | | | | |
| 24 hours | 23 | 73% | 43% | 1 | | | |
| | 18.4 | 81% | 28% | 2 | 0.8 | 0.3 | 2.0 |
| | 9.51 | 92% | 12% | 3 | 1.4 | 0.7 | 2.9 |
| | 30.6 | 46% | 71% | 4 | 2.1 | 1.1 | 3.9 |
| | 33.6 | 31% | 80% | | | | |
| | 40 | 15% | 90% | | | | |
| 48 hours | 20.6 | 71% | 34% | 1 | | | |
| | 6.01 | 86% | 10% | 2 | 0.7 | 0.1 | 3.5 |
| | 0.416 | 93% | 5% | 3 | 1.0 | 0.3 | 3.8 |
| | 30.6 | 50% | 71% | 4 | 2.0 | 0.7 | 5.6 |
| | 33.6 | 29% | 80% | | | | |
| | 40 | 21% | 90% | | | | |

UO only

| Time prior AKI stage | Cutoff value | sens | spec | Quartile | OR | 95% CI of OR | |
|---|---|---|---|---|---|---|---|
| 0 hours | 12.5 | 70% | 15% | 1 | | | |
| | 8.12 | 81% | 8% | 2 | 0.5 | 0.3 | 0.7 |
| | 0.416 | 91% | 4% | 3 | 0.4 | 0.2 | 0.7 |
| | 29.4 | 36% | 70% | 4 | 1.2 | 0.9 | 1.7 |
| | 32 | 30% | 80% | | | | |
| | 36.3 | 15% | 90% | | | | |
| 24 hours | 22.3 | 71% | 39% | 1 | | | |
| | 17.3 | 81% | 23% | 2 | 0.4 | 0.2 | 0.7 |
| | 0 | 100% | 0% | 3 | 0.5 | 0.3 | 0.8 |
| | 29.4 | 56% | 70% | 4 | 2.7 | 1.9 | 3.6 |
| | 32 | 50% | 80% | | | | |
| | 36.3 | 35% | 90% | | | | |
| 48 hours | 24.6 | 72% | 48% | 1 | | | |
| | 22.3 | 80% | 39% | 2 | 3.2 | 0.8 | 12.8 |
| | 20.1 | 92% | 31% | 3 | 2.6 | 0.6 | 11.2 |
| | 29.4 | 56% | 70% | 4 | 7.1 | 2.1 | 24.1 |
| | 32 | 48% | 80% | | | | |
| | 36.3 | 24% | 90% | | | | |

# FIG. 1 - 6

**Netrin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 8.800 | 2.990 | 8.800 | 2.814 | 8.800 | 3.387 |
| stdev | 39.131 | 5.006 | 39.131 | 5.383 | 39.131 | 6.989 |
| p (t-test) | | 0.293 | | 0.253 | | 0.484 |
| min | 0.000 | 0.211 | 0.000 | 0.000 | 0.000 | 0.000 |
| max | 418.455 | 21.067 | 418.455 | 26.584 | 418.455 | 31.601 |
| n (Samp) | 118 | 51 | 118 | 57 | 118 | 26 |
| n (Pat) | 99 | 51 | 99 | 57 | 99 | 26 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 1.611 | 0.211 | 0.211 |
| average | 5.955 | 1.008 | 5.955 | 2.990 | 5.955 | 3.605 |
| stdev | 26.936 | 2.929 | 26.936 | 4.754 | 26.936 | 8.568 |
| p (t-test) | | 0.450 | | 0.599 | | 0.745 |
| min | 0.000 | 0.211 | 0.000 | 0.211 | 0.000 | 0.000 |
| max | 418.455 | 12.289 | 418.455 | 21.067 | 418.455 | 31.601 |
| n (Samp) | 257 | 17 | 257 | 23 | 257 | 14 |
| n (Pat) | 160 | 17 | 160 | 23 | 160 | 14 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.246 | 0.211 | 0.246 | 0.211 | 0.246 | 0.211 |
| average | 9.426 | 3.706 | 9.426 | 2.503 | 9.426 | 2.831 |
| stdev | 41.106 | 5.752 | 41.106 | 5.036 | 41.106 | 5.014 |
| p (t-test) | | 0.355 | | 0.248 | | 0.445 |
| min | 0.000 | 0.000 | 0.000 | 0.000 | 0.000 | 0.000 |
| max | 418.455 | 21.067 | 418.455 | 26.584 | 418.455 | 19.312 |
| n (Samp) | 106 | 45 | 106 | 48 | 106 | 23 |
| n (Pat) | 84 | 45 | 84 | 48 | 84 | 23 |

sCr or UO

| Time prior AKI stage | AUC | SE | nCohort 1 | nCohort 2 | p |
|---|---|---|---|---|---|
| 0 hours | 0.47 | 0.048 | 118 | 51 | 0.484 |
| 24 hours | 0.47 | 0.046 | 118 | 57 | 0.468 |
| 48 hours | 0.46 | 0.061 | 118 | 26 | 0.495 |

sCr only

| Time prior AKI stage | AUC | SE | nCohort 1 | nCohort 2 | p |
|---|---|---|---|---|---|
| 0 hours | 0.34 | 0.061 | 257 | 17 | 0.008 |
| 24 hours | 0.54 | 0.064 | 257 | 23 | 0.503 |
| 48 hours | 0.38 | 0.071 | 257 | 14 | 0.082 |

UO only

| Time prior AKI stage | AUC | SE | nCohort 1 | nCohort 2 | p |
|---|---|---|---|---|---|
| 0 hours | 0.47 | 0.051 | 106 | 45 | 0.551 |
| 24 hours | 0.44 | 0.049 | 106 | 48 | 0.188 |
| 48 hours | 0.43 | 0.064 | 106 | 23 | 0.269 |

sCr or UO

| Time prior AKI stage | Cutoff value | sens | spec | Quartile | OR | 95% CI of OR | |
|---|---|---|---|---|---|---|---|
| 0 hours | 1.118E-14 | 100% | 3% | 1 | | | |
| | 1.118E-14 | 100% | 3% | 2 | 1.2 | 0.7 | 1.9 |
| | 1.118E-14 | 100% | 3% | 3 | 1.8 | 1.2 | 2.9 |
| | 3.2216495 | 27% | 70% | 4 | 1.8 | 1.2 | 2.9 |
| | 11.363636 | 10% | 81% | | | | |
| | 21.067416 | 0% | 92% | | | | |
| 24 hours | 1.118E-14 | 98% | 3% | 1 | | | |
| | 1.118E-14 | 98% | 3% | 2 | 1.4 | 0.9 | 2.2 |

# FIG. 1 - 7

| | Cutoff value | sens | spec | Quartile | OR | 95% CI of OR | |
|---|---|---|---|---|---|---|---|
| | 1.118E-14 | 98% | 3% | 3 | 1.7 | 1.1 | 2.6 |
| | 3.2216495 | 26% | 70% | 4 | 1.8 | 1.2 | 2.7 |
| | 11.363636 | 7% | 81% | | | | |
| | 21.067416 | 2% | 92% | | | | |
| 48 hours | 1.118E-14 | 88% | 3% | 1 | | | |
| | 1.118E-14 | 88% | 3% | 2 | 2.3 | 1.0 | 5.4 |
| | 0 | 100% | 0% | 3 | 0.2 | 0.0 | 3.0 |
| | 3.2216495 | 27% | 70% | 4 | 4.5 | 2.1 | 9.9 |
| | 11.363636 | 8% | 81% | | | | |
| | 21.067416 | 4% | 92% | | | | |

sCr only

| Time prior AKI stage | Cutoff value | sens | spec | Quartile | OR | 95% CI of OR | |
|---|---|---|---|---|---|---|---|
| 0 hours | 1.118E-14 | 100% | 3% | 1 | | | |
| | 1.118E-14 | 100% | 3% | 2 | 1.0 | 0.0 | 54.2 |
| | 1.118E-14 | 100% | 3% | 3 | 4.2 | 0.3 | 51.4 |
| | 3.0857271 | 6% | 70% | 4 | 13.1 | 1.5 | 118.6 |
| | 7.5954861 | 6% | 80% | | | | |
| | 15.800562 | 0% | 90% | | | | |
| 24 hours | 1.118E-14 | 100% | 3% | 1 | | | |
| | 1.118E-14 | 100% | 3% | 2 | 0.0 | 0.0 | na |
| | 1.118E-14 | 100% | 3% | 3 | 1.1 | 0.7 | 1.8 |
| | 3.0857271 | 35% | 70% | 4 | 0.4 | 0.2 | 0.9 |
| | 7.5954861 | 9% | 80% | | | | |
| | 15.800562 | 4% | 90% | | | | |
| 48 hours | 1.118E-14 | 79% | 3% | 1 | | | |
| | 0 | 100% | 0% | 2 | 0.3 | 0.0 | 4.7 |
| | 0 | 100% | 0% | 3 | 0.3 | 0.0 | 4.7 |
| | 3.0857271 | 21% | 70% | 4 | 3.4 | 1.3 | 8.6 |
| | 7.5954861 | 14% | 80% | | | | |
| | 15.800562 | 7% | 90% | | | | |

UO only

| Time prior AKI stage | Cutoff value | sens | spec | Quartile | OR | 95% CI of OR | |
|---|---|---|---|---|---|---|---|
| 0 hours | 1.118E-14 | 98% | 3% | 1 | | | |
| | 1.118E-14 | 98% | 3% | 2 | 1.5 | 0.9 | 2.5 |
| | 1.118E-14 | 98% | 3% | 3 | 1.2 | 0.7 | 2.0 |
| | 4.4883303 | 24% | 71% | 4 | 2.0 | 1.2 | 3.3 |
| | 12.289326 | 11% | 80% | | | | |
| | 21.067416 | 0% | 92% | | | | |
| 24 hours | 1.118E-14 | 98% | 3% | 1 | | | |
| | 1.118E-14 | 98% | 3% | 2 | 2.7 | 1.5 | 4.7 |
| | 1.118E-14 | 98% | 3% | 3 | 2.0 | 1.1 | 3.6 |
| | 4.4883303 | 15% | 71% | 4 | 3.0 | 1.7 | 5.2 |
| | 12.289326 | 4% | 80% | | | | |
| | 21.067416 | 2% | 92% | | | | |
| 48 hours | 1.118E-14 | 91% | 3% | 1 | | | |
| | 1.118E-14 | 91% | 3% | 2 | 3.3 | 1.2 | 9.5 |
| | 1.118E-14 | 91% | 3% | 3 | 4.5 | 1.7 | 12.4 |
| | 4.4883303 | 17% | 71% | 4 | 0.7 | 0.1 | 3.9 |
| | 12.289326 | 9% | 80% | | | | |
| | 21.067416 | 0% | 92% | | | | |

## FIG. 1 - 8

**Osteopontin**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 404845.234 | 401338.489 | 404845.234 | 447313.763 | 404845.234 | 33028.494 |
| average | 387716.320 | 407611.236 | 387716.320 | 424081.472 | 387716.320 | 256742.220 |
| stdev | 161977.983 | 141972.031 | 161977.983 | 153858.743 | 161977.983 | na |
| p (t-test) | | 0.626 | | 0.367 | | na |
| min | 33028.494 | 113938.640 | 33028.494 | 94230.433 | 33028.494 | 256742.220 |
| max | 729905.544 | 656561.188 | 729905.544 | 677771.212 | 729905.544 | 256742.220 |
| n (Samp) | 51 | 21 | 51 | 23 | 51 | 1 |
| n (Pat) | 40 | 21 | 40 | 23 | 40 | 1 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 425821.608 | 400506.412 | 425821.608 | 400366.819 | 425821.608 | 414928.520 |
| average | 405335.794 | 396500.431 | 405335.794 | 416501.814 | 405335.794 | 414928.520 |
| stdev | 161298.780 | 115861.924 | 161298.780 | 206933.501 | 161298.780 | 107724.720 |
| p (t-test) | | 0.895 | | 0.863 | | 0.934 |
| min | 33028.494 | 205200.154 | 33028.494 | 139179.187 | 33028.494 | 338755.640 |
| max | 729905.544 | 553924.484 | 729905.544 | 677771.212 | 729905.544 | 491101.400 |
| n (Samp) | 93 | 6 | 93 | 7 | 93 | 2 |
| n (Pat) | 73 | 6 | 73 | 7 | 73 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 319607.582 | 402738.482 | 319607.582 | 487336.350 | 319607.582 | 370747.605 |
| average | 367969.008 | 412651.333 | 367969.008 | 468151.417 | 367969.008 | 378110.290 |
| stdev | 159652.206 | 149454.437 | 159652.206 | 151489.722 | 159652.206 | 105689.231 |
| p (t-test) | | 0.337 | | 0.020 | | 0.902 |
| min | 33028.494 | 113938.640 | 33028.494 | 94230.433 | 33028.494 | 256742.220 |
| max | 729905.544 | 656561.188 | 729905.544 | 750295.504 | 729905.544 | 514203.729 |
| n (Samp) | 42 | 16 | 42 | 21 | 42 | 4 |
| n (Pat) | 33 | 16 | 33 | 21 | 33 | 4 |

sCr or UO

| Time prior AKI stage | AUC | SE | nCohort 1 | nCohort 2 | p |
|---|---|---|---|---|---|
| 0 hours | 0.54 | 0.076 | 51 | 21 | 0.559 |
| 24 hours | 0.58 | 0.073 | 51 | 23 | 0.287 |
| 48 hours | 0.22 | 0.181 | 51 | 1 | 0.116 |

sCr only

| Time prior AKI stage | AUC | SE | nCohort 1 | nCohort 2 | p |
|---|---|---|---|---|---|
| 0 hours | 0.46 | 0.119 | 93 | 6 | 0.729 |
| 24 hours | 0.50 | 0.114 | 93 | 7 | 0.984 |
| 48 hours | 0.50 | 0.207 | 93 | 2 | 1.000 |

UO only

| Time prior AKI stage | AUC | SE | nCohort 1 | nCohort 2 | p |
|---|---|---|---|---|---|
| 0 hours | 0.60 | 0.086 | 42 | 16 | 0.232 |
| 24 hours | 0.70 | 0.074 | 42 | 21 | 0.008 |
| 48 hours | 0.54 | 0.155 | 42 | 4 | 0.789 |

sCr or UO

| Time prior AKI stage | Cutoff value | sens | spec | Quartile | OR | 95% CI of OR | |
|---|---|---|---|---|---|---|---|
| 0 hours | 340143.98 | 71% | 43% | 1 | | | |
| | 328095.34 | 81% | 43% | 2 | 2.2 | 0.8 | 6.6 |
| | 200846.51 | 90% | 12% | 3 | 1.0 | 0.3 | 3.5 |
| | 483256.13 | 38% | 71% | 4 | 1.8 | 0.6 | 5.4 |
| | 510459.45 | 29% | 80% | | | | |
| | 595894.54 | 5% | 90% | | | | |
| 24 hours | 328095.34 | 74% | 43% | 1 | | | |
| | 269008.11 | 83% | 25% | 2 | 0.7 | 0.2 | 2.2 |

**FIG. 1 - 9**

| Time prior AKI stage | Cutoff value | sens | spec | Quartile | OR | 95% CI of OR | |
|---|---|---|---|---|---|---|---|
| | 250376.36 | 91% | 22% | 3 | 1.7 | 0.6 | 4.5 |
| | 483256.13 | 43% | 71% | 4 | 1.5 | 0.6 | 4.1 |
| | 510459.45 | 30% | 80% | | | | |
| | 595894.54 | 9% | 90% | | | | |
| 48 hours | 250376.36 | 100% | 22% | 1 | | | |
| | 250376.36 | 100% | 22% | 2 | na | na | na |
| | 250376.36 | 100% | 22% | 3 | na | na | na |
| | 483256.13 | 0% | 71% | 4 | na | na | na |
| | 510459.45 | 0% | 80% | | | | |
| | 595894.54 | 0% | 90% | | | | |

sCr only

| Time prior AKI stage | Cutoff value | sens | spec | Quartile | OR | 95% CI of OR | |
|---|---|---|---|---|---|---|---|
| 0 hours | 353219.39 | 83% | 35% | 1 | | | |
| | 353219.39 | 83% | 35% | 2 | 2.1 | 0.1 | 46.6 |
| | 200846.51 | 100% | 12% | 3 | 2.1 | 0.1 | 46.6 |
| | 499943.54 | 17% | 71% | 4 | 1.0 | 0.0 | 62.1 |
| | 546506.06 | 17% | 81% | | | | |
| | 593762.72 | 0% | 90% | | | | |
| 24 hours | 269008.11 | 71% | 23% | 1 | | | |
| | 250376.36 | 86% | 18% | 2 | 0.5 | 0.0 | 10.7 |
| | 138277.07 | 100% | 9% | 3 | 0.5 | 0.0 | 10.7 |
| | 499943.54 | 29% | 71% | 4 | 1.6 | 0.3 | 9.6 |
| | 546506.06 | 29% | 81% | | | | |
| | 593762.72 | 29% | 90% | | | | |
| 48 hours | 328095.34 | 100% | 33% | 1 | | | |
| | 328095.34 | 100% | 33% | 2 | na | na | na |
| | 328095.34 | 100% | 33% | 3 | na | na | na |
| | 499943.54 | 0% | 71% | 4 | na | na | na |
| | 546506.06 | 0% | 81% | | | | |
| | 593762.72 | 0% | 90% | | | | |

UO only

| Time prior AKI stage | Cutoff value | sens | spec | Quartile | OR | 95% CI of OR | |
|---|---|---|---|---|---|---|---|
| 0 hours | 338755.64 | 75% | 52% | 1 | | | |
| | 328095.34 | 81% | 52% | 2 | 0.9 | 0.2 | 4.8 |
| | 171339.12 | 94% | 10% | 3 | 1.0 | 0.2 | 5.3 |
| | 456794.78 | 44% | 71% | 4 | 3.2 | 0.8 | 12.5 |
| | 492543.32 | 44% | 81% | | | | |
| | 553924.48 | 19% | 90% | | | | |
| 24 hours | 409417.34 | 71% | 60% | 1 | | | |
| | 349742.27 | 81% | 52% | 2 | 2.2 | 0.4 | 12.9 |
| | 297511.13 | 90% | 40% | 3 | 5.1 | 1.0 | 25.8 |
| | 456794.78 | 57% | 71% | 4 | 6.5 | 1.3 | 32.9 |
| | 492543.32 | 43% | 81% | | | | |
| | 553924.48 | 29% | 90% | | | | |
| 48 hours | 328095.34 | 75% | 52% | 1 | | | |
| | 255537.24 | 100% | 24% | 2 | 0.0 | 0.0 | na |
| | 255537.24 | 100% | 24% | 3 | 2.2 | 0.1 | 63.6 |
| | 456794.78 | 25% | 71% | 4 | 0.9 | 0.0 | 66.6 |
| | 492543.32 | 25% | 81% | | | | |
| | 553924.48 | 0% | 90% | | | | |

# FIG. 1 - 10

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 15.679 | 7.682 | 15.679 | 0.036 | 15.679 | 0.036 |
| average | 29.109 | 51.017 | 29.109 | 8.162 | 29.109 | 2.792 |
| stdev | 38.178 | 76.676 | 38.178 | na | 38.178 | na |
| p (t-test) | | 0.142 | | na | | na |
| min | 0.036 | 0.036 | 0.036 | 8.162 | 0.036 | 2.792 |
| max | 221.429 | 270.067 | 221.429 | 8.162 | 221.429 | 2.792 |
| n (Samp) | 54 | 13 | 54 | 1 | 54 | 1 |
| n (Pat) | 36 | 13 | 36 | 1 | 36 | 1 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 13.531 | 58.494 | 13.531 | 7.088 | 13.531 | 15.538 |
| average | 32.165 | 65.959 | 32.165 | 29.914 | 32.165 | 15.538 |
| stdev | 45.733 | 39.773 | 45.733 | 43.309 | 45.733 | 16.048 |
| p (t-test) | | 0.081 | | 0.933 | | 0.611 |
| min | 0.036 | 7.682 | 0.036 | 2.792 | 0.036 | 4.190 |
| max | 270.067 | 111.111 | 270.067 | 79.861 | 270.067 | 26.885 |
| n (Samp) | 86 | 6 | 86 | 3 | 86 | 2 |
| n (Pat) | 60 | 6 | 60 | 3 | 60 | 2 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 12.046 | 7.385 | 12.046 | 34.529 | 12.046 | 0.036 |
| average | 27.503 | 50.461 | 27.503 | 34.529 | 27.503 | 2.792 |
| stdev | 41.364 | 80.041 | 41.364 | 37.290 | 41.364 | na |
| p (t-test) | | 0.189 | | 0.815 | | na |
| min | 0.036 | 0.036 | 0.036 | 8.162 | 0.036 | 2.792 |
| max | 221.429 | 270.067 | 221.429 | 60.897 | 221.429 | 2.792 |
| n (Samp) | 40 | 12 | 40 | 2 | 40 | 1 |
| n (Pat) | 26 | 12 | 26 | 2 | 26 | 1 |

sCr or UO

| Time prior AKI stage | AUC | SE | nCohort 1 | nCohort 2 | p |
|---|---|---|---|---|---|
| 0 hours | 0.53 | 0.091 | 54 | 13 | 0.712 |
| 24 hours | 0.36 | 0.254 | 54 | 1 | 0.584 |
| 48 hours | 0.27 | 0.211 | 54 | 1 | 0.272 |

sCr only

| Time prior AKI stage | AUC | SE | nCohort 1 | nCohort 2 | p |
|---|---|---|---|---|---|
| 0 hours | 0.78 | 0.113 | 86 | 6 | 0.012 |
| 24 hours | 0.50 | 0.170 | 86 | 3 | 1.000 |
| 48 hours | 0.46 | 0.202 | 86 | 2 | 0.852 |

UO only

| Time prior AKI stage | AUC | SE | nCohort 1 | nCohort 2 | p |
|---|---|---|---|---|---|
| 0 hours | 0.52 | 0.097 | 40 | 12 | 0.804 |
| 24 hours | 0.63 | 0.218 | 40 | 2 | 0.567 |
| 48 hours | 0.30 | 0.229 | 40 | 1 | 0.383 |

sCr or UO

| Time prior AKI stage | Cutoff value | sens | spec | Quartile | OR | 95% CI of OR | |
|---|---|---|---|---|---|---|---|
| 0 hours | 2.7920962 | 77% | 28% | 1 | | | |
| | 0.6443299 | 85% | 15% | 2 | 1.3 | 0.3 | 5.7 |
| | 0 | 100% | 0% | 3 | 0.3 | 0.0 | 4.9 |
| | 33.653846 | 46% | 70% | 4 | 1.8 | 0.5 | 7.0 |
| | 54.487179 | 31% | 81% | | | | |
| | 74.652778 | 23% | 91% | | | | |
| 24 hours | 6.0137457 | 100% | 35% | 1 | | | |
| | 6.0137457 | 100% | 35% | 2 | na | na | na |

## FIG. 1 - 11

| Time prior AKI stage | Cutoff value | sens | spec | Quartile | OR | 95% CI of OR | |
|---|---|---|---|---|---|---|---|
| | 6.0137457 | 100% | 35% | 3 | na | na | na |
| | 33.653846 | 0% | 70% | 4 | na | na | na |
| | 54.487179 | 0% | 81% | | | | |
| | 74.652778 | 0% | 91% | | | | |
| 48 hours | 2.4441341 | 100% | 26% | 1 | | | |
| | 2.4441341 | 100% | 26% | 2 | na | na | na |
| | 2.4441341 | 100% | 26% | 3 | na | na | na |
| | 33.653846 | 0% | 70% | 4 | na | na | na |
| | 54.487179 | 0% | 81% | | | | |
| | 74.652778 | 0% | 91% | | | | |

sCr only

| Time prior AKI stage | Cutoff value | sens | spec | Quartile | OR | 95% CI of OR | |
|---|---|---|---|---|---|---|---|
| 0 hours | 48.076923 | 83% | 77% | 1 | | | |
| | 48.076923 | 83% | 77% | 2 | na | na | na |
| | 7.0876289 | 100% | 36% | 3 | na | na | na |
| | 35.25641 | 83% | 71% | 4 | na | na | na |
| | 59.294872 | 50% | 80% | | | | |
| | 83.333333 | 33% | 91% | | | | |
| 24 hours | 2.4441341 | 100% | 26% | 1 | | | |
| | 2.4441341 | 100% | 26% | 2 | 0.0 | 0.0 | na |
| | 2.4441341 | 100% | 26% | 3 | 2.2 | 0.1 | 50.2 |
| | 35.25641 | 33% | 71% | 4 | 0.0 | 0.0 | na |
| | 59.294872 | 33% | 80% | | | | |
| | 83.333333 | 0% | 91% | | | | |
| 48 hours | 2.7920962 | 100% | 29% | 1 | | | |
| | 2.7920962 | 100% | 29% | 2 | na | na | na |
| | 2.7920962 | 100% | 29% | 3 | na | na | na |
| | 35.25641 | 0% | 71% | 4 | na | na | na |
| | 59.294872 | 0% | 80% | | | | |
| | 83.333333 | 0% | 91% | | | | |

UO only

| Time prior AKI stage | Cutoff value | sens | spec | Quartile | OR | 95% CI of OR | |
|---|---|---|---|---|---|---|---|
| 0 hours | 2.7920962 | 75% | 33% | 1 | | | |
| | 0.0360285 | 83% | 13% | 2 | 1.5 | 0.3 | 7.0 |
| | 0 | 100% | 0% | 3 | 0.3 | 0.0 | 5.4 |
| | 30.377095 | 42% | 73% | 4 | 1.5 | 0.3 | 7.0 |
| | 41.666667 | 33% | 80% | | | | |
| | 74.652778 | 25% | 90% | | | | |
| 24 hours | 6.0137457 | 100% | 40% | 1 | | | |
| | 6.0137457 | 100% | 40% | 2 | na | na | na |
| | 6.0137457 | 100% | 40% | 3 | na | na | na |
| | 30.377095 | 50% | 73% | 4 | na | na | na |
| | 41.666667 | 50% | 80% | | | | |
| | 74.652778 | 0% | 90% | | | | |
| 48 hours | 2.4441341 | 100% | 28% | 1 | | | |
| | 2.4441341 | 100% | 28% | 2 | na | na | na |
| | 2.4441341 | 100% | 28% | 3 | na | na | na |
| | 30.377095 | 0% | 73% | 4 | na | na | na |
| | 41.666667 | 0% | 80% | | | | |
| | 74.652778 | 0% | 90% | | | | |

**FIG. 1 - 12**

**Bone morphogenetic protein 7**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.275 | 0.163 | 0.275 | 4.405 | 0.275 | 0.219 |
| average | 2.870 | 0.363 | 2.870 | 6.126 | 2.870 | 0.219 |
| stdev | 5.205 | 0.522 | 5.205 | 3.442 | 5.205 | 0.079 |
| p (t-test) |  | 0.179 |  | 0.221 |  | 0.476 |
| min | 0.163 | 0.163 | 0.163 | 4.405 | 0.163 | 0.163 |
| max | 22.302 | 1.652 | 22.302 | 11.289 | 22.302 | 0.275 |
| n (Samp) | 77 | 8 | 77 | 4 | 77 | 2 |
| n (Pat) | 55 | 8 | 55 | 4 | 55 | 2 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.275 | 0.163 | 0.275 | 4.405 | 0.275 | 0.964 |
| average | 2.566 | 0.275 | 2.566 | 4.405 | 2.566 | 0.964 |
| stdev | 4.891 | na | 4.891 | 0.000 | 4.891 | 0.973 |
| p (t-test) |  | na |  | 0.598 |  | 0.646 |
| min | 0.163 | 0.275 | 0.163 | 4.405 | 0.163 | 0.275 |
| max | 22.302 | 0.275 | 22.302 | 4.405 | 22.302 | 1.652 |
| n (Samp) | 94 | 1 | 94 | 2 | 94 | 2 |
| n (Pat) | 67 | 1 | 67 | 2 | 67 | 2 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.275 | 0.163 | 0.275 | 7.847 | 0.275 | 0.163 |
| average | 2.791 | 0.363 | 2.791 | 7.847 | 2.791 | 0.163 |
| stdev | 4.973 | 0.522 | 4.973 | 4.867 | 4.973 | na |
| p (t-test) |  | 0.175 |  | 0.162 |  | na |
| min | 0.163 | 0.163 | 0.163 | 4.405 | 0.163 | 0.163 |
| max | 20.925 | 1.652 | 20.925 | 11.289 | 20.925 | 0.163 |
| n (Samp) | 60 | 8 | 60 | 2 | 60 | 1 |
| n (Pat) | 42 | 8 | 42 | 2 | 42 | 1 |

sCr or UO

| Time prior AKI stage | AUC | SE | nCohort 1 | nCohort 2 | p |
|---|---|---|---|---|---|
| 0 hours | 0.32 | 0.089 | 77 | 8 | 0.045 |
| 24 hours | 0.82 | 0.131 | 77 | 4 | 0.015 |
| 48 hours | 0.38 | 0.185 | 77 | 2 | 0.515 |

sCr only

| Time prior AKI stage | AUC | SE | nCohort 1 | nCohort 2 | p |
|---|---|---|---|---|---|
| 0 hours | 0.56 | 0.300 | 94 | 1 | 0.845 |
| 24 hours | 0.81 | 0.186 | 94 | 2 | 0.091 |
| 48 hours | 0.63 | 0.215 | 94 | 2 | 0.536 |

UO only

| Time prior AKI stage | AUC | SE | nCohort 1 | nCohort 2 | p |
|---|---|---|---|---|---|
| 0 hours | 0.33 | 0.093 | 60 | 8 | 0.070 |
| 24 hours | 0.85 | 0.171 | 60 | 2 | 0.039 |
| 48 hours | 0.24 | 0.195 | 60 | 1 | 0.186 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 1.1 | 0.0 | 64.1 |
|  | 0 | 100% | 0% | 3 | 6.6 | 0.5 | 85.6 |
|  | 1.6519824 | 0% | 74% | 4 | 1.1 | 0.0 | 64.1 |
|  | 4.4052863 | 0% | 83% |  |  |  |  |
|  | 11.288546 | 0% | 91% |  |  |  |  |
| 24 hours | 3.0286344 | 100% | 75% | 1 |  |  |  |
|  | 3.0286344 | 100% | 75% | 2 | na | na | na |
|  | 3.0286344 | 100% | 75% | 3 | na | na | na |
|  | 1.6519824 | 100% | 74% | 4 | na | na | na |
|  | 4.4052863 | 25% | 83% |  |  |  |  |

## FIG. 2 - 1

| | 11.288546 | 0% | 91% | | | | |
|---|---|---|---|---|---|---|---|
| 48 hours | 0 | 100% | 0% | 1 | | | |
| | 0 | 100% | 0% | 2 | na | na | na |
| | 0 | 100% | 0% | 3 | na | na | na |
| | 1.6519824 | 0% | 74% | 4 | na | na | na |
| | 4.4052863 | 0% | 83% | | | | |
| | 11.288546 | 0% | 91% | | | | |

**Complement C3**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.000 | 0.000 | 0.000 | 0.000 | 0.000 | 0.000 |
| average | 0.000 | 0.000 | 0.000 | 0.000 | 0.000 | 0.000 |
| stdev | 0.000 | 0.000 | 0.000 | 0.000 | 0.000 | 0.000 |
| p (t-test) | | 0.458 | | 0.968 | | 0.892 |
| min | 0.000 | 0.000 | 0.000 | 0.000 | 0.000 | 0.000 |
| max | 0.002 | 0.002 | 0.002 | 0.001 | 0.002 | 0.002 |
| n (Samp) | 419 | 27 | 419 | 36 | 419 | 18 |
| n (Pat) | 164 | 27 | 164 | 36 | 164 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.000 | 0.000 | 0.000 | 0.000 | 0.000 | 0.000 |
| average | 0.000 | 0.000 | 0.000 | 0.000 | 0.000 | 0.000 |
| stdev | 0.000 | 0.000 | 0.000 | 0.000 | 0.000 | 0.000 |
| p (t-test) | | 0.410 | | 0.860 | | 0.540 |
| min | 0.000 | 0.000 | 0.000 | 0.000 | 0.000 | 0.000 |
| max | 0.002 | 0.000 | 0.002 | 0.001 | 0.002 | 0.000 |
| n (Samp) | 518 | 5 | 518 | 9 | 518 | 7 |
| n (Pat) | 199 | 5 | 199 | 9 | 199 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.000 | 0.000 | 0.000 | 0.000 | 0.000 | 0.000 |
| average | 0.000 | 0.000 | 0.000 | 0.000 | 0.000 | 0.000 |
| stdev | 0.000 | 0.000 | 0.000 | 0.000 | 0.000 | 0.000 |
| p (t-test) | | 0.483 | | 0.727 | | 0.575 |
| min | 0.000 | 0.000 | 0.000 | 0.000 | 0.000 | 0.000 |
| max | 0.002 | 0.002 | 0.002 | 0.001 | 0.002 | 0.002 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.52 | 0.058 | 419 | 27 | 0.780 |
| 24 hours | 0.53 | 0.051 | 419 | 36 | 0.560 |
| 48 hours | 0.51 | 0.070 | 419 | 18 | 0.885 |

sCr only

| Time prior AKI stage | AUC | SE | nCohort 1 | nCohort 2 | p |
|---|---|---|---|---|---|
| 0 hours | 0.39 | 0.116 | 518 | 5 | 0.329 |
| 24 hours | 0.49 | 0.096 | 518 | 9 | 0.916 |
| 48 hours | 0.53 | 0.112 | 518 | 7 | 0.757 |

UO only

| Time prior AKI stage | AUC | SE | nCohort 1 | nCohort 2 | p |
|---|---|---|---|---|---|
| 0 hours | 0.53 | 0.060 | 352 | 26 | 0.572 |
| 24 hours | 0.54 | 0.056 | 352 | 30 | 0.530 |
| 48 hours | 0.52 | 0.075 | 352 | 16 | 0.761 |

sCr or UO

| Time prior AKI stage | Cutoff value | sens | spec | Quartile | OR | 95% CI of OR |
|---|---|---|---|---|---|---|

# FIG. 2 - 2

| | Cutoff value | sens | spec | Quartile | OR | 95% CI of OR | |
|---|---|---|---|---|---|---|---|
| 0 hours | 0.0000522 | 70% | 37% | 1 | | | |
| | 0.0000402 | 81% | 30% | 2 | 5.9 | 1.8 | 19.6 |
| | 0.0000372 | 93% | 28% | 3 | 3.1 | 0.8 | 11.9 |
| | 0.000173 | 30% | 70% | 4 | 4.2 | 1.2 | 14.8 |
| | 0.000276 | 11% | 80% | | | | |
| | 0.000736 | 4% | 90% | | | | |
| 24 hours | 0.0000525 | 72% | 37% | 1 | | | |
| | 0.0000384 | 81% | 29% | 2 | 1.9 | 1.1 | 3.3 |
| | 8.97E-06 | 92% | 6% | 3 | 1.5 | 0.9 | 2.7 |
| | 0.000173 | 33% | 70% | 4 | 1.7 | 1.0 | 3.0 |
| | 0.000276 | 25% | 80% | | | | |
| | 0.000736 | 8% | 90% | | | | |
| 48 hours | 0.000058 | 72% | 40% | 1 | | | |
| | 0.0000212 | 83% | 17% | 2 | 1.5 | 0.7 | 3.6 |
| | 0.000018 | 94% | 14% | 3 | 1.0 | 0.4 | 2.8 |
| | 0.000173 | 33% | 70% | 4 | 1.0 | 0.4 | 2.7 |
| | 0.000276 | 17% | 80% | | | | |
| | 0.000736 | 11% | 90% | | | | |

sCr only

| Time prior AKI stage | Cutoff value | sens | spec | Quartile | OR | 95% CI of OR | |
|---|---|---|---|---|---|---|---|
| 0 hours | 0.0000402 | 80% | 28% | 1 | | | |
| | 0.0000402 | 80% | 28% | 2 | 1.0 | 0.0 | 51.9 |
| | 0.0000025 | 100% | 0% | 3 | 2.0 | 0.1 | 39.3 |
| | 0.000171 | 20% | 70% | 4 | 1.0 | 0.0 | 52.4 |
| | 0.000276 | 0% | 80% | | | | |
| | 0.000684 | 0% | 90% | | | | |
| 24 hours | 0.0000373 | 78% | 27% | 1 | | | |
| | 0.0000212 | 89% | 16% | 2 | 0.3 | 0.0 | 4.6 |
| | 7.57E-06 | 100% | 4% | 3 | 1.0 | 0.3 | 3.8 |
| | 0.000171 | 33% | 70% | 4 | 0.7 | 0.1 | 3.5 |
| | 0.000276 | 22% | 80% | | | | |
| | 0.000684 | 22% | 90% | | | | |
| 48 hours | 0.0000802 | 71% | 48% | 1 | | | |
| | 0.000074 | 86% | 46% | 2 | 3.0 | 0.2 | 42.9 |
| | 0.0000212 | 100% | 16% | 3 | 1.0 | 0.0 | 51.9 |
| | 0.000171 | 29% | 70% | 4 | 2.0 | 0.1 | 39.0 |
| | 0.000276 | 14% | 80% | | | | |
| | 0.000684 | 0% | 90% | | | | |

UO only

| Time prior AKI stage | Cutoff value | sens | spec | Quartile | OR | 95% CI of OR | |
|---|---|---|---|---|---|---|---|
| 0 hours | 0.000064 | 73% | 41% | 1 | | | |
| | 0.0000481 | 81% | 35% | 2 | 5.4 | 1.6 | 18.3 |
| | 0.0000368 | 92% | 28% | 3 | 4.3 | 1.2 | 15.2 |
| | 0.000181 | 31% | 70% | 4 | 3.1 | 0.8 | 12.0 |
| | 0.000307 | 4% | 80% | | | | |
| | 0.000736 | 4% | 90% | | | | |
| 24 hours | 0.0000617 | 70% | 41% | 1 | | | |
| | 0.000045 | 80% | 33% | 2 | 2.6 | 1.3 | 5.5 |
| | 0.00003 | 90% | 23% | 3 | 2.1 | 1.0 | 4.6 |
| | 0.000181 | 30% | 70% | 4 | 2.1 | 0.9 | 4.5 |
| | 0.000307 | 23% | 80% | | | | |
| | 0.000736 | 7% | 90% | | | | |
| 48 hours | 0.0000406 | 75% | 30% | 1 | | | |
| | 0.0000402 | 81% | 29% | 2 | 2.1 | 0.7 | 5.8 |
| | 0.000018 | 94% | 13% | 3 | 1.0 | 0.3 | 3.9 |
| | 0.000181 | 38% | 70% | 4 | 1.3 | 0.4 | 4.4 |
| | 0.000307 | 25% | 80% | | | | |
| | 0.000736 | 13% | 90% | | | | |

# FIG. 2 - 3

**Epidermal growth factor**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 19000.000 | 22100.000 | 19000.000 | 18750.000 | 19000.000 | 13750.000 |
| average | 27046.117 | 29251.333 | 27046.117 | 22999.167 | 27046.117 | 19610.556 |
| stdev | 24371.617 | 23389.968 | 24371.617 | 18256.375 | 24371.617 | 21088.948 |
| p (t-test) | | 0.648 | | 0.331 | | 0.203 |
| min | 100.000 | 1430.000 | 100.000 | 2340.000 | 100.000 | 2830.000 |
| max | 109224.000 | 88986.000 | 109224.000 | 73300.000 | 109224.000 | 91500.000 |
| n (Samp) | 419 | 27 | 419 | 36 | 419 | 18 |
| n (Pat) | 164 | 27 | 164 | 36 | 164 | 18 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 20850.000 | 9220.000 | 20850.000 | 16700.000 | 20850.000 | 14600.000 |
| average | 27267.942 | 8298.000 | 27267.942 | 22023.333 | 27267.942 | 14932.857 |
| stdev | 24047.577 | 5862.569 | 24047.577 | 26372.248 | 24047.577 | 9483.285 |
| p (t-test) | | 0.079 | | 0.517 | | 0.176 |
| min | 100.000 | 1430.000 | 100.000 | 2340.000 | 100.000 | 5480.000 |
| max | 109224.000 | 16600.000 | 109224.000 | 69200.000 | 109224.000 | 33700.000 |
| n (Samp) | 518 | 5 | 518 | 9 | 518 | 7 |
| n (Pat) | 199 | 5 | 199 | 9 | 199 | 7 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 17400.000 | 22550.000 | 17400.000 | 21300.000 | 17400.000 | 14150.000 |
| average | 25840.389 | 30314.462 | 25840.389 | 24006.667 | 25840.389 | 20496.875 |
| stdev | 23890.322 | 23301.176 | 23890.322 | 16764.426 | 23890.322 | 22291.579 |
| p (t-test) | | 0.357 | | 0.681 | | 0.381 |
| min | 100.000 | 1850.000 | 100.000 | 3410.000 | 100.000 | 2610.000 |
| max | 109224.000 | 88986.000 | 109224.000 | 73300.000 | 109224.000 | 91500.000 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.55 | 0.059 | 419 | 27 | 0.438 |
| 24 hours | 0.48 | 0.050 | 419 | 36 | 0.697 |
| 48 hours | 0.41 | 0.064 | 419 | 18 | 0.139 |

sCr only

| Time prior AKI stage | AUC | SE | nCohort 1 | nCohort 2 | p |
|---|---|---|---|---|---|
| 0 hours | 0.22 | 0.079 | 518 | 5 | 0.000 |
| 24 hours | 0.38 | 0.086 | 518 | 9 | 0.146 |
| 48 hours | 0.37 | 0.096 | 518 | 7 | 0.182 |

UO only

| Time prior AKI stage | AUC | SE | nCohort 1 | nCohort 2 | p |
|---|---|---|---|---|---|
| 0 hours | 0.58 | 0.060 | 352 | 26 | 0.191 |
| 24 hours | 0.53 | 0.056 | 352 | 30 | 0.558 |
| 48 hours | 0.42 | 0.069 | 352 | 16 | 0.280 |

sCr or UO

| Time prior AKI stage | Cutoff value | sens | spec | Quartile | OR | 95% CI of OR | |
|---|---|---|---|---|---|---|---|
| 0 hours | 15100 | 70% | 42% | 1 | | | |
| | 11500 | 81% | 35% | 2 | 2.1 | 1.0 | 4.5 |
| | 4110 | 93% | 9% | 3 | 1.8 | 0.8 | 4.0 |
| | 33100 | 33% | 70% | 4 | 2.1 | 1.0 | 4.5 |
| | 43300 | 22% | 80% | | | | |
| | 65100 | 11% | 90% | | | | |
| 24 hours | 12300 | 72% | 37% | 1 | | | |
| | 9050 | 81% | 27% | 2 | 2.1 | 1.2 | 3.6 |
| | 3330 | 92% | 7% | 3 | 1.9 | 1.1 | 3.3 |
| | 33100 | 19% | 70% | 4 | 1.2 | 0.6 | 2.3 |

# FIG. 2 - 4

| | Cutoff value | sens | spec | Quartile | OR | 95% CI of OR | |
|---|---|---|---|---|---|---|---|
| | 43300 | 11% | 80% | | | | |
| | 65100 | 8% | 90% | · | | | |
| 48 hours | 6690 | 72% | 20% | 1 | | | |
| | 5400 | 83% | 13% | 2 | 2.1 | 0.5 | 9.3 |
| | 3000 | 94% | 6% | 3 | 3.1 | 0.8 | 12.1 |
| | 33100 | 11% | 70% | 4 | 3.1 | 0.8 | 12.1 |
| | 43300 | 11% | 80% | | | | |
| | 65100 | 6% | 90% | | | | |

sCr only

| Time prior AKI stage | Cutoff value | sens | spec | Quartile | OR | 95% CI of OR | |
|---|---|---|---|---|---|---|---|
| 0 hours | 4110 | 80% | 9% | 1 | | | |
| | 4110 | 80% | 9% | 2 | na | na | na |
| | 1410 | 100% | 2% | 3 | na | na | na |
| | 32500 | 0% | 70% | 4 | na | na | na |
| | 43000 | 0% | 80% | | | | |
| | 65300 | 0% | 90% | | | | |
| 24 hours | 2500 | 78% | 4% | 1 | | | |
| | 2340 | 89% | 4% | 2 | 0.0 | 0.0 | na |
| | 2230 | 100% | 4% | 3 | 1.5 | 0.3 | 8.0 |
| | 32500 | 22% | 70% | · 4 | 2.0 | 0.5 | 9.2 |
| | 43000 | 22% | 80% | | | | |
| | 65300 | 11% | 90% | | | | |
| 48 hours | 9950 | 71% | 28% | 1 | | | |
| | 6690 | 86% | 19% | 2 | na | na | na |
| | 5460 | 100% | · 14% | 3 | na | na | na |
| | 32500 | 14% | 70% | 4 | na | na | na |
| | 43000 | 0% | 80% | | | | |
| | 65300 | 0% | 90% | | | | |

UO only

| Time prior AKI stage | Cutoff value | sens | spec | Quartile | OR | 95% CI of OR | |
|---|---|---|---|---|---|---|---|
| 0 hours | 15100 | 73% | 44% | 1 | | | |
| | 12900 | 81% | 39% | 2 | 1.3 | 0.5 | 3.2 |
| | 6340 | 92% | 18% | 3 | 2.4 | 1.1 | 5.1 |
| | 30900 | 35% | 70% | 4 | 2.1 | 0.9 | 4.5 |
| | 41100 | 27% | 80% | | | | |
| | 62200 | 12% | 90% | | | | |
| 24 hours | 13100 | 70% | 40% | 1 | | | |
| | 11100 | 80% | 35% | 2 | 2.1 | 0.9 | 4.5 |
| | 6340 | 90% | 18% | 3 | 3.3 | 1.6 | 6.6 |
| | 30900 | 27% | 70% | 4 | 1.5 | 0.6 | 3.6 |
| | 41100 | 10% | 80% | | | | |
| | 62200 | 7% | 90% | | | | |
| 48 hours | 5400 | 75% | 13% | 1 | | | |
| | 3930 | 81% | 9% | 2 | 2.0 | 0.5 | 9.3 |
| | 2810 | 94% | 5% | 3 | 2.6 | 0.6 | 10.7 |
| | 30900 | 13% | 70% | 4 | 2.6 | 0.6 | 10.7 |
| | 41100 | 13% | 80% | | | | |
| | 62200 | 6% | 90% | | | | |

**Interleukin-1 alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.002 | 0.001 | 0.002 | 0.001 | 0.002 | 0.001 |
| average | 0.002 | 0.002 | 0.002 | 0.002 | 0.002 | 0.002 |
| stdev | 0.003 | 0.002 | 0.003 | 0.005 | 0.003 | 0.003 |
| p (t-test) | | 0.411 | | 0.839 | | 0.811 |
| min | 0.000 | 0.000 | 0.000 | 0.000 | 0.000 | 0.000 |
| max | 0.038 | 0.007 | 0.038 | 0.027 | 0.038 | 0.012 |
| n (Samp) | 419 | 27 | 419 | 36 | 419 | 18 |
| n (Pat) | 164 | 27 | 164 | 36 | 164 | 18 |

## FIG. 2 - 5

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.001 | 0.003 | 0.001 | 0.002 | 0.001 | 0.003 |
| average | 0.002 | 0.004 | 0.002 | 0.002 | 0.002 | 0.004 |
| stdev | 0.003 | 0.005 | 0.003 | 0.002 | 0.003 | 0.004 |
| p (t-test) | | 0.193 | | 0.948 | | 0.205 |
| min | 0.000 | 0.000 | 0.000 | 0.000 | 0.000 | 0.000 |
| max | 0.038 | 0.012 | 0.038 | 0.007 | 0.038 | 0.012 |
| n (Samp) | 518 | 5 | 518 | 9 | 518 | 7 |
| n (Pat) | 199 | 5 | 199 | 9 | 199 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.002 | 0.001 | 0.002 | 0.000 | 0.002 | 0.001 |
| average | 0.002 | 0.002 | 0.002 | 0.002 | 0.002 | 0.001 |
| stdev | 0.003 | 0.002 | 0.003 | 0.005 | 0.003 | 0.001 |
| p (t-test) | | 0.505 | | 0.714 | | 0.356 |
| min | 0.000 | 0.000 | 0.000 | 0.000 | 0.000 | 0.000 |
| max | 0.033 | 0.007 | 0.033 | 0.027 | 0.033 | 0.004 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.46 | 0.056 | 419 | 27 | 0.445 |
| 24 hours | 0.45 | 0.048 | 419 | 36 | 0.284 |
| 48 hours | 0.46 | 0.068 | 419 | 18 | 0.587 |

sCr only

| Time prior AKI stage | AUC | SE | nCohort 1 | nCohort 2 | p |
|---|---|---|---|---|---|
| 0 hours | 0.67 | 0.133 | 518 | 5 | 0.191 |
| 24 hours | 0.52 | 0.099 | 518 | 9 | 0.804 |
| 48 hours | 0.68 | 0.113 | 518 | 7 | 0.118 |

UO only

| Time prior AKI stage | AUC | SE | nCohort 1 | nCohort 2 | p |
|---|---|---|---|---|---|
| 0 hours | 0.47 | 0.057 | 352 | 26 | 0.557 |
| 24 hours | 0.45 | 0.053 | 352 | 30 | 0.304 |
| 48 hours | 0.44 | 0.071 | 352 | 16 | 0.431 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.4 | 0.1 | 0.9 |
| | 0 | 100% | 0% | 3 | 1.0 | 0.6 | 1.7 |
| | 0.0024 | 33% | 70% | 4 | 1.0 | 0.6 | 1.7 |
| | 0.00308 | 11% | 80% | | | | |
| | 0.00423 | 7% | 90% | | | | |
| 24 hours | 0 | 100% | 0% | 1 | | | |
| | 0 | 100% | 0% | 2 | 0.6 | 0.4 | 1.2 |
| | 0 | 100% | 0% | 3 | 0.9 | 0.5 | 1.5 |
| | 0.0024 | 28% | 70% | 4 | 1.5 | 1.0 | 2.3 |
| | 0.00308 | 17% | 80% | | | | |
| | 0.00423 | 8% | 90% | | | | |
| 48 hours | 0 | 100% | 0% | 1 | | | |
| | 0 | 100% | 0% | 2 | 0.5 | 0.1 | 2.2 |
| | 0 | 100% | 0% | 3 | 2.7 | 1.3 | 5.5 |
| | 0.0024 | 28% | 70% | 4 | 0.5 | 0.1 | 2.2 |
| | 0.00308 | 22% | 80% | | | | |
| | 0.00423 | 6% | 90% | | | | |

sCr only

| Time prior AKI stage | Cutoff value | sens | spec | Quartile | OR | 95% CI of OR | |
|---|---|---|---|---|---|---|---|
| 0 hours | 0.00201 | 80% | 63% | 1 | | | |

## FIG. 2 - 6

| | | | | | | | |
|---|---|---|---|---|---|---|---|
| | 0.00201 | 80% | 63% | 2 | na | na | na |
| | 0 | 100% | 0% | 3 | na | na | na |
| | 0.00231 | 60% | 70% | 4 | na | na | na |
| | 0.00294 | 40% | 80% | | | | |
| | 0.00416 | 20% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 0.00231 | 33% | 70% | 4 | na | na | na |
| | 0.00294 | 22% | 80% | | | | |
| | 0.00416 | 11% | 90% | | | | |
| 48 hours | 0.00212 | 71% | 66% | 1 | | | |
| | 0.00098 | 86% | 43% | 2 | 1.0 | 0.0 | 51.9 |
| | 0 | 100% | 0% | 3 | 2.0 | 0.1 | 39.3 |
| | 0.00231 | 57% | 70% | 4 | 3.0 | 0.2 | 42.5 |
| | 0.00294 | 43% | 80% | | | | |
| | 0.00416 | 14% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.2 | 0.1 | 0.7 |
| | 0 | 100% | 0% | 3 | 0.3 | 0.1 | 0.8 |
| | 0.00231 | 38% | 71% | 4 | 1.4 | 0.9 | 2.1 |
| | 0.00288 | 23% | 80% | | | | |
| | 0.00389 | 8% | 90% | | | | |
| 24 hours | 0 | 100% | 0% | 1 | | | |
| | 0 | 100% | 0% | 2 | 0.5 | 0.2 | 1.1 |
| | 0 | 100% | 0% | 3 | 2.2 | 1.5 | 3.3 |
| | 0.00231 | 27% | 71% | 4 | 0.2 | 0.1 | 0.8 |
| | 0.00288 | 20% | 80% | | | | |
| | 0.00389 | 10% | 90% | | | | |
| 48 hours | 0 | 100% | 0% | 1 | | | |
| | 0 | 100% | 0% | 2 | 0.2 | 0.0 | 2.9 |
| | 0 | 100% | 0% | 3 | 1.3 | 0.5 | 3.2 |
| | 0.00231 | 25% | 71% | 4 | 1.5 | 0.6 | 3.6 |
| | 0.00288 | 19% | 80% | | | | |
| | 0.00389 | 6% | 90% | | | | |

**Interleukin-4**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 25.200 | 25.200 | 25.200 | 22.750 | 25.200 | 24.850 |
| average | 24.595 | 24.147 | 24.595 | 22.464 | 24.595 | 24.585 |
| stdev | 11.809 | 13.033 | 11.809 | 12.920 | 11.809 | 15.993 |
| p (t-test) | | 0.849 | | 0.303 | | 0.997 |
| min | 0.416 | 0.416 | 0.416 | 0.416 | 0.416 | 0.416 |
| max | 62.400 | 52.700 | 62.400 | 48.600 | 62.400 | 56.700 |
| n (Samp) | 419 | 27 | 419 | 36 | 419 | 18 |
| n (Pat) | 164 | 27 | 164 | 36 | 164 | 18 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 25.000 | 25.500 | 25.000 | 23.200 | 25.000 | 33.400 |
| average | 24.270 | 28.620 | 24.270 | 21.913 | 24.270 | 30.114 |
| stdev | 11.992 | 10.637 | 11.992 | 13.025 | 11.992 | 16.540 |
| p (t-test) | | 0.420 | | 0.560 | | 0.203 |
| min | 0.416 | 15.800 | 0.416 | 0.416 | 0.416 | 3.800 |
| max | 62.400 | 44.600 | 62.400 | 40.600 | 62.400 | 48.600 |
| n (Samp) | 518 | 5 | 518 | 9 | 518 | 7 |
| n (Pat) | 199 | 5 | 199 | 9 | 199 | 7 |

UO only

| 0 hr prior to AKI stage | 24 hr prior to AKI stage | 48 hr prior to AKI stage |
|---|---|---|

## FIG. 2 - 7

|  | Cohort 1 | Cohort 2 | Cohort 1 | Cohort 2 | Cohort 1 | Cohort 2 |
|---|---|---|---|---|---|---|
| median | 25.400 | 24.100 | 25.400 | 23.750 | 25.400 | 25.300 |
| average | 24.861 | 24.233 | 24.861 | 23.803 | 24.861 | 26.021 |
| stdev | 12.554 | 13.682 | 12.554 | 13.219 | 12.554 | 15.468 |
| p (t-test) |  | 0.807 |  | 0.659 |  | 0.721 |
| min | 0.416 | 0.416 | 0.416 | 0.416 | 0.416 | 0.416 |
| max | 66.200 | 52.700 | 66.200 | 48.600 | 66.200 | 56.700 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.50 | 0.057 | 419 | 27 | 0.948 |
| 24 hours | 0.45 | 0.049 | 419 | 36 | 0.354 |
| 48 hours | 0.51 | 0.070 | 419 | 18 | 0.923 |

sCr only

| Time prior AKI stage | AUC | SE | nCohort 1 | nCohort 2 | p |
|---|---|---|---|---|---|
| 0 hours | 0.60 | 0.135 | 518 | 5 | 0.472 |
| 24 hours | 0.46 | 0.094 | 518 | 9 | 0.633 |
| 48 hours | 0.66 | 0.114 | 518 | 7 | 0.161 |

UO only

| Time prior AKI stage | AUC | SE | nCohort 1 | nCohort 2 | p |
|---|---|---|---|---|---|
| 0 hours | 0.49 | 0.058 | 352 | 26 | 0.859 |
| 24 hours | 0.48 | 0.054 | 352 | 30 | 0.718 |
| 48 hours | 0.53 | 0.075 | 352 | 16 | 0.721 |

sCr or UO

| Time prior AKI stage | Cutoff value | sens | spec | Quartile | OR | 95% CI of OR | |
|---|---|---|---|---|---|---|---|
| 0 hours | 20.1 | 74% | 32% | 1 |  |  |  |
|  | 12.6 | 81% | 17% | 2 | 0.6 | 0.3 | 1.2 |
|  | 0 | 100% | 0% | 3 | 0.9 | 0.5 | 1.5 |
|  | 30.6 | 30% | 71% | 4 | 0.9 | 0.5 | 1.5 |
|  | 33.4 | 19% | 81% |  |  |  |  |
|  | 39.6 | 7% | 90% |  |  |  |  |
| 24 hours | 16.4 | 72% | 23% | 1 |  |  |  |
|  | 12.6 | 81% | 17% | 2 | 0.4 | 0.2 | 0.8 |
|  | 0 | 100% | 0% | 3 | 1.1 | 0.7 | 1.7 |
|  | 30.6 | 28% | 71% | 4 | 1.1 | 0.7 | 1.7 |
|  | 33.4 | 22% | 81% |  |  |  |  |
|  | 39.6 | 11% | 90% |  |  |  |  |
| 48 hours | 20.1 | 72% | 32% | 1 |  |  |  |
|  | 0.416 | 89% | 4% | 2 | 0.8 | 0.3 | 2.0 |
|  | 0 | 100% | 0% | 3 | 0.6 | 0.2 | 1.7 |
|  | 30.6 | 39% | 71% | 4 | 1.2 | 0.6 | 2.6 |
|  | 33.4 | 28% | 81% |  |  |  |  |
|  | 39.6 | 17% | 90% |  |  |  |  |

sCr only

| Time prior AKI stage | Cutoff value | sens | spec | Quartile | OR | 95% CI of OR | |
|---|---|---|---|---|---|---|---|
| 0 hours | 25.1 | 80% | 51% | 1 |  |  |  |
|  | 25.1 | 80% | 51% | 2 | 0.0 | 0.0 | na |
|  | 15.7 | 100% | 22% | 3 | 2.0 | 0.1 | 39.0 |
|  | 30.4 | 40% | 70% | 4 | 2.0 | 0.1 | 39.0 |
|  | 33.4 | 20% | 81% |  |  |  |  |
|  | 39.6 | 20% | 90% |  |  |  |  |
| 24 hours | 12.6 | 78% | 17% | 1 |  |  |  |
|  | 8.12 | 89% | 10% | 2 | 0.5 | 0.0 | 9.7 |
|  | 0 | 100% | 0% | 3 | 1.5 | 0.3 | 8.0 |
|  | 30.4 | 33% | 70% | 4 | 1.5 | 0.3 | 8.0 |
|  | 33.4 | 22% | 81% |  |  |  |  |
|  | 39.6 | 11% | 90% |  |  |  |  |
| 48 hours | 32.9 | 71% | 79% | 1 |  |  |  |
|  | 11.1 | 86% | 15% | 2 | 0.0 | 0.0 | na |

## FIG. 2 - 8

| | | | | | | | |
|---|---|---|---|---|---|---|---|
| | 0.416 | 100% | 5% | 3 | 0.0 | 0.0 | na |
| | 30.4 | 71% | 70% | 4 | 2.5 | 0.6 | 10.3 |
| | 33.4 | 43% | 81% | | | | |
| | 39.6 | 29% | 90% | | | | |

UO only

| Time prior AKI stage | Cutoff value | sens | spec | Quartile | OR | 95% CI of OR | |
|---|---|---|---|---|---|---|---|
| 0 hours | 20.1 | 73% | 32% | 1 | | | |
| | 12.5 | 81% | 18% | 2 | 0.7 | 0.3 | 1.4 |
| | 0 | 100% | 0% | 3 | 1.0 | 0.5 | 1.8 |
| | 30.6 | 27% | 70% | 4 | 1.0 | 0.6 | 1.9 |
| | 33.6 | 19% | 80% | | | | |
| | 40.1 | 8% | 90% | | | | |
| 24 hours | 18 | 70% | 26% | 1 | | | |
| | 12.5 | 83% | 18% | 2 | 0.4 | 0.2 | 0.9 |
| | 5.81 | 90% | 7% | 3 | 1.0 | 0.6 | 1.6 |
| | 30.6 | 30% | 70% | 4 | 0.9 | 0.5 | 1.5 |
| | 33.6 | 23% | 80% | | | | |
| | 40.1 | 13% | 90% | | | | |
| 48 hours | 21.7 | 75% | 38% | 1 | | | |
| | 20.1 | 81% | 32% | 2 | 1.7 | 0.6 | 5.1 |
| | 0 | 100% | 0% | 3 | 1.0 | 0.3 | 3.9 |
| | 30.6 | 38% | 70% | 4 | 1.7 | 0.6 | 5.1 |
| | 33.6 | 25% | 80% | | | | |
| | 40.1 | 19% | 90% | | | | |

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 13.225 | 21.012 | 13.225 | 107.639 | 13.225 | 40.253 |
| average | 29.987 | 40.904 | 29.987 | 75.637 | 29.987 | 40.253 |
| stdev | 44.245 | 46.289 | 44.245 | 58.461 | 44.245 | 56.015 |
| p (t-test) | | 0.510 | | 0.086 | | 0.748 |
| min | 0.036 | 0.036 | 0.036 | 8.162 | 0.036 | 0.644 |
| max | 270.067 | 111.111 | 270.067 | 111.111 | 270.067 | 79.861 |
| n (Samp) | 78 | 8 | 78 | 3 | 78 | 2 |
| n (Pat) | 56 | 8 | 56 | 3 | 56 | 2 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 13.225 | 0.036 | 13.225 | 0.036 | 13.225 | 53.373 |
| average | 31.603 | 111.111 | 31.603 | 111.111 | 31.603 | 53.373 |
| stdev | 44.147 | na | 44.147 | na | 44.147 | 37.459 |
| p (t-test) | | na | | na | | 0.491 |
| min | 0.036 | 111.111 | 0.036 | 111.111 | 0.036 | 26.885 |
| max | 270.067 | 111.111 | 270.067 | 111.111 | 270.067 | 79.861 |
| n (Samp) | 96 | 1 | 96 | 1 | 96 | 2 |
| n (Pat) | 69 | 1 | 69 | 1 | 69 | 2 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 11.173 | 21.012 | 11.173 | 57.900 | 11.173 | 0.036 |
| average | 28.646 | 40.904 | 28.646 | 57.900 | 28.646 | 0.644 |
| stdev | 47.943 | 46.289 | 47.943 | 70.341 | 47.943 | na |
| p (t-test) | | 0.497 | | 0.404 | | na |
| min | 0.036 | 0.036 | 0.036 | 8.162 | 0.036 | 0.644 |
| max | 270.067 | 111.111 | 270.067 | 107.639 | 270.067 | 0.644 |
| n (Samp) | 61 | 8 | 61 | 2 | 61 | 1 |
| n (Pat) | 43 | 8 | 43 | 2 | 43 | 1 |

sCr or UO

| Time prior AKI stage | AUC | SE | nCohort 1 | nCohort 2 | p |
|---|---|---|---|---|---|
| 0 hours | 0.55 | 0.110 | 78 | 8 | 0.621 |
| 24 hours | 0.78 | 0.160 | 78 | 3 | 0.075 |

**FIG. 2 - 9**

**sCr only**

| | | | | | |
|---|---|---|---|---|---|
| 48 hours | 0.53 | 0.211 | 78 | 2 | 0.903 |

| Time prior AKI stage | AUC | SE | nCohort 1 | nCohort 2 | p |
|---|---|---|---|---|---|
| 0 hours | 0.97 | 0.112 | 96 | 1 | 0.000 |
| 24 hours | 0.97 | 0.112 | 96 | 1 | 0.000 |
| 48 hours | 0.76 | 0.201 | 96 | 2 | 0.199 |

**UO only**

| Time prior AKI stage | AUC | SE | nCohort 1 | nCohort 2 | p |
|---|---|---|---|---|---|
| 0 hours | 0.57 | 0.112 | 61 | 8 | 0.533 |
| 24 hours | 0.71 | 0.210 | 61 | 2 | 0.320 |
| 48 hours | 0.12 | 0.116 | 61 | 1 | 0.001 |

**sCr or UO**

| Time prior AKI stage | Cutoff value | sens | spec | Quartile | OR | 95% CI of OR | |
|---|---|---|---|---|---|---|---|
| 0 hours | 6.0137457 | 75% | 35% | 1 | | | |
| | 0 | 100% | 0% | 2 | .0.5 | 0.0 | 10.4 |
| | 0 | 100% | 0% | 3 | 1.0 | 0.1 | 8.7 |
| | 35.227273 | 38% | 71% | 4 | 1.5 | 0.2 | 9.4 |
| | 54.487179 | 38% | 81% | | | | |
| | 74.652778 | 25% | 91% | | | | |
| 24 hours | 7.6815642 | 100% | 40% | 1 | | | |
| | 7.6815642 | 100% | 40% | 2 | na | na | na |
| | 7.6815642 | 100% | 40% | 3 | na | na | na |
| | 35.227273 | 67% | 71% | 4 | na | na | na |
| | 54.487179 | 67% | 81% | | | | |
| | 74.652778 | 67% | 91% | | | | |
| 48 hours | 0.0360285 | 100% | 12% | 1 | | | |
| | 0.0360285 | 100% | 12% | 2 | 0.0 | 0.0 | na |
| | 0.0360285 | 100% | 12% | 3 | 0.0 | 0.0 | na |
| | 35.227273 | 50% | 71% | 4 | 1.0 | 0.0 | 61.9 |
| | 54.487179 | 50% | 81% | | | | |
| | 74.652778 | 50% | 91% | | | | |

**UO only**

| Time prior AKI stage | Cutoff value | sens | spec | Quartile | OR | 95% CI of OR | |
|---|---|---|---|---|---|---|---|
| 0 hours | 6.0137457 | 75% | 38% | 1 | | | |
| | 0 | 100% | 0% | 2 | 0.5 | 0.0 | 11.4 |
| | 0 | 100% | 0% | 3 | 1.0 | 0.1 | 9.2 |
| | 30.377095 | 38% | 72% | 4 | 1.5 | 0.2 | 10.0 |
| | 41.666667 | 38% | 80% | | | | |
| | 74.652778 | 25% | 90% | | | | |
| 24 hours | 7.6815642 | 100% | 44% | 1 | | | |
| | 7.6815642 | 100% | 44% | 2 | na | na | na |
| | 7.6815642 | 100% | 44% | 3 | na | na | na |
| | 30.377095 | 50% | 72% | 4 | na | na | na |
| | 41.666667 | 50% | 80% | | | | |
| | 74.652778 | 50% | 90% | | | | |
| 48 hours | 0.0360285 | 100% | 11% | 1 | | | |
| | 0.0360285 | 100% | 11% | 2 | na | na | na |
| | 0.0360285 | 100% | 11% | 3 | na | na | na |
| | 30.377095 | 0% | 72% | 4 | na | na | na |
| | 41.666667 | 0% | 80% | | | | |
| | 74.652778 | 0% | 90% | | | | |

**Tubulointerstitial nephritis antigen**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.225 | 0.308 | 0.225 | 0.721 | 0.225 | 0.688 |
| average | 4.669 | 1.844 | 4.669 | 1.720 | 4.669 | 3.017 |
| stdev | 39.078 | 4.980 | 39.078 | 3.307 | 39.078 | 5.952 |

## FIG. 2 - 10

| | | 0.767 | | | 0.713 | | | 0.879 |
|---|---|---|---|---|---|---|---|---|
| p (t-test) | | 0.767 | | | 0.713 | | | 0.879 |
| min | 0.000 | 0.004 | 0.000 | 0.104 | | 0.000 | 0.225 | |
| max | 480.918 | 20.934 | 480.918 | 14.608 | | 480.918 | 19.127 | |
| n (Samp) | 165 | 17 | 165 | 24 | | 165 | 13 | |
| n (Pat) | 106 | 17 | 106 | 24 | | 106 | 13 | |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.241 | 0.156 | 0.241 | 0.835 | 0.241 | 2.170 |
| average | 4.142 | 0.156 | 4.142 | 0.765 | 4.142 | 3.641 |
| stdev | 34.385 | 0.215 | 34.385 | 0.443 | 34.385 | 5.398 |
| p (t-test) | | 0.870 | | 0.827 | | 0.974 |
| min | 0.000 | 0.004 | 0.000 | 0.270 | 0.000 | 0.189 |
| max | 480.918 | 0.308 | 480.918 | 1.410 | 480.918 | 13.102 |
| n (Samp) | 214 | 2 | 214 | 5 | 214 | 5 |
| n (Pat) | 125 | 2 | 125 | 5 | 125 | 5 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.225 | 0.458 | 0.225 | 0.638 | 0.225 | 0.688 |
| average | 5.288 | 1.884 | 5.288 | 1.808 | 5.288 | 2.101 |
| stdev | 42.256 | 4.969 | 42.256 | 3.448 | 42.256 | 5.135 |
| p (t-test) | | 0.741 | | 0.701 | | 0.787 |
| min | 0.000 | 0.118 | 0.000 | 0.104 | 0.000 | 0.004 |
| max | 480.918 | 20.934 | 480.918 | 14.608 | 480.918 | 19.127 |
| n (Samp) | 141 | 17 | 141 | 22 | 141 | 13 |
| n (Pat) | 85 | 17 | 85 | 22 | 85 | 13 |

sCr or UO

| Time prior AKI stage | AUC | SE | nCohort 1 | nCohort 2 | p |
|---|---|---|---|---|---|
| 0 hours | 0.57 | 0.076 | 165 | 17 | 0.337 |
| 24 hours | 0.62 | 0.065 | 165 | 24 | 0.061 |
| 48 hours | 0.69 | 0.084 | 165 | 13 | 0.026 |

sCr only

| Time prior AKI stage | AUC | SE | nCohort 1 | nCohort 2 | p |
|---|---|---|---|---|---|
| 0 hours | 0.27 | 0.145 | 214 | 2 | 0.108 |
| 24 hours | 0.69 | 0.133 | 214 | 5 | 0.157 |
| 48 hours | 0.65 | 0.135 | 214 | 5 | 0.281 |

UO only

| Time prior AKI stage | AUC | SE | nCohort 1 | nCohort 2 | p |
|---|---|---|---|---|---|
| 0 hours | 0.59 | 0.076 | 141 | 17 | 0.215 |
| 24 hours | 0.62 | 0.068 | 141 | 22 | 0.088 |
| 48 hours | 0.66 | 0.085 | 141 | 13 | 0.057 |

sCr or UO

| Time prior AKI stage | Cutoff value | sens | spec | Quartile | OR | 95% CI of OR | |
|---|---|---|---|---|---|---|---|
| 0 hours | 0.2077793 | 94% | 15% | 1 | | | |
| | 0.2077793 | 94% | 15% | 2 | 0.0 | 0.0 | na |
| | 0.2077793 | 94% | 15% | 3 | 0.5 | 0.2 | 1.3 |
| | 0.5926724 | 35% | 70% | 4 | 0.8 | 0.4 | 1.7 |
| | 0.9698276 | 24% | 81% | | | | |
| | 1.3135453 | 18% | 90% | | | | |
| 24 hours | 0.2077793 | 88% | 15% | 1 | | | |
| | 0.2077793 | 88% | 15% | 2 | 0.1 | 0.0 | 1.3 |
| | 0.1814851 | 92% | 13% | 3 | 1.0 | 0.5 | 1.9 |
| | 0.5926724 | 58% | 70% | 4 | 1.3 | 0.7 | 2.4 |
| | 0.9698276 | 33% | 81% | | | | |
| | 1.3135453 | 25% | 90% | | | | |
| 48 hours | 0.4580337 | 77% | 64% | 1 | | | |
| | 0.2077793 | 100% | 15% | 2 | 2.0 | 0.1 | 41.4 |
| | 0.2077793 | 100% | 15% | 3 | 6.8 | 0.6 | 73.6 |
| | 0.5926724 | 62% | 70% | 4 | 4.2 | 0.3 | 53.4 |
| | 0.9698276 | 23% | 81% | | | | |
| | 1.3135453 | 23% | 90% | | | | |

## FIG. 2 - 11

sCr only

| Time prior AKI stage | Cutoff value | sens | spec | Quartile | OR | 95% CI of OR | |
|---|---|---|---|---|---|---|---|
| 0 hours | 2.884E-16 | 100% | 0% | 1 | | | |
| | 2.884E-16 | 100% | 0% | 2 | na | na | na |
| | 2.884E-16 | 100% | 0% | 3 | na | na | na |
| | 0.8021594 | 0% | 71% | 4 | na | na | na |
| | 1.0302616 | 0% | 80% | | | | |
| | 1.5625 | 0% | 91% | | | | |
| 24 hours | 0.434988 | 80% | 57% | 1 | | | |
| | 0.434988 | 80% | 57% | 2 | na | na | na |
| | 0.2693966 | 100% | 51% | 3 | na | na | na |
| | 0.8021594 | 60% | 71% | 4 | na | na | na |
| | 1.0302616 | 20% | 80% | | | | |
| | 1.5625 | 0% | 91% | | | | |
| 48 hours | 0.2077793 | 80% | 13% | 1 | | | |
| | 0.2077793 | 80% | 13% | 2 | 1.0 | 0.0 | 53.2 |
| | 0.1870013 | 100% | 12% | 3 | 0.0 | 0.0 | na |
| | 0.8021594 | 60% | 71% | 4 | 3.1 | 0.2 | 45.0 |
| | 1.0302616 | 60% | 80% | | | | |
| | 1.5625 | 60% | 91% | | | | |

UO only

| Time prior AKI stage | Cutoff value | sens | spec | Quartile | OR | 95% CI of OR | |
|---|---|---|---|---|---|---|---|
| 0 hours | 0.1870013 | 94% | 16% | 1 | | | |
| | 0.1870013 | 94% | 16% | 2 | 2.6 | 0.6 | 11.6 |
| | 0.1870013 | 94% | 16% | 3 | 2.1 | 0.4 | 10.3 |
| | 0.5402261 | 47% | 71% | 4 | 3.3 | 0.8 | 13.5 |
| | 0.9698276 | 24% | 81% | | | | |
| | 1.2997909 | 18% | 90% | | | | |
| 24 hours | 0.1870013 | 86% | 16% | 1 | | | |
| | 0.1870013 | 86% | 16% | 2 | 1.7 | 0.5 | 5.4 |
| | 0.1814851 | 91% | 15% | 3 | 2.1 | 0.7 | 6.3 |
| | 0.5402261 | 55% | 71% | 4 | 3.0 | 1.1 | 8.2 |
| | 0.9698276 | 36% | 81% | | | | |
| | 1.2997909 | 27% | 90% | | | | |
| 48 hours | 0.4580337 | 77% | 66% | 1 | | | |
| | 0.1870013 | 92% | 16% | 2 | 0.0 | 0.0 | na |
| | 0.1870013 | 92% | 16% | 3 | 1.8 | 0.6 | 5.6 |
| | 0.5402261 | 62% | 71% | 4 | 1.7 | 0.5 | 5.5 |
| | 0.9698276 | 23% | 81% | | | | |
| | 1.2997909 | 23% | 90% | | | | |

**FIG. 2 - 12**

**Netrin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 3.593 | 0.211 | 3.593 | 0.211 | 3.593 |
| average | 3.264 | 6.068 | 3.264 | 6.068 | 3.264 | 6.068 |
| stdev | 5.300 | 6.484 | 5.300 | 6.484 | 5.300 | 6.484 |
| p (t-test) | | 0.053 | | 0.053 | | 0.053 |
| min | 0.211 | 0.211 | 0.211 | 0.211 | 0.211 | 0.211 |
| max | 21.067 | 17.556 | 21.067 | 17.556 | 21.067 | 17.556 |
| n (Samp) | 55 | 22 | 55 | 22 | 55 | 22 |
| n (Pat) | 55 | 22 | 55 | 22 | 55 | 22 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 2.802 | 0.228 | 2.802 | 0.228 | 2.802 | 0.228 |
| stdev | 4.433 | 0.035 | 4.433 | 0.035 | 4.433 | 0.035 |
| p (t-test) | | 0.266 | | 0.266 | | 0.266 |
| min | 0.211 | 0.211 | 0.211 | 0.211 | 0.211 | 0.211 |
| max | 15.734 | 0.281 | 15.734 | 0.281 | 15.734 | 0.281 |
| n (Samp) | 21 | 4 | 21 | 4 | 21 | 4 |
| n (Pat) | 21 | 4 | 21 | 4 | 21 | 4 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.211 | 4.100 | 0.211 | 4.100 | 0.211 | 4.100 |
| average | 4.180 | 5.915 | 4.180 | 5.915 | 4.180 | 5.915 |
| stdev | 6.530 | 5.913 | 6.530 | 5.913 | 6.530 | 5.913 |
| p (t-test) | | 0.344 | | 0.344 | | 0.344 |
| min | 0.211 | 0.211 | 0.211 | 0.211 | 0.211 | 0.211 |
| max | 21.067 | 16.108 | 21.067 | 16.108 | 21.067 | 16.108 |
| n (Samp) | 44 | 17 | 44 | 17 | 44 | 17 |
| n (Pat) | 44 | 17 | 44 | 17 | 44 | 17 |

sCr or UO

| Time prior AKI stage | AUC | SE | nCohort 1 | nCohort 2 | p |
|---|---|---|---|---|---|
| 0 hours | 0.65 | 0.072 | 55 | 22 | 0.038 |
| 24 hours | 0.65 | 0.072 | 55 | 22 | 0.038 |
| 48 hours | 0.65 | 0.072 | 55 | 22 | 0.038 |

sCr only

| Time prior AKI stage | AUC | SE | nCohort 1 | nCohort 2 | p |
|---|---|---|---|---|---|
| 0 hours | 0.36 | 0.144 | 21 | 4 | 0.341 |
| 24 hours | 0.36 | 0.144 | 21 | 4 | 0.341 |
| 48 hours | 0.36 | 0.144 | 21 | 4 | 0.341 |

UO only

| Time prior AKI stage | AUC | SE | nCohort 1 | nCohort 2 | p |
|---|---|---|---|---|---|
| 0 hours | 0.63 | 0.083 | 44 | 17 | 0.113 |
| 24 hours | 0.63 | 0.083 | 44 | 17 | 0.113 |
| 48 hours | 0.63 | 0.083 | 44 | 17 | 0.113 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 3.0 | 0.6 | 15.5 |
| | 0 | 100% | 0% | 3 | 3.9 | 0.8 | 18.9 |
| | 3.0857271 | 50% | 71% | 4 | 7.0 | 1.6 | 30.9 |
| | 4.4883303 | 45% | 80% | | | | |
| | 12.289326 | 23% | 91% | | | | |
| 24 hours | 0 | 100% | 0% | 1 | | | |
| | 0 | 100% | 0% | 2 | 3.0 | 0.6 | 15.5 |
| | 0 | 100% | 0% | 3 | 3.9 | 0.8 | 18.9 |
| | 3.0857271 | 50% | 71% | 4 | 7.0 | 1.6 | 30.9 |
| | 4.4883303 | 45% | 80% | | | | |

# FIG. 3 - 1

|  | 12.289326 | 23% | 91% |  |  |  |  |
|---|---|---|---|---|---|---|---|
| 48 hours | 0 | 100% | 0% | 1 |  |  |  |
|  | 0 | 100% | 0% | 2 | 3.0 | 0.6 | 15.5 |
|  | 0 | 100% | 0% | 3 | 3.9 | 0.8 | 18.9 |
|  | 3.0857271 | 50% | 71% | 4 | 7.0 | 1.6 | 30.9 |
|  | 4.4883303 | 45% | 80% |  |  |  |  |
|  | 12.289326 | 23% | 91% |  |  |  |  |

UO only

| Time prior AKI stage | Cutoff value | sens | spec | Quartile | OR | 95% CI of OR | |
|---|---|---|---|---|---|---|---|
| 0 hours | 0.2805206 | 71% | 61% | 1 |  |  |  |
|  | 0 | 100% | 0% | 2 | 0.6 | 0.1 | 4.3 |
|  | 0 | 100% | 0% | 3 | 2.7 | 0.7 | 10.4 |
|  | 4.0998542 | 47% | 73% | 4 | 2.4 | 0.6 | 9.2 |
|  | 10.30816 | 24% | 82% |  |  |  |  |
|  | 15.800562 | 6% | 93% |  |  |  |  |
| 24 hours | 0.2805206 | 71% | 61% | 1 |  |  |  |
|  | 0 | 100% | 0% | 2 | 0.6 | 0.1 | 4.3 |
|  | 0 | 100% | 0% | 3 | 2.7 | 0.7 | 10.4 |
|  | 4.0998542 | 47% | 73% | 4 | 2.4 | 0.6 | 9.2 |
|  | 10.30816 | 24% | 82% |  |  |  |  |
|  | 15.800562 | 6% | 93% |  |  |  |  |
| 48 hours | 0.2805206 | 71% | 61% | 1 |  |  |  |
|  | 0 | 100% | 0% | 2 | 0.6 | 0.1 | 4.3 |
|  | 0 | 100% | 0% | 3 | 2.7 | 0.7 | 10.4 |
|  | 4.0998542 | 47% | 73% | 4 | 2.4 | 0.6 | 9.2 |
|  | 10.30816 | 24% | 82% |  |  |  |  |
|  | 15.800562 | 6% | 93% |  |  |  |  |

# FIG. 3 - 2

Growth-regulated alpha protein .

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 2.181 | 1.141 | 2.181 | 3.744 | 2.181 | 3.744 |
| average | 55.603 | 6.449 | 55.603 | 8.476 | 55.603 | 8.476 |
| stdev | 291.484 | 10.560 | 291.484 | 11.679 | 291.484 | 11.679 |
| p (t-test) | | 0.639 | | 0.697 | | 0.697 |
| min | 0.368 | 0.368 | 0.368 | 0.368 | 0.368 | 0.368 |
| max | 1754.386 | 30.516 | 1754.386 | 30.516 | 1754.386 | 30.516 |
| n (Samp) | 36 | 8 | 36 | 6 | 36 | 6 |
| n (Pat) | 36 | 8 | 36 | 6 | 36 | 6 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.368 | 0.368 | 0.368 | 1.141 | 0.368 | 1.141 |
| average | 36.842 | 0.754 | 36.842 | 1.141 | 36.842 | 1.141 |
| stdev | 226.235 | 0.773 | 226.235 | 1.093 | 226.235 | 1.093 |
| p (t-test) | | 0.753 | | 0.826 | | 0.826 |
| min | 0.368 | 0.368 | 0.368 | 0.368 | 0.368 | 0.368 |
| max | 1754.386 | 1.913 | 1754.386 | 1.913 | 1754.386 | 1.913 |
| n (Samp) | 60 | 4 | 60 | 2 | 60 | 2 |
| n (Pat) | 60 | 4 | 60 | 2 | 60 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 3.189 | 8.846 | 3.189 | 8.846 | 3.189 | 8.846 |
| average | 74.235 | 12.144 | 74.235 | 12.144 | 74.235 | 12.144 |
| stdev | 342.889 | 13.158 | 342.889 | 13.158 | 342.889 | 13.158 |
| p (t-test) | | 0.724 | | 0.724 | | 0.724 |
| min | 0.368 | 0.368 | 0.368 | 0.368 | 0.368 | 0.368 |
| max | 1754.386 | 30.516 | 1754.386 | 30.516 | 1754.386 | 30.516 |
| n (Samp) | 26 | 4 | 26 | 4 | 26 | 4 |
| n (Pat) | 26 | 4 | 26 | 4 | 26 | 4 |

sCr or UO

| Time prior AKI stage | AUC | SE | nCohort 1 | nCohort 2 | p |
|---|---|---|---|---|---|
| 0 hours | 0.48 | 0.113 | 36 | 8 | 0.866 |
| 24 hours | 0.56 | 0.131 | 36 | 6 | 0.634 |
| 48 hours | 0.56 | 0.131 | 36 | 6 | 0.634 |

sCr only

| Time prior AKI stage | AUC | SE | nCohort 1 | nCohort 2 | p |
|---|---|---|---|---|---|
| 0 hours | 0.34 | 0.127 | 60 | 4 | 0.220 |
| 24 hours | 0.43 | 0.197 | 60 | 2 | 0.720 |
| 48 hours | 0.43 | 0.197 | 60 | 2 | 0.720 |

UO only

| Time prior AKI stage | AUC | SE | nCohort 1 | nCohort 2 | p |
|---|---|---|---|---|---|
| 0 hours | 0.64 | 0.160 | 26 | 4 | 0.367 |
| 24 hours | 0.64 | 0.160 | 26 | 4 | 0.367 |
| 48 hours | 0.64 | 0.160 | 26 | 4 | 0.367 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.0 | 0.1 | 11.0 |
| | 0 | 100% | 0% | 3 | 0.0 | 0.0 | na |
| | 7.0422535 | 25% | 72% | 4 | 2.6 | 0.4 | 18.4 |
| | 10.841837 | 25% | 81% | | | | |
| | 36.847015 | 0% | 92% | | | | |
| 24 hours | 0 | 100% | 0% | 1 | | | |
| | 0 | 100% | 0% | 2 | 0.4 | 0.0 | 11.8 |
| | 0 | 100% | 0% | 3 | 0.4 | 0.0 | 13.4 |
| | 7.0422535 | 33% | 72% | 4 | 0.9 | 0.1 | 10.0 |

## FIG. 4 - 1

| | 10.841837 | 33% | 81% | | | | | |
|---|---|---|---|---|---|---|---|---|
| | 36.847015 | 0% | 92% | | | | | |
| 48 hours | 0 | 100% | 0% | 1 | | | | |
| | 0 | 100% | 0% | 2 | 0.4 | 0.0 | 11.8 | |
| | 0 | 100% | 0% | 3 | 0.4 | 0.0 | 13.4 | |
| | 7.0422535 | 33% | 72% | 4 | 0.9 | 0.1 | 10.0 | |
| | 10.841837 | 33% | 81% | | | | | |
| | 36.847015 | 0% | 92% | | | | | |

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 19.902 | 78.251 | 19.902 | 67.262 | 19.902 | 53.373 |
| average | 34.968 | 94.697 | 34.968 | 100.418 | 34.968 | 94.135 |
| stdev | 42.968 | 112.170 | 42.968 | 130.087 | 42.968 | 131.094 |
| p (t-test) | | 0.015 | | 0.020 | | 0.035 |
| min | 0.036 | 0.036 | 0.036 | 2.792 | 0.036 | 0.644 |
| max | 221.429 | 346.990 | 221.429 | 346.990 | 221.429 | 346.990 |
| n (Samp) | 36 | 8 | 36 | 6 | 36 | 6 |
| n (Pat) | 36 | 8 | 36 | 6 | 36 | 6 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 16.045 | 111.111 | 16.045 | 229.051 | 16.045 | 213.426 |
| average | 35.530 | 142.312 | 35.530 | 229.051 | 35.530 | 213.426 |
| stdev | 50.240 | 146.153 | 50.240 | 166.792 | 50.240 | 188.889 |
| p (t-test) | | 0.001 | | 0.000 | | 0.000 |
| min | 0.036 | 0.036 | 0.036 | 111.111 | 0.036 | 79.861 |
| max | 270.067 | 346.990 | 270.067 | 346.990 | 270.067 | 346.990 |
| n (Samp) | 60 | 4 | 60 | 2 | 60 | 2 |
| n (Pat) | 60 | 4 | 60 | 2 | 60 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 18.156 | 37.875 | 18.156 | 16.987 | 18.156 | 14.839 |
| average | 35.686 | 47.082 | 35.686 | 36.101 | 35.686 | 34.490 |
| stdev | 47.364 | 44.175 | 47.364 | 48.833 | 47.364 | 50.196 |
| p (t-test) | | 0.655 | | 0.987 | | 0.963 |
| min | 0.036 | 4.940 | 0.036 | 2.792 | 0.036 | 0.644 |
| max | 221.429 | 107.639 | 221.429 | 107.639 | 221.429 | 107.639 |
| n (Samp) | 26 | 4 | 26 | 4 | 26 | 4 |
| n (Pat) | 26 | 4 | 26 | 4 | 26 | 4 |

sCr or UO

| Time prior AKI stage | AUC | SE | nCohort 1 | nCohort 2 | p |
|---|---|---|---|---|---|
| 0 hours | 0.69 | 0.112 | 36 | 8 | 0.099 |
| 24 hours | 0.67 | 0.129 | 36 | 6 | 0.191 |
| 48 hours | 0.63 | 0.131 | 36 | 6 | 0.340 |

sCr only

| Time prior AKI stage | AUC | SE | nCohort 1 | nCohort 2 | p |
|---|---|---|---|---|---|
| 0 hours | 0.74 | 0.146 | 60 | 4 | 0.098 |
| 24 hours | 0.98 | 0.072 | 60 | 2 | 0.000 |
| 48 hours | 0.93 | 0.130 | 60 | 2 | 0.001 |

UO only

| Time prior AKI stage | AUC | SE | nCohort 1 | nCohort 2 | p |
|---|---|---|---|---|---|
| 0 hours | 0.63 | 0.160 | 26 | 4 | 0.401 |
| 24 hours | 0.51 | 0.158 | 26 | 4 | 0.952 |
| 48 hours | 0.45 | 0.153 | 26 | 4 | 0.754 |

sCr or UO

| Time prior AKI stage | Cutoff value | sens | spec | Quartile | OR | 95% CI of OR | |
|---|---|---|---|---|---|---|---|
| 0 hours | 23.393855 | 75% | 58% | 1 | | | |

## FIG. 4 - 2

| | 2.7920962 | 88% | 22% | 2 | 0.0 | 0.0 | na |
|---|---|---|---|---|---|---|---|
| | 0 | 100% | 0% | 3 | 1.0 | 0.1 | 11.0 |
| | 44.318182 | 63% | 72% | 4 | 2.6 | 0.4 | 18.4 |
| | 60.897436 | 50% | 81% | | | | |
| | 81.597222 | 50% | 92% | | | | |
| 24 hours | 6.0137457 | 83% | 28% | 1 | | | |
| | 6.0137457 | 83% | 28% | 2 | 0.9 | 0.0 | 68.6 |
| | 2.4441341 | 100% | 19% | 3 | 1.0 | 0.0 | 77.9 |
| | 44.318182 | 50% | 72% | 4 | 3.4 | 0.2 | 73.1 |
| | 60.897436 | 50% | 81% | | | | |
| | 81.597222 | 50% | 92% | | | | |
| 48 hours | 2.4441341 | 83% | 19% | 1 | | | |
| | 2.4441341 | 83% | 19% | 2 | 0.0 | 0.0 | na |
| | 0.0360285 | 100% | 8% | 3 | 0.4 | 0.0 | 13.4 |
| | 44.318182 | 50% | 72% | 4 | 1.5 | 0.2 | 12.5 |
| | 60.897436 | 50% | 81% | | | | |
| | 81.597222 | 33% | 92% | | | | |

sCr only

| Time prior AKI stage | Cutoff value | sens | spec | Quartile | OR | 95% CI of OR | |
|---|---|---|---|---|---|---|---|
| 0 hours | 107.63889 | 75% | 95% | 1 | | | |
| | 0 | 100% | 0% | 2 | 0.0 | 0.0 | na |
| | 0 | 100% | 0% | 3 | 0.0 | 0.0 | na |
| | 37.5 | 75% | 70% | 4 | 3.5 | 0.2 | 62.6 |
| | 60.897436 | 75% | 80% | | | | |
| | 83.333333 | 75% | 90% | | | | |
| 24 hours | 107.63889 | 100% | 95% | 1 | | | |
| | 107.63889 | 100% | 95% | 2 | na | na | na |
| | 107.63889 | 100% | 95% | 3 | na | na | na |
| | 37.5 | 100% | 70% | 4 | na | na | na |
| | 60.897436 | 100% | 80% | | | | |
| | 83.333333 | 100% | 90% | | | | |
| 48 hours | 76.388889 | 100% | 85% | 1 | | | |
| | 76.388889 | 100% | 85% | 2 | na | na | na |
| | 76.388889 | 100% | 85% | 3 | na | na | na |
| | 37.5 | 100% | 70% | 4 | na | na | na |
| | 60.897436 | 100% | 80% | | | | |
| | 83.333333 | 50% | 90% | | | | |

UO only

| Time prior AKI stage | Cutoff value | sens | spec | Quartile | OR | 95% CI of OR | |
|---|---|---|---|---|---|---|---|
| 0 hours | 23.393855 | 75% | 62% | 1 | | | |
| | 2.7920962 | 100% | 23% | 2 | 0.0 | 0.0 | na |
| | 2.7920962 | 100% | 23% | 3 | 2.4 | 0.1 | 93.1 |
| | 41.666667 | 50% | 73% | 4 | 0.9 | 0.0 | 79.2 |
| | 60.897436 | 25% | 81% | | | | |
| | 81.597222 | 25% | 92% | | | | |
| 24 hours | 6.0137457 | 75% | 27% | 1 | | | |
| | 2.4441341 | 100% | 15% | 2 | 0.9 | 0.0 | 79.2 |
| | 2.4441341 | 100% | 15% | 3 | 1.0 | 0.0 | 96.9 |
| | 41.666667 | 25% | 73% | 4 | 0.9 | 0.0 | 79.2 |
| | 60.897436 | 25% | 81% | | | | |
| | 81.597222 | 25% | 92% | | | | |
| 48 hours | 2.4441341 | 75% | 15% | 1 | | | |
| | 0.0360285 | 100% | 4% | 2 | 1.2 | 0.0 | 107.9 |
| | 0.0360285 | 100% | 4% | 3 | 0.0 | 0.0 | na |
| | 41.666667 | 25% | 73% | 4 | 2.8 | 0.1 | 103.7 |
| | 60.897436 | 25% | 81% | | | | |
| | 81.597222 | 25% | 92% | | | | |

**Tubulointerstitial nephritis antigen**

sCr or
UO

# FIG. 4 - 3

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.431 | 3.314 | 0.431 | 2.170 | 0.431 | 3.314 |
| average | 9.166 | 20.058 | 9.166 | 7.413 | 9.166 | 10.517 |
| stdev | 61.004 | 41.398 | 61.004 | 10.032 | 61.004 | 11.651 |
| p (t-test) |  | 0.572 |  | 0.925 |  | 0.954 |
| min | 0.000 | 0.436 | 0.000 | 0.436 | 0.000 | 0.673 |
| max | 480.918 | 141.270 | 480.918 | 27.778 | 480.918 | 27.778 |
| n (Samp) | 62 | 11 | 62 | 11 | 62 | 7 |
| n (Pat) | 62 | 11 | 62 | 11 | 62 | 7 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.553 | 11.172 | 0.553 | 11.172 | 0.553 | 11.552 |
| average | 7.757 | 32.110 | 7.757 | 32.110 | 7.757 | 12.929 |
| stdev | 48.692 | 54.742 | 48.692 | 54.742 | 48.692 | 13.498 |
| p (t-test) |  | 0.239 |  | 0.239 |  | 0.833 |
| min | 0.000 | 0.436 | 0.000 | 0.436 | 0.000 | 0.835 |
| max | 480.918 | 141.270 | 480.918 | 141.270 | 480.918 | 27.778 |
| n (Samp) | 106 | 6 | 106 | 6 | 106 | 4 |
| n (Pat) | 106 | 6 | 106 | 6 | 106 | 4 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.481 | 3.906 | 0.481 | 3.314 | 0.481 | 3.314 |
| average | 11.094 | 27.169 | 11.094 | 7.297 | 11.094 | 9.001 |
| stdev | 67.911 | 51.026 | 67.911 | 8.790 | 67.911 | 10.141 |
| p (t-test) |  | 0.550 |  | 0.884 |  | 0.946 |
| min | 0.000 | 0.673 | 0.000 | 0.673 | 0.000 | 0.673 |
| max | 480.918 | 141.270 | 480.918 | 20.934 | 480.918 | 20.934 |
| n (Samp) | 50 | 7 | 50 | 7 | 50 | 5 |
| n (Pat) | 50 | 7 | 50 | 7 | 50 | 5 |

sCr or UO

| Time prior AKI stage | AUC | SE | nCohort 1 | nCohort 2 | p |
|---|---|---|---|---|---|
| 0 hours | 0.82 | 0.081 | 62 | 11 | 0.000 |
| 24 hours | 0.81 | 0.082 | 62 | 11 | 0.000 |
| 48 hours | 0.81 | 0.101 | 62 | 7 | 0.002 |

sCr only

| Time prior AKI stage | AUC | SE | nCohort 1 | nCohort 2 | p |
|---|---|---|---|---|---|
| 0 hours | 0.80 | 0.111 | 106 | 6 | 0.007 |
| 24 hours | 0.80 | 0.111 | 106 | 6 | 0.007 |
| 48 hours | 0.85 | 0.123 | 106 | 4 | 0.005 |

UO only

| Time prior AKI stage | AUC | SE | nCohort 1 | nCohort 2 | p |
|---|---|---|---|---|---|
| 0 hours | 0.84 | 0.096 | 50 | 7 | 0.000 |
| 24 hours | 0.83 | 0.098 | 50 | 7 | 0.001 |
| 48 hours | 0.80 | 0.122 | 50 | 5 | 0.012 |

**FIG. 4 - 4**

**Epidermal growth factor**

sCr or UO

|  | 0 hr prior to AKI stage | | 24 hr prior to AKI stage | | 48 hr prior to AKI stage | |
| --- | --- | --- | --- | --- | --- | --- |
|  | Cohort 1 | Cohort 2 | Cohort 1 | Cohort 2 | Cohort 1 | Cohort 2 |
| median | 86.700 | 62.300 | 86.700 | 58.500 | 86.700 | 69.700 |
| average | 140.511 | 94.066 | 140.511 | 91.869 | 140.511 | 102.827 |
| stdev | 162.049 | 96.009 | 162.049 | 118.520 | 162.049 | 120.782 |
| p (t-test) |  | 0.040 |  | 0.028 |  | 0.250 |
| min | 0.074 | 0.074 | 0.074 | 0.074 | 0.074 | 6.500 |
| max | 1330.000 | 477.000 | 1330.000 | 847.000 | 1330.000 | 577.000 |
| n (Samp) | 255 | 56 | 255 | 61 | 255 | 26 |
| n (Pat) | 111 | 56 | 111 | 61 | 111 | 26 |

sCr only

|  | 0 hr prior toAKI stage | | 24 hr prior toAKI stage | | 48 hr prior toAKI stage | |
| --- | --- | --- | --- | --- | --- | --- |
|  | Cohort 1 | Cohort 2 | Cohort 1 | Cohort 2 | Cohort 1 | Cohort 2 |
| median | 75.700 | 68.200 | 75.700 | 68.050 | 75.700 | 78.850 |
| average | 128.800 | 111.272 | 128.800 | 91.880 | 128.800 | 96.193 |
| stdev | 152.832 | 138.412 | 152.832 | 72.955 | 152.832 | 55.422 |
| p (t-test) |  | 0.590 |  | 0.222 |  | 0.426 |
| min | 0.074 | 0.074 | 0.074 | 0.074 | 0.074 | 34.700 |
| max | 1330.000 | 601.000 | 1330.000 | 239.000 | 1330.000 | 219.000 |
| n (Samp) | 457 | 23 | 457 | 26 | 457 | 14 |
| n (Pat) | 179 | 23 | 179 | 26 | 179 | 14 |

UO only

|  | 0 hr prior toAKI stage | | 24 hr prior toAKI stage | | 48 hr prior toAKI stage | |
| --- | --- | --- | --- | --- | --- | --- |
|  | Cohort 1 | Cohort 2 | Cohort 1 | Cohort 2 | Cohort 1 | Cohort 2 |
| median | 88.100 | 61.300 | 88.100 | 67.800 | 88.100 | 55.100 |
| average | 138.633 | 85.987 | 138.633 | 107.000 | 138.633 | 100.617 |
| stdev | 161.635 | 86.129 | 161.635 | 139.082 | 161.635 | 129.099 |
| p (t-test) |  | 0.025 |  | 0.192 |  | 0.277 |
| min | 7.440 | 0.074 | 7.440 | 0.074 | 7.440 | 6.500 |
| max | 1330.000 | 477.000 | 1330.000 | 847.000 | 1330.000 | 577.000 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | nCohort 1 | nCohort 2 | p |
| --- | --- | --- | --- | --- | --- |
| 0 hours | 0.40 | 0.040 | 255 | 56 | 0.013 |
| 24 hours | 0.39 | 0.038 | 255 | 61 | 0.003 |
| 48 hours | 0.42 | 0.056 | 255 | 26 | 0.133 |

sCr only

| Time prior AKI stage | AUC | SE | nCohort 1 | nCohort 2 | p |
| --- | --- | --- | --- | --- | --- |
| 0 hours | 0.47 | 0.060 | 457 | 23 | 0.568 |
| 24 hours | 0.46 | 0.057 | 457 | 26 | 0.509 |
| 48 hours | 0.53 | 0.080 | 457 | 14 | 0.742 |

UO only

| Time prior AKI stage | AUC | SE | nCohort 1 | nCohort 2 | p |
| --- | --- | --- | --- | --- | --- |
| 0 hours | 0.38 | 0.041 | 213 | 51 | 0.003 |
| 24 hours | 0.40 | 0.042 | 213 | 53 | 0.020 |
| 48 hours | 0.38 | 0.057 | 213 | 23 | 0.030 |

sCr or UO

| Time prior AKI stage | Cutoff value | sens | spec | Quartile | OR | 95% CI of OR | |
| --- | --- | --- | --- | --- | --- | --- | --- |
| 0 hours | 33.8 | 71% | 20% | 1 |  |  |  |
|  | 27.1 | 80% | 14% | 2 | 1.8 | 1.2 | 2.7 |
|  | 12 | 91% | 5% | 3 | 1.5 | 1.0 | 2.4 |
|  | 157 | 21% | 70% | 4 | 2.5 | 1.7 | 3.7 |
|  | 216 | 9% | 80% |  |  |  |  |
|  | 298 | 4% | 90% |  |  |  |  |
| 24 hours | 36.7 | 70% | 22% | 1 |  |  |  |
|  | 25.2 | 80% | 12% | 2 | 1.5 | 1.0 | 2.3 |
|  | 17 | 90% | 10% | 3 | 2.3 | 1.6 | 3.4 |
|  | 157 | 18% | 70% | 4 | 2.8 | 1.9 | 4.1 |

## FIG. 5 - 1

| | 216 | 8% | 80% | | | | |
| --- | --- | --- | --- | --- | --- | --- | --- |
| | 298 | 2% | 90% | | | | |
| 48 hours | 34.6 | 73% | 20% | 1 | | | |
| | 32.2 | 81% | 18% | 2 | 2.5 | 0.9 | 6.8 |
| | 18.7 | 92% | 11% | 3 | 2.1 | 0.8 | 6.0 |
| | 157 | 12% | 70% | 4 | 3.8 | 1.5 | 9.4 |
| | 216 | 12% | 80% | | | | |
| | 298 | 8% | 90% | | | | |

sCr only

| Time prior AKI stage | Cutoff value | sens | spec | Quartile | OR | 95% CI of OR | |
| --- | --- | --- | --- | --- | --- | --- | --- |
| 0 hours | 36.7 | 74% | 26% | 1 | | | |
| | 31.2 | 83% | 21% | 2 | 2.8 | 1.1 | 7.1 |
| | 27.2 | 91% | 17% | 3 | 2.1 | 0.7 | 5.7 |
| | 141 | 17% | 70% | 4 | 2.1 | 0.7 | 5.7 |
| | 201 | 13% | 80% | | | | |
| | 295 | 9% | 90% | | | | |
| 24 hours | 38.1 | 73% | 27% | 1 | | | |
| | 37.2 | 81% | 26% | 2 | 1.2 | 0.6 | 2.2 |
| | 18.4 | 92% | 12% | 3 | 1.4 | 0.7 | 2.5 |
| | 141 | 27% | 70% | 4 | 0.8 | 0.4 | 1.8 |
| | 201 | 12% | 80% | | | | |
| | 295 | 0% | 90% | | | | |
| 48 hours | 67.9 | 71% | 46% | 1 | | | |
| | 48 | 86% | 34% | 2 | 5.1 | 0.5 | 55.8 |
| | 38.5 | 93% | 27% | 3 | 6.2 | 0.6 | 63.3 |
| | 141 | 21% | 70% | 4 | 2.0 | 0.1 | 39.1 |
| | 201 | 7% | 80% | | | | |
| | 295 | 0% | 90% | | | | |

UO only

| Time prior AKI stage | Cutoff value | sens | spec | Quartile | OR | 95% CI of OR | |
| --- | --- | --- | --- | --- | --- | --- | --- |
| 0 hours | 33.8 | 71% | 17% | 1 | | | |
| | 20.5 | 80% | 10% | 2 | 2.1 | 1.3 | 3.4 |
| | 12.7 | 90% | 4% | 3 | 1.9 | 1.1 | 3.1 |
| | 154 | 18% | 70% | 4 | 3.4 | 2.2 | 5.4 |
| | 202 | 8% | 80% | | | | |
| | 295 | 2% | 90% | | | | |
| 24 hours | 37.6 | 72% | 20% | 1 | | | |
| | 25.2 | 81% | 11% | 2 | 1.1 | 0.7 | 1.8 |
| | 17.3 | 91% | 8% | 3 | 1.5 | 1.0 | 2.3 |
| | 154 | 21% | 70% | 4 | 2.1 | 1.5 | 3.1 |
| | 202 | 13% | 80% | | | | |
| | 295 | 4% | 90% | | | | |
| 48 hours | 32.2 | 74% | 16% | 1 | | | |
| | 30.2 | 83% | 15% | 2 | 1.4 | 0.4 | 4.6 |
| | 18.7 | 91% | 9% | 3 | 2.1 | 0.7 | 6.1 |
| | 154 | 22% | 70% | 4 | 3.8 | 1.5 | 9.6 |
| | 202 | 13% | 80% | | | | |
| | 295 | 9% | 90% | | | | |

Interleukin-1 alpha

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 0.002 | 0.000 | 0.002 | 0.000 | 0.002 | 0.000 |
| average | 0.003 | 0.002 | 0.003 | 0.002 | 0.003 | 0.001 |
| stdev | 0.004 | 0.003 | 0.004 | 0.003 | 0.004 | 0.002 |
| p (t-test) | | 0.005 | | 0.142 | | 0.009 |
| min | 0.000 | 0.000 | 0.000 | 0.000 | 0.000 | 0.000 |
| max | 0.020 | 0.010 | 0.020 | 0.009 | 0.020 | 0.007 |
| n (Samp) | 255 | 56 | 255 | 61 | 255 | 26 |
| n (Pat) | 111 | 56 | 111 | 61 | 111 | 26 |

# FIG. 5 - 2

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 0.001 | 0.000 | 0.001 | 0.001 | 0.001 | 0.000 |
| average | 0.003 | 0.002 | 0.003 | 0.002 | 0.003 | 0.002 |
| stdev | 0.003 | 0.003 | 0.003 | 0.003 | 0.003 | 0.002 |
| p (t-test) | | 0.086 | | 0.520 | | 0.220 |
| min | 0.000 | 0.000 | 0.000 | 0.000 | 0.000 | 0.000 |
| max | 0.020 | 0.011 | 0.020 | 0.009 | 0.020 | 0.007 |
| n (Samp) | 457 | 23 | 457 | 26 | 457 | 14 |
| n (Pat) | 179 | 23 | 179 | 26 | 179 | 14 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 0.003 | 0.000 | 0.003 | 0.000 | 0.003 | 0.000 |
| average | 0.003 | 0.002 | 0.003 | 0.002 | 0.003 | 0.001 |
| stdev | 0.004 | 0.003 | 0.004 | 0.003 | 0.004 | 0.002 |
| p (t-test) | | 0.022 | | 0.161 | | 0.007 |
| min | 0.000 | 0.000 | 0.000 | 0.000 | 0.000 | 0.000 |
| max | 0.020 | 0.009 | 0.020 | 0.009 | 0.020 | 0.006 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | nCohort 1 | nCohort 2 | p |
|---|---|---|---|---|---|
| 0 hours | 0.39 | 0.040 | 255 | 56 | 0.007 |
| 24 hours | 0.46 | 0.040 | 255 | 61 | 0.293 |
| 48 hours | 0.35 | 0.052 | 255 | 26 | 0.005 |

sCr only

| Time prior AKI stage | AUC | SE | nCohort 1 | nCohort 2 | p |
|---|---|---|---|---|---|
| 0 hours | 0.37 | 0.054 | 457 | 23 | 0.020 |
| 24 hours | 0.48 | 0.057 | 457 | 26 | 0.674 |
| 48 hours | 0.42 | 0.073 | 457 | 14 | 0.293 |

UO only

| Time prior AKI stage | AUC | SE | nCohort 1 | nCohort 2 | p |
|---|---|---|---|---|---|
| 0 hours | 0.41 | 0.043 | 213 | 51 | 0.034 |
| 24 hours | 0.46 | 0.043 | 213 | 53 | 0.311 |
| 48 hours | 0.33 | 0.053 | 213 | 23 | 0.001 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.4 | 0.9 | 2.2 |
| | 0 | 100% | 0% | 3 | 2.8 | 1.9 | 4.1 |
| | 0.0045 | 16% | 70% | 4 | 1.7 | 1.1 | 2.6 |
| | 0.00581 | 9% | 80% | | | | |
| | 0.00749 | 4% | 90% | | | | |
| 24 hours | 0 | 100% | 0% | 1 | | | |
| | 0 | 100% | 0% | 2 | 1.6 | 1.1 | 2.3 |
| | 0 | 100% | 0% | 3 | 2.4 | 1.7 | 3.3 |
| | 0.0045 | 25% | 70% | 4 | 1.1 | 0.7 | 1.7 |
| | 0.00581 | 15% | 80% | | | | |
| | 0.00749 | 5% | 90% | | | | |
| 48 hours | 0 | 100% | 0% | 1 | | | |
| | 0 | 100% | 0% | 2 | 2.7 | 0.6 | 11.1 |
| | 0 | 100% | 0% | 3 | 12.9 | 4.1 | 40.6 |
| | 0.0045 | 8% | 70% | 4 | 0.0 | 0.0 | na |
| | 0.00581 | 8% | 80% | | | | |
| | 0.00749 | 0% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |

## FIG. 5 - 3

70

|  | Cutoff value | sens | spec | Quartile | OR | 95% CI of OR | |
|---|---|---|---|---|---|---|---|
|  | , 0 | 100% | 0% | 2 | 0.7 | 0.2 | 2.4 |
|  | 0 | 100% | 0% | 3 | 4.5 | 2.3 | 8.5 |
|  | 0.00432 | 17% | 70% | 4 | 0.0 | 0.0 | na |
|  | 0.00552 | 13% | 80% |  |  |  |  |
|  | 0.00689 | 9% | 90% |  |  |  |  |
| 24 hours | 0 | 100% | 0% | 1 |  |  |  |
|  | 0 | 100% | 0% | 2 | 1.6 | 0.8 | 3.2 |
|  | 0 | 100% | 0% | 3 | 2.8 | 1.6 | 5.0 |
|  | 0.00432 | 27% | 70% | 4 | 0.0 | 0.0 | na |
|  | 0.00552 | 12% | 80% |  |  |  |  |
|  | 0.00689 | 8% | 90% |  |  |  |  |
| 48 hours | 0 | 100% | 0% | 1 |  |  |  |
|  | 0 | 100% | 0% | 2 | 5.2 | 0.5 | 56.3 |
|  | 0 | 100% | 0% | 3 | 8.5 | 0.9 | 79.9 |
|  | 0.00432 | 7% | 70% | 4 | 0.0 | 0.0 | na |
|  | 0.00552 | 7% | 80% |  |  |  |  |
|  | 0.00689 | 0% | 90% |  |  |  |  |

UO only

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 1.1 | 0.7 | 1.8 |
|  | 0 | 100% | 0% | 3 | 1.4 | 0.9 | 2.2 |
|  | 0.00463 | 18% | 71% | 4 | 2.8 | 1.9 | 4.1 |
|  | 0.00565 | 12% | 80% |  |  |  |  |
|  | 0.00716 | 4% | 90% |  |  |  |  |
| 24 hours | 0 | 100% | 0% | 1 |  |  |  |
|  | 0 | 100% | 0% | 2 | 0.9 | 0.6 | 1.4 |
|  | 0 | 100% | 0% | 3 | 3.7 | 2.7 | 5.1 |
|  | 0.00463 | 26% | 71% | 4 | 0.0 | 0.0 | na |
|  | 0.00565 | 17% | 80% |  |  |  |  |
|  | 0.00716 | 4% | 90% |  |  |  |  |
| 48 hours | 0 | 100% | 0% | 1 |  |  |  |
|  | 0 | 100% | 0% | 2 | 1.0 | 0.1 | 7.6 |
|  | 0 | 100% | 0% | 3 | 13.5 | 4.2 | 43.5 |
|  | 0.00463 | 9% | 71% | 4 | 0.0 | 0.0 | na |
|  | 0.00565 | 9% | 80% |  |  |  |  |
|  | 0.00716 | 0% | 90% |  |  |  |  |

**Interleukin-4**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 46.700 | 34.500 | 46.700 | 40.700 | 46.700 | 46.900 |
| average | 49.356 | 32.492 | 49.356 | 41.603 | 49.356 | 42.916 |
| stdev | 36.161 | 26.650 | 36.161 | 25.553 | 36.161 | 21.763 |
| p (t-test) |  | 0.001 |  | 0.115 |  | 0.374 |
| min | 1.040 | 1.040 | 1.040 | 1.040 | 1.040 | 1.040 |
| max | 370.000 | 106.000 | 370.000 | 93.500 | 370.000 | 83.800 |
| n (Samp) | 255 | 56 | 255 | 61 | 255 | 26 |
| n (Pat) | 111 | 56 | 111 | 61 | 111 | 26 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 46.900 | 38.000 | 46.900 | 38.250 | 46.900 | 33.900 |
| average | 48.883 | 39.743 | 48.883 | 40.913 | 48.883 | 33.516 |
| stdev | 39.541 | 34.308 | 39.541 | 25.682 | 39.541 | 21.309 |
| p (t-test) |  | 0.277 |  | 0.311 |  | 0.149 |
| min | 1.040 | 1.040 | 1.040 | 1.040 | 1.040 | 1.040 |
| max | 451.000 | 140.000 | 451.000 | 93.500 | 451.000 | 69.100 |
| n (Samp) | 457 | 23 | 457 | 26 | 457 | 14 |
| n (Pat) | 179 | 23 | 179 | 26 | 179 | 14 |

UO only

| 0 hr prior toAKI stage | 24 hr prior toAKI stage | 48 hr prior toAKI stage |
|---|---|---|

## FIG. 5 - 4

| | Cohort 1 | Cohort 2 | Cohort 1 | Cohort 2 | Cohort 1 | Cohort 2 |
|---|---|---|---|---|---|---|
| median | 45.900 | 33.000 | 45.900 | 49.400 | 45.900 | 48.100 |
| average | 47.955 | 33.498 | 47.955 | 42.768 | 47.955 | 47.997 |
| stdev | 38.165 | 26.840 | 38.165 | 28.493 | 38.165 | 17.540 |
| p (t-test) | | 0.011 | | 0.355 | | 0.996 |
| min | 1.040 | 1.040 | 1.040 | 1.040 | 1.040 | 1.040 |
| max | 370.000 | 106.000 | 370.000 | 109.000 | 370.000 | 83.800 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | nCohort 1 | nCohort 2 | p |
|---|---|---|---|---|---|
| 0 hours | 0.35 | 0.038 | 255 | 56 | 0.000 |
| 24 hours | 0.44 | 0.040 | 255 | 61 | 0.122 |
| 48 hours | 0.45 | 0.058 | 255 | 26 | 0.416 |

sCr only

| Time prior AKI stage | AUC | SE | nCohort 1 | nCohort 2 | p |
|---|---|---|---|---|---|
| 0 hours | 0.42 | 0.058 | 457 | 23 | 0.174 |
| 24 hours | 0.44 | 0.056 | 457 | 26 | 0.291 |
| 48 hours | 0.37 | 0.068 | 457 | 14 | 0.048 |

UO only

| Time prior AKI stage | AUC | SE | nCohort 1 | nCohort 2 | p |
|---|---|---|---|---|---|
| 0 hours | 0.38 | 0.041 | 213 | 51 | 0.003 |
| 24 hours | 0.47 | 0.044 | 213 | 53 | 0.501 |
| 48 hours | 0.53 | 0.064 | 213 | 23 | 0.659 |

sCr or UO

| Time prior AKI stage | Cutoff value | sens | spec | Quartile | OR | 95% CI of OR | |
|---|---|---|---|---|---|---|---|
| 0 hours | 10.9 | 71% | 15% | 1 | | | |
| | 0 | 100% | 0% | 2 | 3.8 | 2.1 | 6.7 |
| | 0 | 100% | 0% | 3 | 2.9 | 1.6 | 5.3 |
| | 64.2 | 9% | 70% | 4 | 5.8 | 3.4 | 10.1 |
| | 73.3 | 4% | 80% | | | | |
| | 86.2 | 4% | 91% | | | | |
| 24 hours | 31 | 72% | 26% | 1 | | | |
| | 10.9 | 80% | 15% | 2 | 2.3 | 1.6 | 3.3 |
| | 0 | 100% | 0% | 3 | 1.6 | 1.1 | 2.4 |
| | 64.2 | 20% | 70% | 4 | 1.8 | 1.2 | 2.6 |
| | 73.3 | 7% | 80% | | | | |
| | 86.2 | 2% | 91% | | | | |
| 48 hours | 32.5 | 77% | 29% | 1 | | | |
| | 31 | 81% | 26% | 2 | 2.8 | 1.3 | 5.9 |
| | 0 | 100% | 0% | 3 | 1.9 | 0.8 | 4.3 |
| | 64.2 | 15% | 70% | 4 | 1.3 | 0.5 | 3.3 |
| | 73.3 | 8% | 80% | | | | |
| | 86.2 | 0% | 91% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.8 | 0.3 | 2.0 |
| | 0 | 100% | 0% | 3 | 1.4 | 0.7 | 2.9 |
| | 63.2 | 22% | 70% | 4 | 1.4 | 0.7 | 2.9 |
| | 70.5 | 13% | 80% | | | | |
| | 86.2 | 4% | 90% | | | | |
| 24 hours | 31 | 73% | 28% | 1 | | | |
| | 22.7 | 81% | 23% | 2 | 0.7 | 0.3 | 1.5 |
| | 0 | 100% | 0% | 3 | 1.7 | 1.0 | 3.0 |
| | 63.2 | 23% | 70% | 4 | 1.0 | 0.5 | 2.0 |
| | 70.5 | 12% | 80% | | | | |
| | 86.2 | 4% | 90% | | | | |
| 48 hours | 31 | 71% | 28% | 1 | | | |
| | 0 | 100% | 0% | 2 | 3.1 | 0.2 | 43.1 |

**FIG. 5 - 5**

| | | | | | | | |
|---|---|---|---|---|---|---|---|
| | 0 | 100% | 0% | 3 | 6.3 | 0.6 | 63.8 |
| | 63.2 | 7% | 70% | 4 | 4.1 | 0.3 | 49.7 |
| | 70.5 | 0% | 80% | | | | |
| | 86.2 | 0% | 90% | | | | |

UO only

| Time prior AKI stage | Cutoff value | sens | spec | Quartile | OR | 95% CI of OR | |
|---|---|---|---|---|---|---|---|
| 0 hours | 13.4 | 71% | 21% | 1 | | | |
| | 0 | 100% | 0% | 2 | 3.3 | 1.8 | 6.0 |
| | 0 | 100% | 0% | 3 | 3.0 | 1.6 | 5.5 |
| | 63 | 16% | 70% | 4 | 4.9 | 2.8 | 8.7 |
| | 72.2 | 8% | 80% | | | | |
| | 84.3 | 4% | 90% | | | | |
| 24 hours | 28.2 | 72% | 27% | 1 | | | |
| | 9.1 | 81% | 17% | 2 | 1.8 | 1.2 | 2.6 |
| | 0 | 100% | 0% | 3 | 1.0 | 0.7 | 1.5 |
| | 63 | 26% | 70% | 4 | 1.4 | 0.9 | 2.0 |
| | 72.2 | 15% | 80% | | | | |
| | 84.3 | 6% | 90% | | | | |
| 48 hours | 45.9 | 74% | 52% | 1 | | | |
| | 33 | 83% | 30% | 2 | 2.6 | 0.6 | 11.2 |
| | 31 | 91% | 28% | 3 | 8.1 | 2.4 | 26.9 |
| | 63 | 17% | 70% | 4 | 1.5 | 0.3 | 8.4 |
| | 72.2 | 9% | 80% | | | | |
| | 84.3 | 0% | 90% | | | | |

**Netrin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 4.105 | 1.386 | 4.105 | 16.012 | 4.105 | 1.494 |
| stdev | 9.254 | 2.678 | 9.254 | 61.390 | 9.254 | 2.798 |
| p (t-test) | | 0.225 | | 0.161 | | 0.434 |
| min | 0.211 | 0.211 | 0.211 | 0.211 | 0.211 | 0.211 |
| max | 54.172 | 8.915 | 54.172 | 315.934 | 54.172 | 8.107 |
| n (Samp) | 55 | 18 | 55 | 26 | 55 | 8 |
| n (Pat) | 55 | 18 | 55 | 26 | 55 | 8 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 3.401 | 0.211 | 158.072 |
| average | 3.089 | 0.524 | 3.089 | 5.353 | 3.089 | 158.072 |
| stdev | 7.132 | 0.628 | 7.132 | 5.751 | 7.132 | 223.250 |
| p (t-test) | | 0.345 | | 0.447 | | 0.000 |
| min | 0.211 | 0.211 | 0.211 | 0.211 | 0.211 | 0.211 |
| max | 54.172 | 1.877 | 54.172 | 13.635 | 54.172 | 315.934 |
| n (Samp) | 107 | 7 | 107 | 6 | 107 | 2 |
| n (Pat) | 92 | 7 | 92 | 6 | 92 | 2 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 2.840 | 1.670 | 2.840 | 18.101 | 2.840 | 1.351 |
| stdev | 5.898 | 3.110 | 5.898 | 65.215 | 5.898 | 2.652 |
| p (t-test) | | 0.495 | | 0.106 | | 0.463 |
| min | 0.211 | 0.211 | 0.211 | 0.211 | 0.211 | 0.211 |
| max | 32.376 | 8.915 | 32.376 | 315.934 | 32.376 | 8.107 |
| n (Samp) | 49 | 13 | 49 | 23 | 49 | 9 |
| n (Pat) | 47 | 13 | 47 | 23 | 47 | 9 |

sCr or UO

| Time prior AKI stage | AUC | SE | nCohort 1 | nCohort 2 | p |
|---|---|---|---|---|---|
| 0 hours | 0.42 | 0.075 | 55 | 18 | 0.278 |
| 24 hours | 0.55 | 0.070 | 55 | 26 | 0.478 |

## FIG. 5 - 6

sCr only

| 48 hours | 0.42 | 0.104 | 55 | 8 | 0.417 |

| Time prior AKI stage | AUC | SE | nCohort 1 | nCohort 2 | p |
|---|---|---|---|---|---|
| 0 hours | 0.43 | 0.107 | 107 | 7 | 0.521 |
| 24 hours | 0.67 | 0.124 | 107 | 6 | 0.159 |
| 48 hours | 0.66 | 0.213 | 107 | 2 | 0.443 |

UO only

| Time prior AKI stage | AUC | SE | nCohort 1 | nCohort 2 | p |
|---|---|---|---|---|---|
| 0 hours | 0.45 | 0.088 | 49 | 13 | 0.539 |
| 24 hours | 0.58 | 0.074 | 49 | 23 | 0.301 |
| 48 hours | 0.44 | 0.101 | 49 | 9 | 0.532 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.5 | 0.4 | 6.2 |
| | 0 | 100% | 0% | 3 | 1.5 | 0.4 | 6.2 |
| | 1.8768769 | 17% | 71% | 4 | 3.4 | 1.0 | 11.7 |
| | 4.4221698 | 11% | 80% | | | | |
| | 11.792453 | 0% | 91% | | | | |
| 24 hours | 0 | 100% | 0% | 1 | | | |
| | 0 | 100% | 0% | 2 | 0.8 | 0.3 | 2.0 |
| | 0 | 100% | 0% | 3 | 0.5 | 0.2 | 1.3 |
| | 1.8768769 | 46% | 71% | 4 | 1.4 | 0.6 | 3.1 |
| | 4.4221698 | 31% | 80% | | | | |
| | 11.792453 | 19% | 91% | | | | |
| 48 hours | 0 | 100% | 0% | 1 | | | |
| | 0 | 100% | 0% | 2 | 3.5 | 0.2 | 62.6 |
| | 0 | 100% | 0% | 3 | 3.5 | 0.2 | 62.6 |
| | 1.8768769 | 25% | 71% | 4 | 1.1 | 0.0 | 70.8 |
| | 4.4221698 | 13% | 80% | | | | |
| | 11.792453 | 0% | 91% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.3 | 0.0 | 5.6 |
| | 0 | 100% | 0% | 3 | 1.4 | 0.3 | 6.2 |
| | 0.7370283 | 23% | 71% | 4 | 2.2 | 0.5 | 8.7 |
| | 4.4221698 | 15% | 82% | | | | |
| | 11.261261 | 0% | 92% | | | | |
| 24 hours | 0 | 100% | 0% | 1 | | | |
| | 0 | 100% | 0% | 2 | 0.8 | 0.3 | 2.0 |
| | 0 | 100% | 0% | 3 | 0.2 | 0.0 | 0.9 |
| | 0.7370283 | 43% | 71% | 4 | 1.3 | 0.5 | 3.1 |
| | 4.4221698 | 35% | 82% | | | | |
| | 11.261261 | 22% | 92% | | | | |
| 48 hours | 0 | 100% | 0% | 1 | | | |
| | 0 | 100% | 0% | 2 | 0.0 | 0.0 | na |
| | 0 | 100% | 0% | 3 | 1.6 | 0.2 | 11.4 |
| | 0.7370283 | 22% | 71% | 4 | 2.6 | 0.4 | 16.0 |
| | 4.4221698 | 11% | 82% | | | | |
| | 11.261261 | 0% | 92% | | | | |

**Osteopontin**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 82732.956 | 148298.000 | 82732.956 | 126476.357 | 82732.956 | na |
| average | 97936.896 | 146269.169 | 97936.896 | 114809.657 | 97936.896 | na |
| stdev | 47673.593 | 40964.688 | 47673.593 | 51308.820 | 47673.593 | na |

## FIG. 5 - 7

| | | | | | |
|---|---|---|---|---|---|
| p (t-test) | | 0.015 | | 0.277 | na |
| min | 48003.207 | 86632.026 | 48003.207 | 41626.547 | 48003.207 | na |
| max | 198386.623 | 199267.052 | 198386.623 | 199459.230 | 198386.623 | na |
| n (Samp) | 26 | 8 | 26 | 17 | 26 | 0 |
| n (Pat) | 25 | 8 | 25 | 17 | 25 | 0 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 88676.926 | 152264.239 | 88676.926 | 126476.357 | 88676.926 | 41626.547 |
| average | 105077.742 | 152264.239 | 105077.742 | 118147.752 | 105077.742 | 105459.492 |
| stdev | 48274.555 | 45283.240 | 48274.555 | 50526.597 | 48274.555 | na |
| p (t-test) | | 0.182 | | 0.463 | | na |
| min | 41626.547 | 120244.154 | 41626.547 | 42968.358 | 41626.547 | 105459.492 |
| max | 199267.052 | 184284.325 | 199267.052 | 199459.230 | 199267.052 | 105459.492 |
| n (Samp) | 47 | 2 | 47 | 9 | 47 | 1 |
| n (Pat) | 44 | 2 | 44 | 9 | 44 | 1 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 102075.984 | 136473.394 | 102075.984 | 132828.668 | 102075.984 | 48003.207 |
| average | 108201.322 | 140838.433 | 108201.322 | 118728.451 | 108201.322 | 218222.247 |
| stdev | 52403.038 | 41018.568 | 52403.038 | 48865.063 | 52403.038 | na |
| p (t-test) | | 0.137 | | 0.571 | | na |
| min | 48003.207 | 86632.026 | 48003.207 | 41626.547 | 48003.207 | 218222.247 |
| max | 199459.230 | 199267.052 | 199459.230 | 180359.177 | 199459.230 | 218222.247 |
| n (Samp) | 27 | 7 | 27 | 11 | 27 | 1 |
| n (Pat) | 25 | 7 | 25 | 11 | 25 | 1 |

sCr or UO

| Time prior AKI stage | AUC | SE | nCohort 1 | nCohort 2 | p |
|---|---|---|---|---|---|
| 0 hours | 0.80 | 0.101 | 26 | 8 | 0.003 |
| 24 hours | 0.59 | 0.090 | 26 | 17 | 0.304 |
| 48 hours | nd | nd | 26 | 0 | nd |

sCr only

| Time prior AKI stage | AUC | SE | nCohort 1 | nCohort 2 | p |
|---|---|---|---|---|---|
| 0 hours | 0.82 | 0.186 | 47 | 2 | 0.086 |
| 24 hours | 0.57 | 0.108 | 47 | 9 | 0.504 |
| 48 hours | 0.62 | 0.305 | 47 | 1 | 0.702 |

UO only

| Time prior AKI stage | AUC | SE | nCohort 1 | nCohort 2 | p |
|---|---|---|---|---|---|
| 0 hours | 0.70 | 0.121 | 27 | 7 | 0.100 |
| 24 hours | 0.55 | 0.105 | 27 | 11 | 0.620 |
| 48 hours | 1.00 | 0.000 | 27 | 1 | nd |

sCr or UO

| Time prior AKI stage | Cutoff value | sens | spec | Quartile | OR | 95% CI of OR | |
|---|---|---|---|---|---|---|---|
| 0 hours | 113148.86 | 75% | 77% | 1 | | | |
| | 103188.14 | 88% | 62% | 2 | na | na | na |
| | 83136.946 | 100% | 54% | 3 | na | na | na |
| | 110437.36 | 75% | 73% | 4 | na | na | na |
| | 129350.4 | 63% | 81% | | | | |
| | 180841.68 | 25% | 92% | | | | |
| 24 hours | 83136.946 | 71% | 54% | 1 | | | |
| | 55731.945 | 82% | 27% | 2 | 1.3 | 0.2 | 7.3 |
| | 41626.547 | 94% | 0% | 3 | 1.3 | 0.2 | 7.3 |
| | 110437.36 | 53% | 73% | 4 | 2.8 | 0.5 | 14.6 |
| | 129350.4 | 47% | 81% | | | | |
| | 180841.68 | 6% | 92% | | | | |
| 48 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |

## FIG. 5 - 8

sCr only

| Time prior AKI stage | Cutoff value | sens | spec | Quartile | OR | 95% CI of OR | | |
|---|---|---|---|---|---|---|---|---|
| 0 hours | 113148.86 | 100% | 68% | 1 | | | | |
| | 113148.86 | 100% | 68% | 2 | na | na | na | |
| | 113148.86 | 100% | 68% | 3 | na | na | na | |
| | 129350.4 | 50% | 70% | 4 | na | na | na | |
| | 160480.15 | 50% | 81% | | | | | |
| | 180359.18 | 50% | 91% | | | | | |
| 24 hours | 83136.946 | 78% | 45% | 1 | | | | |
| | 49396.377 | 89% | 13% | 2 | 0.5 | 0.0 | 12.0 | |
| | 41626.547 | 100% | 2% | 3 | 2.4 | 0.4 | 15.0 | |
| | 129350.4 | 44% | 70% | 4 | 1.0 | 0.1 | 9.8 | |
| | 160480.15 | 11% | 81% | | | | | |
| | 180359.18 | 11% | 91% | | | | | |
| 48 hours | 104509.04 | 100% | 62% | 1 | | | | |
| | 104509.04 | 100% | 62% | 2 | na | na | na | |
| | 104509.04 | 100% | 62% | 3 | na | na | na | |
| | 129350.4 | 0% | 70% | 4 | na | na | na | |
| | 160480.15 | 0% | 81% | | | | | |
| | 180359.18 | 0% | 91% | | | | | |

UO only

| Time prior AKI stage | Cutoff value | sens | spec | Quartile | OR | 95% CI of OR | | |
|---|---|---|---|---|---|---|---|---|
| 0 hours | 110437.36 | 71% | 67% | 1 | | | | |
| | 103188.14 | 86% | 56% | 2 | na | na | na | |
| | 83136.946 | 100% | 44% | 3 | na | na | na | |
| | 129350.4 | 57% | 70% | 4 | na | na | na | |
| | 178151.08 | 29% | 81% | | | | | |
| | 184284.33 | 14% | 93% | | | | | |
| 24 hours | 83136.946 | 73% | 44% | 1 | | | | |
| | 78055.258 | 82% | 37% | 2 | 1.5 | 0.2 | 13.4 | |
| | 55731.945 | 91% | 26% | 3 | 1.8 | 0.2 | 16.4 | |
| | 129350.4 | 55% | 70% | 4 | 1.5 | 0.2 | 13.4 | |
| | 178151.08 | 9% | 81% | | | | | |
| | 184284.33 | 0% | 93% | | | | | |
| 48 hours | 199459.23 | 100% | 100% | 1 | | | | |
| | 199459.23 | 100% | 100% | 2 | na | na | na | |
| | 199459.23 | 100% | 100% | 3 | na | na | na | |
| | 129350.4 | 100% | 70% | 4 | na | na | na | |
| | 178151.08 | 100% | 81% | | | | | |
| | 184284.33 | 100% | 93% | | | | | |

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 21.535 | 12.228 | 21.535 | 2.717 | 21.535 | 0.036 |
| average | 33.087 | 14.917 | 33.087 | 2.717 | 33.087 | 0.036 |
| stdev | 33.822 | 13.757 | 33.822 | 1.921 | 33.822 | na |
| p (t-test) | | 0.063 | | 0.213 | | na |
| min | 0.036 | 0.486 | 0.036 | 1.359 | 0.036 | 0.036 |
| max | 166.667 | 52.557 | 166.667 | 4.076 | 166.667 | 0.036 |
| n (Samp) | 57 | 13 | 57 | 2 | 57 | 1 |
| n (Pat) | 37 | 13 | 37 | 2 | 37 | 1 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 20.380 | 9.046 | 20.380 | 3.221 | 20.380 | 11.820 |
| average | 29.375 | 9.629 | 29.375 | 2.969 | 29.375 | 11.820 |
| stdev | 31.017 | 4.325 | 31.017 | 1.218 | 31.017 | 11.449 |
| p (t-test) | | 0.124 | | 0.094 | | 0.428 |
| min | 0.036 | 5.343 | 0.036 | 1.359 | 0.036 | 3.724 |
| max | 166.667 | 16.677 | 166.667 | 4.076 | 166.667 | 19.916 |

# FIG. 5 - 9

| n (Samp) | 89 | 6 | 89 | 4 | 89 | 2 |
| n (Pat) | 61 | 6 | 61 | 4 | 61 | 2 |

UO only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 22.447 | 8.832 | 22.447 | 40.910 | 22.447 | 0.036 |
| average | 37.376 | 13.071 | 37.376 | 40.910 | 37.376 | 0.036 |
| stdev | 36.083 | 14.115 | 36.083 | 52.091 | 36.083 | na |
| p (t-test) | | 0.018 | | 0.894 | | na |
| min | 0.036 | 0.036 | 0.036 | 4.076 | 0.036 | 0.036 |
| max | 166.667 | 52.557 | 166.667 | 77.744 | 166.667 | 0.036 |
| n (Samp) | 42 | 14 | 42 | 2 | 42 | 1 |
| n (Pat) | 27 | 14 | 27 | 2 | 27 | 1 |

sCr or UO

| Time prior AKI stage | AUC | SE | nCohort 1 | nCohort 2 | p |
| --- | --- | --- | --- | --- | --- |
| 0 hours | 0.32 | 0.076 | 57 | 13 | 0.018 |
| 24 hours | 0.09 | 0.065 | 57 | 2 | 0.000 |
| 48 hours | 0.02 | 0.024 | 57 | 1 | 0.000 |

sCr only

| Time prior AKI stage | AUC | SE | nCohort 1 | nCohort 2 | p |
| --- | --- | --- | --- | --- | --- |
| 0 hours | 0.28 | 0.092 | 89 | 6 | 0.016 |
| 24 hours | 0.13 | 0.064 | 89 | 4 | 0.000 |
| 48 hours | 0.32 | 0.167 | 89 | 2 | 0.281 |

UO only

| Time prior AKI stage | AUC | SE | nCohort 1 | nCohort 2 | p |
| --- | --- | --- | --- | --- | --- |
| 0 hours | 0.23 | 0.066 | 42 | 14 | 0.000 |
| 24 hours | 0.44 | 0.202 | 42 | 2 | 0.768 |
| 48 hours | 0.01 | 0.020 | 42 | 1 | 0.000 |

sCr or UO

| Time prior AKI stage | Cutoff value | sens | spec | Quartile | OR | 95% CI of OR | |
| --- | --- | --- | --- | --- | --- | --- | --- |
| 0 hours | 3.7240933 | 77% | 12% | 1 | | | |
| | 2.7173913 | 85% | 12% | 2 | 3.6 | 0.2 | 64.2 |
| | 1.3586957 | 92% | 7% | 3 | 6.5 | 0.5 | 89.6 |
| | 38.352273 | 8% | 70% | 4 | 5.2 | 0.3 | 79.1 |
| | 53.977273 | 0% | 82% | | | | |
| | 82.417582 | 0% | 91% | | | | |
| 24 hours | 0.0360285 | 100% | 4% | 1 | | | |
| | 0.0360285 | 100% | 4% | 2 | na | na | na |
| | 0.0360285 | 100% | 4% | 3 | na | na | na |
| | 38.352273 | 0% | 70% | 4 | na | na | na |
| | 53.977273 | 0% | 82% | | | | |
| | 82.417582 | 0% | 91% | | | | |
| 48 hours | 0 | 100% | 0% | 1 | | | |
| | 0 | 100% | 0% | 2 | na | na | na |
| | 0 | 100% | 0% | 3 | na | na | na |
| | 38.352273 | 0% | 70% | 4 | na | na | na |
| | 53.977273 | 0% | 82% | | | | |
| | 82.417582 | 0% | 91% | | | | |

UO only

| Time prior AKI stage | Cutoff value | sens | spec | Quartile | OR | 95% CI of OR | |
| --- | --- | --- | --- | --- | --- | --- | --- |
| 0 hours | 3.7240933 | 71% | 5% | 1 | | | |
| | 1.3586957 | 86% | 5% | 2 | 3.5 | 0.2 | 67.2 |
| | 0.0360285 | 93% | 2% | 3 | 3.5 | 0.2 | 67.2 |
| | 42.613636 | 7% | 71% | 4 | 13.0 | 0.9 | 187.9 |
| | 53.977273 | 0% | 81% | | | | |
| | 99.085366 | 0% | 93% | | | | |
| 24 hours | 1.3586957 | 100% | 5% | 1 | | | |
| | 1.3586957 | 100% | 5% | 2 | 0.0 | 0.0 | na |
| | 1.3586957 | 100% | 5% | 3 | 0.0 | 0.0 | na |
| | 42.613636 | 50% | 71% | 4 | 1.0 | 0.0 | 74.6 |
| | 53.977273 | 50% | 81% | | | | |

**FIG. 5 - 10**

| | 99.085366 | 0% | 93% | | | | |
|---|---|---|---|---|---|---|---|
| 48 hours | 0 | 100% | 0% | 1 | | | |
| | 0 | 100% | 0% | 2 | na | na | na |
| | 0 | 100% | 0% | 3 | na | na | na |
| | 42.613636 | 0% | 71% | 4 | na | na | na |
| | 53.977273 | 0% | 81% | | | | |
| | 99.085366 | 0% | 93% | | | | |

# FIG. 5 - 11

**Bone morphogenetic protein 7**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.163 | 0.163 | 0.163 | 1.508 | 0.163 | 0.163 |
| average | 2.814 | 1.063 | 2.814 | 3.505 | 2.814 | 0.163 |
| stdev | 8.264 | 2.113 | 8.264 | 4.983 | 8.264 | 0.000 |
| p (t-test) |  | 0.530 |  | 0.869 |  | 0.653 |
| min | 0.163 | 0.163 | 0.163 | 0.163 | 0.163 | 0.163 |
| max | 64.356 | 6.616 | 64.356 | 10.839 | 64.356 | 0.163 |
| n (Samp) | 80 | 9 | 80 | 4 | 80 | 2 |
| n (Pat) | 58 | 9 | 58 | 4 | 58 | 2 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.623 | 4.505 | 0.623 | 5.731 | 0.623 | 8.316 |
| average | 2.564 | 4.505 | 2.564 | 5.731 | 2.564 | 8.316 |
| stdev | 7.521 | 2.986 | 7.521 | 7.224 | 7.521 | 11.531 |
| p (t-test) |  | 0.717 |  | 0.557 |  | 0.290 |
| min | 0.163 | 2.393 | 0.163 | 0.623 | 0.163 | 0.163 |
| max | 64.356 | 6.616 | 64.356 | 10.839 | 64.356 | 16.470 |
| n (Samp) | 98 | 2 | 98 | 2 | 98 | 2 |
| n (Pat) | 70 | 2 | 70 | 2 | 70 | 2 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.623 | 0.163 | 0.623 | 1.278 | 0.623 | 0.163 |
| average | 3.333 | 1.063 | 3.333 | 1.278 | 3.333 | 0.163 |
| stdev | 9.312 | 2.113 | 9.312 | 1.577 | 9.312 | na |
| p (t-test) |  | 0.471 |  | 0.758 |  | na |
| min | 0.163 | 0.163 | 0.163 | 0.163 | 0.163 | 0.163 |
| max | 64.356 | 6.616 | 64.356 | 2.393 | 64.356 | 0.163 |
| n (Samp) | 62 | 9 | 62 | 2 | 62 | 1 |
| n (Pat) | 45 | 9 | 45 | 2 | 45 | 1 |

sCr or UO

| Time prior AKI stage | AUC | SE | nCohort 1 | nCohort 2 | p |
|---|---|---|---|---|---|
| 0 hours | 0.41 | 0.095 | 80 | 9 | 0.355 |
| 24 hours | 0.63 | 0.154 | 80 | 4 | 0.416 |
| 48 hours | 0.26 | 0.145 | 80 | 2 | 0.092 |

sCr only

| Time prior AKI stage | AUC | SE | nCohort 1 | nCohort 2 | p |
|---|---|---|---|---|---|
| 0 hours | 0.85 | 0.172 | 98 | 2 | 0.040 |
| 24 hours | 0.74 | 0.204 | 98 | 2 | 0.234 |
| 48 hours | 0.61 | 0.215 | 98 | 2 | 0.618 |

UO only

| Time prior AKI stage | AUC | SE | nCohort 1 | nCohort 2 | p |
|---|---|---|---|---|---|
| 0 hours | 0.39 | 0.095 | 62 | 9 | 0.246 |
| 24 hours | 0.49 | 0.208 | 62 | 2 | 0.969 |
| 48 hours | 0.23 | 0.191 | 62 | 1 | 0.162 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 2.2 | 0.1 | 50.2 |
|  | 0 | 100% | 0% | 3 | 4.9 | 0.3 | 69.1 |
|  | 1.3354701 | 11% | 70% | 4 | 2.2 | 0.1 | 50.2 |
|  | 2.7599715 | 11% | 80% |  |  |  |  |
|  | 5.2083333 | 11% | 93% |  |  |  |  |
| 24 hours | 0.1629274 | 75% | 51% | 1 |  |  |  |
|  | 0 | 100% | 0% | 2 | na | na | na |
|  | 0 | 100% | 0% | 3 | na | na | na |

# FIG. 6 - 1

| | Cutoff value | sens | spec | Quartile | OR | 95% CI of OR | |
|---|---|---|---|---|---|---|---|
| | 1.3354701 | 50% | 70% | 4 | na | na | na |
| | 2.7599715 | 25% | 80% | | | | |
| | 5.2083333 | 25% | 93% | | | | |
| 48 hours | 0 | 100% | 0% | 1 | | | |
| | 0 | 100% | 0% | 2 | na | na | na |
| | 0 | 100% | 0% | 3 | na | na | na |
| | 1.3354701 | 0% | 70% | 4 | na | na | na |
| | 2.7599715 | 0% | 80% | | | | |
| | 5.2083333 | 0% | 93% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 2.1 | 0.1 | 51.0 |
| | 0 | 100% | 0% | 3 | 1.0 | 0.0 | 63.5 |
| | 2.0477208 | 11% | 73% | 4 | 7.1 | 0.5 | 98.3 |
| | 3.8006757 | 11% | 82% | | | | |
| | 5.2083333 | 11% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 2.0477208 | 50% | 73% | 4 | na | na | na |
| | 3.8006757 | 0% | 82% | | | | |
| | 5.2083333 | 0% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 2.0477208 | 0% | 73% | 4 | na | na | na |
| | 3.8006757 | 0% | 82% | | | | |
| | 5.2083333 | 0% | 90% | | | | |

**Complement C3**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.804 | 0.821 | 0.804 | 0.788 | 0.804 | 0.848 |
| average | 0.819 | 0.812 | 0.819 | 0.785 | 0.819 | 0.858 |
| stdev | 0.255 | 0.252 | 0.255 | 0.271 | 0.255 | 0.310 |
| p (t-test) | | 0.889 | | 0.445 | | 0.527 |
| min | 0.186 | 0.402 | 0.186 | 0.269 | 0.186 | 0.290 |
| max | 1.720 | 1.580 | 1.720 | 1.470 | 1.720 | 1.580 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.798 | 0.606 | 0.798 | 0.850 | 0.798 | 0.845 |
| average | 0.810 | 0.638 | 0.810 | 0.825 | 0.810 | 0.860 |
| stdev | 0.262 | 0.116 | 0.262 | 0.273 | 0.262 | 0.131 |
| p (t-test) | | 0.110 | | 0.860 | | 0.616 |
| min | 0.186 | 0.538 | 0.186 | 0.466 | 0.186 | 0.633 |
| max | 1.730 | 0.864 | 1.730 | 1.220 | 1.730 | 1.030 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.794 | 0.867 | 0.794 | 0.768 | 0.794 | 0.845 |
| average | 0.803 | 0.832 | 0.803 | 0.766 | 0.803 | 0.831 |
| stdev | 0.241 | 0.249 | 0.241 | 0.251 | 0.241 | 0.334 |
| p (t-test) | | 0.542 | | 0.414 | | 0.659 |
| min | 0.216 | 0.402 | 0.216 | 0.269 | 0.216 | 0.290 |
| max | 1.680 | 1.580 | 1.680 | 1.470 | 1.680 | 1.580 |
| n (Samp) | 356 | 27 | 356 | 32 | 356 | 16 |
| n (Pat) | 138 | 27 | 138 | 32 | 138 | 16 |

sCr or UO

# FIG. 6 - 2

| Time prior AKI stage | AUC | SE | nCohort 1 | nCohort 2 | p |
|---|---|---|---|---|---|
| 0 hours | 0.49 | 0.056 | 434 | 28 | 0.832 |
| 24 hours | 0.47 | 0.049 | 434 | 36 | 0.480 |
| 48 hours | 0.54 | 0.071 | 434 | 18 | 0.615 |

sCr only

| Time prior AKI stage | AUC | SE | nCohort 1 | nCohort 2 | p |
|---|---|---|---|---|---|
| 0 hours | 0.27 | 0.085 | 542 | 6 | 0.007 |
| 24 hours | 0.52 | 0.093 | 542 | 10 | 0.842 |
| 48 hours | 0.59 | 0.114 | 542 | 7 | 0.416 |

UO only

| Time prior AKI stage | AUC | SE | nCohort 1 | nCohort 2 | p |
|---|---|---|---|---|---|
| 0 hours | 0.54 | 0.059 | 356 | 27 | 0.515 |
| 24 hours | 0.46 | 0.052 | 356 | 32 | 0.432 |
| 48 hours | 0.51 | 0.074 | 356 | 16 | 0.845 |

sCr or UO

| Time prior AKI stage | Cutoff value | sens | spec | Quartile | OR | 95% CI of OR | |
|---|---|---|---|---|---|---|---|
| 0 hours | 0.652 | 71% | 25% | 1 | | | |
| | 0.602 | 82% | 19% | 2 | 3.0 | 1.5 | 6.0 |
| | 0.518 | 93% | 11% | 3 | 1.5 | 0.7 | 3.6 |
| | 0.931 | 32% | 70% | 4 | 1.8 | 0.8 | 4.1 |
| | 1.03 | 11% | 82% | | | | |
| | 1.16 | 7% | 90% | | | | |
| 24 hours | 0.637 | 72% | 23% | 1 | | | |
| | 0.508 | 81% | 10% | 2 | 1.1 | 0.7 | 1.9 |
| | 0.453 | 92% | 7% | 3 | 1.0 | 0.6 | 1.7 |
| | 0.931 | 28% | 70% | 4 | 1.4 | 0.9 | 2.3 |
| | 1.03 | 17% | 82% | | | | |
| | 1.16 | 8% | 90% | | | | |
| 48 hours | 0.717 | 72% | 35% | 1 | | | |
| | 0.632 | 83% | 23% | 2 | 0.7 | 0.2 | 2.4 |
| | 0.446 | 94% | 6% | 3 | 1.8 | 0.8 | 4.0 |
| | 0.931 | 39% | 70% | 4 | 1.0 | 0.4 | 2.8 |
| | 1.03 | 11% | 82% | | | | |
| | 1.16 | 11% | 90% | | | | |

sCr only

| Time prior AKI stage | Cutoff value | sens | spec | Quartile | OR | 95% CI of OR | |
|---|---|---|---|---|---|---|---|
| 0 hours | 0.568 | 83% | 18% | 1 | | | |
| | 0.568 | 83% | 18% | 2 | na | na | na |
| | 0.536 | 100% | 15% | 3 | na | na | na |
| | 0.931 | 0% | 70% | 4 | na | na | na |
| | 1.01 | 0% | 80% | | | | |
| | 1.15 | 0% | 91% | | | | |
| 24 hours | 0.65 | 70% | 27% | 1 | | | |
| | 0.568 | 80% | 18% | 2 | 0.3 | 0.0 | 4.6 |
| | 0.491 | 90% | 11% | 3 | 1.0 | 0.3 | 3.8 |
| | 0.931 | 30% | 70% | 4 | 1.0 | 0.3 | 3.8 |
| | 1.01 | 30% | 80% | | | | |
| | 1.15 | 10% | 91% | | | | |
| 48 hours | 0.831 | 71% | 57% | 1 | | | |
| | 0.799 | 86% | 51% | 2 | 0.0 | 0.0 | na |
| | 0.632 | 100% | 25% | 3 | 4.1 | 0.3 | 48.8 |
| | 0.931 | 29% | 70% | 4 | 2.0 | 0.1 | 38.9 |
| | 1.01 | 14% | 80% | | | | |
| | 1.15 | 0% | 91% | | | | |

UO only

| Time prior AKI stage | Cutoff value | sens | spec | Quartile | OR | 95% CI of OR | |
|---|---|---|---|---|---|---|---|
| 0 hours | 0.684 | 70% | 33% | 1 | | | |
| | 0.65 | 81% | 26% | 2 | 1.0 | 0.4 | 2.3 |

**FIG. 6 - 3**

| | | | | | | | |
|---|---|---|---|---|---|---|---|
| | 0.518 | 93% | 11% | 3 | 2.3 | 1.3 | 4.3 |
| | 0.913 | 33% | 70% | 4 | 1.2 | 0.6 | 2.6 |
| | 1.01 | 11% | 81% | | | | |
| | 1.13 | 7% | 91% | | | | |
| 24 hours | 0.678 | 72% | 32% | 1 | | | |
| | 0.531 | 81% | 12% | 2 | 1.4 | 0.7 | 2.5 |
| | 0.453 | 91% | 6% | 3 | 1.6 | 0.9 | 2.8 |
| | 0.913 | 25% | 70% | 4 | 1.6 | 0.9 | 2.8 |
| | 1.01 | 16% | 81% | | | | |
| | 1.13 | 6% | 91% | | | | |
| 48 hours | 0.704 | 75% | 35% | 1 | | | |
| | 0.544 | 81% | 13% | 2 | 0.7 | 0.2 | 2.4 |
| | 0.435 | 94% | 6% | 3 | 1.3 | 0.5 | 3.2 |
| | 0.913 | 38% | 70% | 4 | 1.0 | 0.4 | 2.8 |
| | 1.01 | 13% | 81% | | | | |
| | 1.13 | 13% | 91% | | | | |

**Epidermal growth factor**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 79.000 | 67.350 | 79.000 | 59.650 | 79.000 | 48.700 |
| average | 130.455 | 142.071 | 130.455 | 71.301 | 130.455 | 89.184 |
| stdev | 152.140 | 170.033 | 152.140 | 58.897 | 152.140 | 87.376 |
| p (t-test) | | 0.698 | | 0.021 | | 0.254 |
| min | 0.074 | 12.700 | 0.074 | 8.880 | 0.074 | 4.320 |
| max | 1330.000 | 604.000 | 1330.000 | 227.000 | 1330.000 | 327.000 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 74.500 | 98.900 | 74.500 | 51.350 | 74.500 | 99.900 |
| average | 124.554 | 187.462 | 124.554 | 75.975 | 124.554 | 98.303 |
| stdev | 145.722 | 230.488 | 145.722 | 80.525 | 145.722 | 62.748 |
| p (t-test) | | 0.297 | | 0.294 | | 0.634 |
| min | 0.074 | 0.074 | 0.074 | 0.074 | 0.074 | 4.320 |
| max | 1330.000 | 601.000 | 1330.000 | 227.000 | 1330.000 | 188.000 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 79.000 | 59.200 | 79.000 | 63.050 | 79.000 | 48.700 |
| average | 129.447 | 117.570 | 129.447 | 85.658 | 129.447 | 116.750 |
| stdev | 155.109 | 142.993 | 155.109 | 95.616 | 155.109 | 154.509 |
| p (t-test) | | 0.700 | | 0.117 | | 0.749 |
| min | 0.074 | 10.900 | 0.074 | 9.550 | 0.074 | 6.500 |
| max | 1330.000 | 604.000 | 1330.000 | 513.000 | 1330.000 | 601.000 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.48 | 0.056 | 434 | 28 | 0.727 |
| 24 hours | 0.38 | 0.045 | 434 | 36 | 0.008 |
| 48 hours | 0.41 | 0.064 | 434 | 18 | 0.157 |

sCr only

| Time prior AKI stage | AUC | SE | nCohort 1 | nCohort 2 | p |
|---|---|---|---|---|---|
| 0 hours | 0.52 | 0.120 | 542 | 6 | 0.841 |
| 24 hours | 0.38 | 0.082 | 542 | 10 | 0.145 |
| 48 hours | 0.52 | 0.111 | 542 | 7 | 0.859 |

UO only

| Time prior | AUC | SE | nCohort 1 | nCohort 2 | p |
|---|---|---|---|---|---|

# FIG. 6 - 4

| AKI stage | | | | | |
|---|---|---|---|---|---|
| 0 hours | 0.45 | 0.056 | 356 | 27 | 0.358 |
| 24 hours | 0.41 | 0.049 | 356 | 32 | 0.058 |
| 48 hours | 0.43 | 0.070 | 356 | 16 | 0.308 |

sCr or UO

| Time prior AKI stage | Cutoff value | sens | spec | Quartile | OR | 95% CI of OR | |
|---|---|---|---|---|---|---|---|
| 0 hours | 32.2 | 71% | 21% | 1 | | | |
| | 27.1 | 82% | 16% | 2 | 0.4 | 0.2 | 0.9 |
| | 20.5 | 93% | 14% | 3 | 0.5 | 0.3 | 1.0 |
| | 149 | 32% | 70% | 4 | 1.1 | 0.7 | 1.8 |
| | 205 | 25% | 80% | | | | |
| | 295 | 14% | 90% | | | | |
| 24 hours | 32 | 72% | 21% | 1 | | | |
| | 30.7 | 81% | 19% | 2 | 2.1 | 0.8 | 5.7 |
| | 15.9 | 92% | 10% | 3 | 5.6 | 2.5 | 12.7 |
| | 149 | 17% | 70% | 4 | 4.4 | 1.9 | 10.3 |
| | 205 | 8% | 80% | | | | |
| | 295 | 0% | 90% | | | | |
| 48 hours | 22.4 | 72% | 14% | 1 | | | |
| | 19.4 | 83% | 13% | 2 | 1.0 | 0.4 | 2.8 |
| | 5.37 | 94% | 2% | 3 | 0.7 | 0.2 | 2.4 |
| | 149 | 28% | 70% | 4 | 1.8 | 0.8 | 4.0 |
| | 205 | 6% | 80% | | | | |
| | 295 | 6% | 90% | | | | |

sCr only

| Time prior AKI stage | Cutoff value | sens | spec | Quartile | OR | 95% CI of OR | |
|---|---|---|---|---|---|---|---|
| 0 hours | 22.4 | 83% | 14% | 1 | | | |
| | 22.4 | 83% | 14% | 2 | 0.5 | 0.0 | 9.7 |
| | 0 | 100% | 0% | 3 | 0.5 | 0.0 | 9.7 |
| | 137 | 50% | 70% | 4 | 1.0 | 0.1 | 7.3 |
| | 189 | 33% | 80% | | | | |
| | 275 | 33% | 90% | | | | |
| 24 hours | 32.7 | 70% | 23% | 1 | | | |
| | 15.9 | 80% | 9% | 2 | 1.0 | 0.1 | 7.3 |
| | 8.64 | 90% | 3% | 3 | 1.0 | 0.1 | 7.3 |
| | 137 | 20% | 70% | 4 | 2.0 | 0.5 | 9.1 |
| | 189 | 20% | 80% | | | | |
| | 275 | 0% | 90% | | | | |
| 48 hours | 92.1 | 71% | 59% | 1 | | | |
| | 38.1 | 86% | 27% | 2 | 1.0 | 0.0 | 51.9 |
| | 0.0735 | 100% | 1% | 3 | 4.1 | 0.3 | 48.8 |
| | 137 | 29% | 70% | 4 | 1.0 | 0.0 | 51.5 |
| | 189 | 0% | 80% | | | | |
| | 275 | 0% | 90% | | | | |

UO only

| Time prior AKI stage | Cutoff value | sens | spec | Quartile | OR | 95% CI of OR | |
|---|---|---|---|---|---|---|---|
| 0 hours | 32.2 | 70% | 20% | 1 | | | |
| | 27.1 | 81% | 16% | 2 | 0.7 | 0.3 | 1.4 |
| | 17.5 | 93% | 11% | 3 | 0.7 | 0.3 | 1.4 |
| | 143 | 26% | 70% | 4 | 1.5 | 0.9 | 2.5 |
| | 188 | 19% | 80% | | | | |
| | 284 | 7% | 90% | | | | |
| 24 hours | 32 | 72% | 19% | 1 | | | |
| | 30.2 | 81% | 18% | 2 | 0.8 | 0.3 | 2.0 |
| | 22.4 | 91% | 14% | 3 | 2.8 | 1.6 | 5.1 |
| | 143 | 19% | 70% | 4 | 2.1 | 1.1 | 4.0 |
| | 188 | 9% | 80% | | | | |
| | 284 | 3% | 90% | | | | |
| 48 hours | 22.4 | 75% | 14% | 1 | | | |
| | 20.5 | 81% | 14% | 2 | 0.7 | 0.2 | 2.4 |
| | 14.1 | 94% | 7% | 3 | 0.7 | 0.2 | 2.4 |

## FIG. 6 - 5

| | 143 | 25% | 70% | 4 | 1.5 | 0.6 | 3.6 |
|---|---|---|---|---|---|---|---|
| | 188 | 13% | 80% | | | | |
| | 284 | 13% | 90% | | | | |

**Interleukin-1 alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.002 | 0.000 | 0.002 | 0.000 | 0.002 | 0.000 |
| average | 0.003 | 0.002 | 0.003 | 0.002 | 0.003 | 0.001 |
| stdev | 0.003 | 0.003 | 0.003 | 0.002 | 0.003 | 0.003 |
| p (t-test) | | 0.100 | | 0.202 | | 0.101 |
| min | 0.000 | 0.000 | 0.000 | 0.000 | 0.000 | 0.000 |
| max | 0.020 | 0.009 | 0.020 | 0.007 | 0.020 | 0.012 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.001 | 0.000 | 0.001 | 0.000 | 0.001 | 0.000 |
| average | 0.003 | 0.000 | 0.003 | 0.001 | 0.003 | 0.000 |
| stdev | 0.003 | 0.000 | 0.003 | 0.002 | 0.003 | 0.000 |
| p (t-test) | | 0.049 | | 0.105 | | 0.034 |
| min | 0.000 | 0.000 | 0.000 | 0.000 | 0.000 | 0.000 |
| max | 0.020 | 0.000 | 0.020 | 0.005 | 0.020 | 0.000 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.002 | 0.000 | 0.002 | 0.001 | 0.002 | 0.000 |
| average | 0.003 | 0.002 | 0.003 | 0.002 | 0.003 | 0.002 |
| stdev | 0.003 | 0.003 | 0.003 | 0.003 | 0.003 | 0.003 |
| p (t-test) | | 0.195 | | 0.421 | | 0.183 |
| min | 0.000 | 0.000 | 0.000 | 0.000 | 0.000 | 0.000 |
| max | 0.020 | 0.009 | 0.020 | 0.007 | 0.020 | 0.012 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.42 | 0.053 | 434 | 28 | 0.116 |
| 24 hours | 0.45 | 0.049 | 434 | 36 | 0.340 |
| 48 hours | 0.37 | 0.061 | 434 | 18 | 0.038 |

sCr only

| Time prior AKI stage | AUC | SE | nCohort 1 | nCohort 2 | p |
|---|---|---|---|---|---|
| 0 hours | 0.25 | 0.080 | 542 | 6 | 0.002 |
| 24 hours | 0.36 | 0.079 | 542 | 10 | 0.075 |
| 48 hours | 0.25 | 0.074 | 542 | 7 | 0.001 |

UO only

| Time prior AKI stage | AUC | SE | nCohort 1 | nCohort 2 | p |
|---|---|---|---|---|---|
| 0 hours | 0.44 | 0.055 | 356 | 27 | 0.251 |
| 24 hours | 0.47 | 0.052 | 356 | 32 | 0.610 |
| 48 hours | 0.39 | 0.066 | 356 | 16 | 0.090 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.2 | 0.6 | 2.6 |
| | 0 | 100% | 0% | 3 | 3.8 | 2.2 | 6.6 |
| | 0.00446 | 18% | 70% | 4 | 0.0 | 0.0 | na |
| | 0.00577 | 7% | 80% | | | | |
| | 0.00716 | 4% | 90% | | | | |

## FIG. 6 - 6

| Time prior AKI stage | Cutoff value | sens | spec | Quartile | OR | 95% CI of OR | |
|---|---|---|---|---|---|---|---|
| 24 hours | 0 | 100% | 0% | 1 | | | |
| | 0 | 100% | 0% | 2 | 2.6 | 1.4 | 4.7 |
| | 0 | 100% | 0% | 3 | 4.3 | 2.5 | 7.4 |
| | 0.00446 | 22% | 70% | 4 | 0.0 | 0.0 | na |
| | 0.00577 | 8% | 80% | | | | |
| | 0.00716 | 0% | 90% | | | | |
| 48 hours | 0 | 100% | 0% | 1 | | | |
| | 0 | 100% | 0% | 2 | 1.5 | 0.3 | 8.0 |
| | 0 | 100% | 0% | 3 | 7.2 | 2.2 | 23.2 |
| | 0.00446 | 11% | 70% | 4 | 0.0 | 0.0 | na |
| | 0.00577 | 11% | 80% | | | | |
| | 0.00716 | 6% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | na | na | na |
| | 0 | 100% | 0% | 3 | na | na | na |
| | 0.00436 | 0% | 70% | 4 | na | na | na |
| | 0.00558 | 0% | 80% | | | | |
| | 0.00705 | 0% | 90% | | | | |
| 24 hours | 0 | 100% | 0% | 1 | | | |
| | 0 | 100% | 0% | 2 | 2.0 | 0.1 | 39.2 |
| | 0 | 100% | 0% | 3 | 0.0 | 0.0 | na |
| | 0.00436 | 10% | 70% | 4 | 7.3 | 0.8 | 70.8 |
| | 0.00558 | 0% | 80% | | | | |
| | 0.00705 | 0% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 0.00436 | 0% | 70% | 4 | na | na | na |
| | 0.00558 | 0% | 80% | | | | |
| | 0.00705 | 0% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.2 | 0.6 | 2.6 |
| | 0 | 100% | 0% | 3 | 1.4 | 0.7 | 2.9 |
| | 0.00446 | 19% | 71% | 4 | 1.9 | 1.0 | 3.7 |
| | 0.00552 | 11% | 80% | | | | |
| | 0.0068 | 4% | 90% | | | | |
| 24 hours | 0 | 100% | 0% | 1 | | | |
| | 0 | 100% | 0% | 2 | 1.7 | 1.0 | 3.1 |
| | 0 | 100% | 0% | 3 | 0.8 | 0.4 | 1.8 |
| | 0.00446 | 25% | 71% | 4 | 1.9 | 1.1 | 3.4 |
| | 0.00552 | 16% | 80% | | | | |
| | 0.0068 | 6% | 90% | | | | |
| 48 hours | 0 | 100% | 0% | 1 | | | |
| | 0 | 100% | 0% | 2 | 1.0 | 0.1 | 7.4 |
| | 0 | 100% | 0% | 3 | 6.7 | 2.1 | 22.1 |
| | 0.00446 | 13% | 71% | 4 | 0.0 | 0.0 | na |
| | 0.00552 | 13% | 80% | | | | |
| | 0.0068 | 6% | 90% | | | | |

**Interleukin-4**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 46.000 | 34.500 | 46.000 | 43.300 | 46.000 | 36.050 |
| average | 48.456 | 37.966 | 48.456 | 37.711 | 48.456 | 38.428 |
| stdev | 40.230 | 26.209 | 40.230 | 25.558 | 40.230 | 25.454 |
| p (t-test) | | 0.174 | | 0.116 | | 0.295 |

# FIG. 6 - 7

| | | | | | | |
|---|---|---|---|---|---|---|
| min | 1.040 | 1.040 | 1.040 | 1.040 | 1.040 | 1.040 |
| max | 451.000 | 108.000 | 451.000 | 72.800 | 451.000 | 96.000 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 45.900 | 37.250 | 45.900 | 34.200 | 45.900 | 28.100 |
| average | 47.210 | 37.097 | 47.210 | 25.741 | 47.210 | 31.234 |
| stdev | 38.771 | 34.954 | 38.771 | 22.139 | 38.771 | 19.648 |
| p (t-test) | | 0.525 | | 0.082 | | 0.277 |
| min | 1.040 | 1.040 | 1.040 | 1.040 | 1.040 | 1.040 |
| max | 451.000 | 78.900 | 451.000 | 67.400 | 451.000 | 58.400 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 45.950 | 33.000 | 45.950 | 48.750 | 45.950 | 48.100 |
| average | 48.810 | 36.227 | 48.810 | 39.293 | 48.810 | 47.429 |
| stdev | 42.530 | 25.100 | 42.530 | 25.932 | 42.530 | 26.122 |
| p (t-test) | | 0.130 | | 0.214 | | 0.898 |
| min | 1.040 | 1.040 | 1.040 | 1.040 | 1.040 | 1.040 |
| max | 451.000 | 108.000 | 451.000 | 72.800 | 451.000 | 96.000 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.41 | 0.052 | 434 | 28 | 0.096 |
| 24 hours | 0.43 | 0.048 | 434 | 36 | 0.141 |
| 48 hours | 0.42 | 0.065 | 434 | 18 | 0.213 |

sCr only

| Time prior AKI stage | AUC | SE | nCohort 1 | nCohort 2 | p |
|---|---|---|---|---|---|
| 0 hours | 0.44 | 0.113 | 542 | 6 | 0.615 |
| 24 hours | 0.30 | 0.071 | 542 | 10 | 0.004 |
| 48 hours | 0.36 | 0.094 | 542 | 7 | 0.125 |

UO only

| Time prior AKI stage | AUC | SE | nCohort 1 | nCohort 2 | p |
|---|---|---|---|---|---|
| 0 hours | 0.40 | 0.053 | 356 | 27 | 0.056 |
| 24 hours | 0.45 | 0.052 | 356 | 32 | 0.375 |
| 48 hours | 0.52 | 0.075 | 356 | 16 | 0.790 |

sCr or UO

| Time prior AKI stage | Cutoff value | sens | spec | Quartile | OR | 95% CI of OR | |
|---|---|---|---|---|---|---|---|
| 0 hours | 17.9 | 71% | 23% | 1 | | | |
| | 10.9 | 86% | 17% | 2 | 2.8 | 1.1 | 7.2 |
| | 0 | 100% | 0% | 3 | 2.8 | 1.1 | 7.1 |
| | 63.7 | 14% | 71% | 4 | 3.2 | 1.3 | 7.9 |
| | 72.2 | 7% | 81% | | | | |
| | 86.2 | 4% | 91% | | | | |
| 24 hours | 13.4 | 72% | 20% | 1 | | | |
| | 0 | 100% | 0% | 2 | 1.9 | 1.1 | 3.3 |
| | 0 | 100% | 0% | 3 | 1.4 | 0.7 | 2.5 |
| | 63.7 | 19% | 71% | 4 | 1.9 | 1.1 | 3.3 |
| | 72.2 | 6% | 81% | | | | |
| | 86.2 | 0% | 91% | | | | |
| 48 hours | 22.7 | 72% | 24% | 1 | | | |
| | 13.4 | 83% | 20% | 2 | 3.1 | 0.8 | 11.9 |
| | 0 | 100% | 0% | 3 | 2.0 | 0.5 | 9.2 |
| | 63.7 | 11% | 71% | 4 | 3.1 | 0.8 | 11.9 |
| | 72.2 | 11% | 81% | | | | |
| | 86.2 | 6% | 91% | | | | |

sCr only

# FIG. 6 - 8

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.5 | 0.0 | 9.7 |
| | 0 | 100% | 0% | 3 | 0.0 | 0.0 | na |
| | 62 | 33% | 71% | 4 | 1.5 | 0.3 | 8.0 |
| | 69.8 | 17% | 80% | | | | |
| | 85 | 0% | 90% | | | | |
| 24 hours | 1.04 | 70% | 16% | 1 | | | |
| | 0 | 100% | 0% | 2 | 0.0 | 0.0 | na |
| | 0 | 100% | 0% | 3 | 5.2 | 0.5 | 55.7 |
| | 62 | 10% | 71% | 4 | 4.1 | 0.3 | 48.8 |
| | 69.8 | 0% | 80% | | | | |
| | 85 | 0% | 90% | | | | |
| 48 hours | 22.7 | 71% | 25% | 1 | | | |
| | 20 | 86% | 24% | 2 | na | na | na |
| | 0 | 100% | 0% | 3 | na | na | na |
| | 62 | 0% | 71% | 4 | na | na | na |
| | 69.8 | 0% | 80% | | | | |
| | 85 | 0% | 90% | | | | |

UO only

| Time prior AKI stage | Cutoff value | sens | spec | Quartile | OR | 95% CI of OR | |
|---|---|---|---|---|---|---|---|
| 0 hours | 17.9 | 70% | 23% | 1 | | | |
| | 10.9 | 85% | 18% | 2 | 9.8 | 1.1 | 90.6 |
| | 0 | 100% | 0% | 3 | 8.6 | 0.9 | 81.8 |
| | 63 | 15% | 71% | 4 | 9.9 | 1.1 | 91.6 |
| | 72.8 | 4% | 81% | | | | |
| | 86.2 | 4% | 90% | | | | |
| 24 hours | 18.8 | 72% | 23% | 1 | | | |
| | 0 | 100% | 0% | 2 | 1.9 | 1.1 | 3.4 |
| | 0 | 100% | 0% | 3 | 0.8 | 0.4 | 1.8 |
| | 63 | 22% | 71% | 4 | 1.7 | 1.0 | 3.1 |
| | 72.8 | 0% | 81% | | | | |
| | 86.2 | 0% | 90% | | | | |
| 48 hours | 32.5 | 75% | 31% | 1 | | | |
| | 25.8 | 81% | 26% | 2 | 1.0 | 0.3 | 3.9 |
| | 9.1 | 94% | 18% | 3 | 2.4 | 0.9 | 6.5 |
| | 63 | 31% | 71% | 4 | 1.0 | 0.3 | 3.9 |
| | 72.8 | 19% | 81% | | | | |
| | 86.2 | 6% | 90% | | | | |

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 19.916 | 6.962 | 19.916 | 6.329 | 19.916 | 5.938 |
| average | 30.020 | 13.519 | 30.020 | 8.888 | 30.020 | 5.938 |
| stdev | 32.147 | 16.183 | 32.147 | 8.942 | 32.147 | 3.131 |
| p (t-test) | | 0.134 | | 0.195 | | 0.295 |
| min | 0.036 | 0.036 | 0.036 | 1.359 | 0.036 | 3.724 |
| max | 166.667 | 52.557 | 166.667 | 21.535 | 166.667 | 8.152 |
| n (Samp) | 82 | 9 | 82 | 4 | 82 | 2 |
| n (Pat) | 59 | 9 | 59 | 4 | 59 | 2 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 18.297 | 11.010 | 18.297 | 4.970 | 18.297 | 3.724 |
| average | 28.305 | 11.010 | 28.305 | 4.970 | 28.305 | 3.724 |
| stdev | 30.341 | 8.014 | 30.341 | 5.107 | 30.341 | 0.000 |
| p (t-test) | | 0.424 | | 0.282 | | 0.257 |
| min | 0.036 | 5.343 | 0.036 | 1.359 | 0.036 | 3.724 |
| max | 166.667 | 16.677 | 166.667 | 8.582 | 166.667 | 3.724 |
| n (Samp) | 101 | 2 | 101 | 2 | 101 | 2 |

# FIG. 6 - 9

| n (Pat) | 71 | 2 | 71 | 2 | 71 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 20.380 | 6.962 | 20.380 | 12.806 | 20.380 | 0.036 |
| average | 32.928 | 13.519 | 32.928 | 12.806 | 32.928 | 8.152 |
| stdev | 34.109 | 16.183 | 34.109 | 12.345 | 34.109 | na |
| p (t-test) | | 0.099 | | 0.411 | | na |
| min | 0.036 | 0.036 | 0.036 | 4.076 | 0.036 | 8.152 |
| max | 166.667 | 52.557 | 166.667 | 21.535 | 166.667 | 8.152 |
| n (Samp) | 64 | 9 | 64 | 2 | 64 | 1 |
| n (Pat) | 46 | 9 | 46 | 2 | 46 | 1 |

sCr or UO

| Time prior AKI stage | AUC | SE | nCohort 1 | nCohort 2 | p |
|---|---|---|---|---|---|
| 0 hours | 0.32 | 0.084 | 82 | 9 | 0.031 |
| 24 hours | 0.27 | 0.108 | 82 | 4 | 0.033 |
| 48 hours | 0.20 | 0.123 | 82 | 2 | 0.016 |

sCr only

| Time prior AKI stage | AUC | SE | nCohort 1 | nCohort 2 | p |
|---|---|---|---|---|---|
| 0 hours | 0.33 | 0.169 | 101 | 2 | 0.313 |
| 24 hours | 0.19 | 0.114 | 101 | 2 | 0.006 |
| 48 hours | 0.16 | 0.102 | 101 | 2 | 0.001 |

UO only

| Time prior AKI stage | AUC | SE | nCohort 1 | nCohort 2 | p |
|---|---|---|---|---|---|
| 0 hours | 0.29 | 0.081 | 64 | 9 | 0.008 |
| 24 hours | 0.33 | 0.171 | 64 | 2 | 0.315 |
| 48 hours | 0.21 | 0.176 | 64 | 1 | 0.101 |

sCr or UO

| Time prior AKI stage | Cutoff value | sens | spec | Quartile | OR | 95% CI of OR | |
|---|---|---|---|---|---|---|---|
| 0 hours | 3.7240933 | 78% | 16% | 1 | | | |
| | 2.7173913 | 89% | 16% | 2 | 1.0 | 0.0 | 60.2 |
| | 0 | 100% | 0% | 3 | 3.3 | 0.2 | 54.3 |
| | 32.670455 | 11% | 71% | 4 | 4.9 | 0.3 | 69.1 |
| | 52.556818 | 0% | 80% | | | | |
| | 79.268293 | 0% | 90% | | | | |
| 24 hours | 2.7173913 | 75% | 16% | 1 | | | |
| | 0.4857513 | 100% | 9% | 2 | na | na | na |
| | 0.4857513 | 100% | 9% | 3 | na | na | na |
| | 32.670455 | 0% | 71% | 4 | na | na | na |
| | 52.556818 | 0% | 80% | | | | |
| | 79.268293 | 0% | 90% | | | | |
| 48 hours | 2.7173913 | 100% | 16% | 1 | | | |
| | 2.7173913 | 100% | 16% | 2 | na | na | na |
| | 2.7173913 | 100% | 16% | 3 | na | na | na |
| | 32.670455 | 0% | 71% | 4 | na | na | na |
| | 52.556818 | 0% | 80% | | | | |
| | 79.268293 | 0% | 90% | | | | |

UO only

| Time prior AKI stage | Cutoff value | sens | spec | Quartile | OR | 95% CI of OR | |
|---|---|---|---|---|---|---|---|
| 0 hours | 3.7240933 | 78% | 13% | 1 | | | |
| | 2.1049223 | 89% | 13% | 2 | 1.1 | 0.0 | 66.8 |
| | 0 | 100% | 0% | 3 | 2.3 | 0.1 | 53.6 |
| | 38.602941 | 11% | 70% | 4 | 6.9 | 0.5 | 94.3 |
| | 53.977273 | 0% | 83% | | | | |
| | 82.417582 | 0% | 91% | | | | |
| 24 hours | 2.1049223 | 100% | 13% | 1 | | | |
| | 2.1049223 | 100% | 13% | 2 | na | na | na |
| | 2.1049223 | 100% | 13% | 3 | na | na | na |
| | 38.602941 | 0% | 70% | 4 | na | na | na |
| | 53.977273 | 0% | 83% | | | | |

# FIG. 6 - 10

| | | | | | | | |
|---|---|---|---|---|---|---|---|
| | 82.417582 | 0% | 91% | | | | |
| 48 hours | 6.9624352 | 100% | 20% | 1 | | | |
| | 6.9624352 | 100% | 20% | 2 | na | na | na |
| | 6.9624352 | 100% | 20% | 3 | na | na | na |
| | 38.602941 | 0% | 70% | 4 | na | na | na |
| | 53.977273 | 0% | 83% | | | | |
| | 82.417582 | 0% | 91% | | | | |

**Tubulointerstitial nephritis antigen**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.927 | na | 0.927 | 0.630 | 0.927 | 0.087 |
| average | 47.568 | na | 47.568 | 77.232 | 47.568 | 0.779 |
| stdev | 111.317 | na | 111.317 | 151.335 | 111.317 | na |
| p (t-test) | | na | | 0.438 | | na |
| min | 0.087 | na | 0.087 | 0.111 | 0.087 | 0.779 |
| max | 408.188 | na | 408.188 | 411.095 | 408.188 | 0.779 |
| n (Samp) | 46 | 0 | 46 | 13 | 46 | 1 |
| n (Pat) | 43 | 0 | 43 | 13 | 43 | 1 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.952 | na | 0.952 | 0.457 | 0.952 | na |
| average | 54.223 | na | 54.223 | 2.208 | 54.223 | na |
| stdev | 120.392 | na | 120.392 | 3.314 | 120.392 | na |
| p (t-test) | | na | | 0.461 | | na |
| min | 0.087 | na | 0.087 | 0.136 | 0.087 | na |
| max | 411.095 | na | 411.095 | 6.029 | 411.095 | na |
| n (Samp) | 59 | 0 | 59 | 3 | 59 | 0 |
| n (Pat) | 54 | 0 | 54 | 3 | 54 | 0 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.853 | na | 0.853 | 0.630 | 0.853 | 0.087 |
| average | 42.123 | na | 42.123 | 77.238 | 42.123 | 0.779 |
| stdev | 111.818 | na | 111.818 | 151.332 | 111.818 | na |
| p (t-test) | | na | | 0.373 | | na |
| min | 0.087 | na | 0.087 | 0.111 | 0.087 | 0.779 |
| max | 408.188 | na | 408.188 | 411.095 | 408.188 | 0.779 |
| n (Samp) | 40 | 0 | 40 | 13 | 40 | 1 |
| n (Pat) | 37 | 0 | 37 | 13 | 37 | 1 |

sCr or UO

| Time prior AKI stage | AUC | SE | nCohort 1 | nCohort 2 | p |
|---|---|---|---|---|---|
| 0 hours | nd | nd | 46 | 0 | nd |
| 24 hours | 0.52 | 0.092 | 46 | 13 | 0.813 |
| 48 hours | 0.41 | 0.273 | 46 | 1 | 0.750 |

sCr only

| Time prior AKI stage | AUC | SE | nCohort 1 | nCohort 2 | p |
|---|---|---|---|---|---|
| 0 hours | nd | nd | 59 | 0 | nd |
| 24 hours | 0.34 | 0.145 | 59 | 3 | 0.276 |
| 48 hours | nd | nd | 59 | 0 | nd |

UO only

| Time prior AKI stage | AUC | SE | nCohort 1 | nCohort 2 | p |
|---|---|---|---|---|---|
| 0 hours | nd | nd | 40 | 0 | nd |
| 24 hours | 0.54 | 0.094 | 40 | 13 | 0.638 |
| 48 hours | 0.45 | 0.285 | 40 | 1 | 0.861 |

sCr or UO

| Time prior AKI stage | Cutoff value | sens | spec | Quartile | OR | 95% CI of OR | |
|---|---|---|---|---|---|---|---|
| 0 hours | na | na | na | 1 | | | |
| | na | na | na | 2 | na | na | na |

## FIG. 6 - 11

| | na | na | na | 3 | na | na | na |
|---|---|---|---|---|---|---|---|
| | na | na | na | 4 | na | na | na |
| | na | na | na | | | | |
| | na | na | na | | | | |
| 24 hours | 0.4821005 | 77% | 35% | 1 | | | |
| | 0.4079312 | 85% | 26% | 2 | 3.0 | 0.5 | 16.9 |
| | 0.111254 | 92% | 4% | 3 | 1.5 | 0.2 | 10.6 |
| | 2.8722426 | 38% | 72% | 4 | 1.5 | 0.2 | 10.6 |
| | 25.091241 | 23% | 80% | | | | |
| | 216.32752 | 15% | 91% | | | | |
| 48 hours | 0.7046084 | 100% | 41% | 1 | | | |
| | 0.7046084 | 100% | 41% | 2 | na | na | na |
| | 0.7046084 | 100% | 41% | 3 | na | na | na |
| | 2.8722426 | 0% | 72% | 4 | na | na | na |
| | 25.091241 | 0% | 80% | | | | |
| | 216.32752 | 0% | 91% | | | | |

UO only

| Time prior AKI stage | Cutoff value | sens | spec | Quartile | OR | 95% CI of OR | |
|---|---|---|---|---|---|---|---|
| 0 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |
| 24 hours | 0.4821005 | 77% | 38% | 1 | | | |
| | 0.4079312 | 85% | 30% | 2 | 3.4 | 0.6 | 20.7 |
| | 0.111254 | 92% | 5% | 3 | 1.0 | 0.1 | 10.1 |
| | 1.8446181 | 38% | 70% | 4 | 2.2 | 0.3 | 13.9 |
| | 7.5577446 | 23% | 80% | | | | |
| | 125.72674 | 23% | 90% | | | | |
| 48 hours | 0.7046084 | 100% | 45% | 1 | | | |
| | 0.7046084 | 100% | 45% | 2 | na | na | na |
| | 0.7046084 | 100% | 45% | 3 | na | na | na |
| | 1.8446181 | 0% | 70% | 4 | na | na | na |
| | 7.5577446 | 0% | 80% | | | | |
| | 125.72674 | 0% | 90% | | | | |

# FIG. 6 - 12

**Netrin-1**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 2.013 | 0.913 | 2.013 | 0.913 | 2.013 | 0.913 |
| stdev | 4.612 | 1.719 | 4.612 | 1.719 | 4.612 | 1.719 |
| p (t-test) |  | 0.574 |  | 0.574 |  | 0.574 |
| min | 0.211 | 0.211 | 0.211 | 0.211 | 0.211 | 0.211 |
| max | 20.646 | 4.422 | 20.646 | 4.422 | 20.646 | 4.422 |
| n (Samp) | 24 | 6 | 24 | 6 | 24 | 6 |
| n (Pat) | 24 | 6 | 24 | 6 | 24 | 6 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.211 | na | 0.211 | na | 0.211 | na |
| average | 2.473 | na | 2.473 | na | 2.473 | na |
| stdev | 6.407 | na | 6.407 | na | 6.407 | na |
| p (t-test) |  | na |  | na |  | na |
| min | 0.211 | na | 0.211 | na | 0.211 | na |
| max | 20.646 | na | 20.646 | na | 20.646 | na |
| n (Samp) | 10 | 0 | 10 | 0 | 10 | 0 |
| n (Pat) | 10 | 0 | 10 | 0 | 10 | 0 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 1.425 | 1.053 | 1.425 | 1.053 | 1.425 | 1.053 |
| stdev | 2.755 | 1.883 | 2.755 | 1.883 | 2.755 | 1.883 |
| p (t-test) |  | 0.782 |  | 0.782 |  | 0.782 |
| min | 0.211 | 0.211 | 0.211 | 0.211 | 0.211 | 0.211 |
| max | 8.915 | 4.422 | 8.915 | 4.422 | 8.915 | 4.422 |
| n (Samp) | 17 | 5 | 17 | 5 | 17 | 5 |
| n (Pat) | 17 | 5 | 17 | 5 | 17 | 5 |

sCr or UO

| Time prior AKI stage | AUC | SE | nCohort 1 | nCohort 2 | p |
|---|---|---|---|---|---|
| 0 hours | 0.44 | 0.130 | 24 | 6 | 0.649 |
| 24 hours | 0.44 | 0.130 | 24 | 6 | 0.649 |
| 48 hours | 0.44 | 0.130 | 24 | 6 | 0.649 |

sCr only

| Time prior AKI stage | AUC | SE | nCohort 1 | nCohort 2 | p |
|---|---|---|---|---|---|
| 0 hours | nd | nd | 10 | 0 | nd |
| 24 hours | nd | nd | 10 | 0 | nd |
| 48 hours | nd | nd | 10 | 0 | nd |

UO only

| Time prior AKI stage | AUC | SE | nCohort 1 | nCohort 2 | p |
|---|---|---|---|---|---|
| 0 hours | 0.48 | 0.149 | 17 | 5 | 0.906 |
| 24 hours | 0.48 | 0.149 | 17 | 5 | 0.906 |
| 48 hours | 0.48 | 0.149 | 17 | 5 | 0.906 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 0.0 | 0.0 | na |
|  | 0 | 100% | 0% | 3 | 11.7 | 0.4 | 311.7 |
|  | 0.2106742 | 17% | 71% | 4 | 0.0 | 0.0 | na |
|  | 1.8768769 | 17% | 83% |  |  |  |  |
|  | 8.1073113 | 0% | 92% |  |  |  |  |
| 24 hours | 0 | 100% | 0% | 1 |  |  |  |
|  | 0 | 100% | 0% | 2 | 0.0 | 0.0 | na |
|  | 0 | 100% | 0% | 3 | 11.7 | 0.4 | 311.7 |
|  | 0.2106742 | 17% | 71% | 4 | 0.0 | 0.0 | na |
|  | 1.8768769 | 17% | 83% |  |  |  |  |

## FIG. 7 - 1

| | 8.1073113 | 0% | 92% | | | | |
|---|---|---|---|---|---|---|---|
| 48 hours | 0 | 100% | 0% | 1 | | | |
| | 0 | 100% | 0% | 2 | 0.0 | 0.0 | na |
| | 0 | 100% | 0% | 3 | 11.7 | 0.4 | 311.7 |
| | 0.2106742 | 17% | 71% | 4 | 0.0 | 0.0 | na |
| | 1.8768769 | 17% | 83% | | | | |
| | 8.1073113 | 0% | 92% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.0 | 0.0 | na |
| | 0 | 100% | 0% | 3 | 10.0 | 0.2 | 457.0 |
| | 0.2106742 | 20% | 76% | 4 | 0.0 | 0.0 | na |
| | 1.8768769 | 20% | 82% | | | | |
| | 8.1073113 | 0% | 94% | | | | |
| 24 hours | 0 | 100% | 0% | 1 | | | |
| | 0 | 100% | 0% | 2 | 0.0 | 0.0 | na |
| | 0 | 100% | 0% | 3 | 10.0 | 0.2 | 457.0 |
| | 0.2106742 | 20% | 76% | 4 | 0.0 | 0.0 | na |
| | 1.8768769 | 20% | 82% | | | | |
| | 8.1073113 | 0% | 94% | | | | |
| 48 hours | 0 | 100% | 0% | 1 | | | |
| | 0 | 100% | 0% | 2 | 0.0 | 0.0 | na |
| | 0 | 100% | 0% | 3 | 10.0 | 0.2 | 457.0 |
| | 0.2106742 | 20% | 76% | 4 | 0.0 | 0.0 | na |
| | 1.8768769 | 20% | 82% | | | | |
| | 8.1073113 | 0% | 94% | | | | |

## FIG. 7 - 2

**Growth-regulated alpha protein**

sCr or
UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 330.612 | 122.944 | 330.612 | 97.074 | 330.612 | 74.379 |
| average | 722.768 | 227.094 | 722.768 | 161.749 | 722.768 | 165.744 |
| stdev | 1191.020 | 230.399 | 1191.020 | 211.282 | 1191.020 | 232.872 |
| p (t-test) | | 0.200 | | 0.225 | | 0.265 |
| min | 0.368 | 15.427 | 0.368 | 19.383 | 0.368 | 19.383 |
| max | 4966.418 | 627.680 | 4966.418 | 625.731 | 4966.418 | 625.731 |
| n (Samp) | 37 | 10 | 37 | 7 | 37 | 6 |
| n (Pat) | 37 | 10 | 37 | 7 | 37 | 6 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 306.452 | 97.074 | 306.452 | 97.074 | 306.452 | 60.207 |
| average | 648.994 | 171.434 | 648.994 | 82.168 | 648.994 | 60.207 |
| stdev | 1001.062 | 240.431 | 1001.062 | 41.082 | 1001.062 | 52.139 |
| p (t-test) | | 0.295 | | 0.334 | | 0.413 |
| min | 0.368 | 15.427 | 0.368 | 35.714 | 0.368 | 23.339 |
| max | 4966.418 | 595.238 | 4966.418 | 113.715 | 4966.418 | 97.074 |
| n (Samp) | 60 | 5 | 60 | 3 | 60 | 2 |
| n (Pat) | 60 | 5 | 60 | 3 | 60 | 2 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 332.111 | 177.254 | 332.111 | 120.311 | 332.111 | 114.468 |
| average | 867.224 | 282.754 | 867.224 | 221.434 | 867.224 | 218.513 |
| stdev | 1357.150 | 232.134 | 1357.150 | 277.619 | 1357.150 | 279.889 |
| p (t-test) | | 0.351 | | 0.357 | | 0.355 |
| min | 0.368 | 66.840 | 0.368 | 19.383 | 0.368 | 19.383 |
| max | 4966.418 | 627.680 | 4966.418 | 625.731 | 4966.418 | 625.731 |
| n (Samp) | 27 | 5 | 27 | 4 | 27 | 4 |
| n (Pat) | 27 | 5 | 27 | 4 | 27 | 4 |

sCr or UO

| Time prior AKI stage | AUC | SE | nCohort 1 | nCohort 2 | p |
|---|---|---|---|---|---|
| 0 hours | 0.35 | 0.092 | 37 | 10 | 0.101 |
| 24 hours | 0.26 | 0.090 | 37 | 7 | 0.007 |
| 48 hours | 0.26 | 0.095 | 37 | 6 | 0.010 |

sCr only

| Time prior AKI stage | AUC | SE | nCohort 1 | nCohort 2 | p |
|---|---|---|---|---|---|
| 0 hours | 0.24 | 0.093 | 60 | 5 | 0.006 |
| 24 hours | 0.14 | 0.079 | 60 | 3 | 0.000 |
| 48 hours | 0.11 | 0.079 | 60 | 2 | 0.000 |

UO only

| Time prior AKI stage | AUC | SE | nCohort 1 | nCohort 2 | p |
|---|---|---|---|---|---|
| 0 hours | 0.40 | 0.133 | 27 | 5 | 0.470 |
| 24 hours | 0.31 | 0.130 | 27 | 4 | 0.154 |
| 48 hours | 0.31 | 0.128 | 27 | 4 | 0.128 |

sCr or UO

| Time prior AKI stage | Cutoff value | sens | spec | Quartile | OR | 95% CI of OR | |
|---|---|---|---|---|---|---|---|
| 0 hours | 77.256944 | 70% | 19% | 1 | | | |
| | 53.571429 | 80% | 14% | 2 | 0.5 | 0.0 | 12.5 |
| | 15.427215 | 90% | 5% | 3 | 1.7 | 0.2 | 12.9 |
| | 442.85714 | 20% | 70% | 4 | 2.9 | 0.4 | 20.0 |
| | 677.94486 | 0% | 81% | | | | |
| | 3418.0139 | 0% | 92% | | | | |
| 24 hours | 53.571429 | 71% | 14% | 1 | | | |
| | 19.382911 | 86% | 5% | 2 | 0.0 | 0.0 | na |
| | 3.5601266 | 100% | 5% | 3 | 2.2 | 0.1 | 63.6 |

# FIG. 8 - 1

| | Cutoff value | sens | spec | Quartile | OR | 95% CI of OR | |
|---|---|---|---|---|---|---|---|
| | 442.85714 | 14% | 70% | 4 | 5.7 | 0.3 | 106.6 |
| | 677.94486 | 0% | 81% | | | | |
| | 3418.0139 | 0% | 92% | | | | |
| 48 hours | 19.382911 | 83% | 5% | 1 | | | |
| | 19.382911 | 83% | 5% | 2 | 0.0 | 0.0 | na |
| | 3.5601266 | 100% | 5% | 3 | 2.2 | 0.1 | 63.6 |
| | 442.85714 | 17% | 70% | 4 | 4.3 | 0.2 | 94.1 |
| | 677.94486 | 0% | 81% | | | | |
| | 3418.0139 | 0% | 92% | | | | |

sCr only

| Time prior AKI stage | Cutoff value | sens | spec | Quartile | OR | 95% CI of OR | |
|---|---|---|---|---|---|---|---|
| 0 hours | 23.338608 | 80% | 7% | 1 | | | |
| | 23.338608 | 80% | 7% | 2 | 0.0 | 0.0 | na |
| | 3.5601266 | 100% | 3% | 3 | 0.0 | 0.0 | na |
| | 477.55102 | 20% | 70% | 4 | 5.3 | 0.3 | 82.3 |
| | 677.94486 | 0% | 80% | | | | |
| | 1787.5289 | 0% | 90% | | | | |
| 24 hours | 23.338608 | 100% | 7% | 1 | | | |
| | 23.338608 | 100% | 7% | 2 | na | na | na |
| | 23.338608 | 100% | 7% | 3 | na | na | na |
| | 477.55102 | 0% | 70% | 4 | na | na | na |
| | 677.94486 | 0% | 80% | | | | |
| | 1787.5289 | 0% | 90% | | | | |
| 48 hours | 19.382911 | 100% | 5% | 1 | | | |
| | 19.382911 | 100% | 5% | 2 | na | na | na |
| | 19.382911 | 100% | 5% | 3 | na | na | na |
| | 477.55102 | 0% | 70% | 4 | na | na | na |
| | 677.94486 | 0% | 80% | | | | |
| | 1787.5289 | 0% | 90% | | | | |

UO only

| Time prior AKI stage | Cutoff value | sens | spec | Quartile | OR | 95% CI of OR | |
|---|---|---|---|---|---|---|---|
| 0 hours | 130.30303 | 80% | 19% | 1 | | | |
| | 130.30303 | 80% | 19% | 2 | 1.0 | 0.0 | 88.2 |
| | 53.571429 | 100% | 11% | 3 | 1.0 | 0.0 | 88.2 |
| | 447.94721 | 20% | 70% | 4 | 2.3 | 0.1 | 81.0 |
| | 647.86967 | 0% | 81% | | | | |
| | 3464.2032 | 0% | 93% | | | | |
| 24 hours | 53.571429 | 75% | 11% | 1 | | | |
| | 0.3677885 | 100% | 4% | 2 | 0.0 | 0.0 | na |
| | 0.3677885 | 100% | 4% | 3 | 1.0 | 0.0 | 88.2 |
| | 447.94721 | 25% | 70% | 4 | 2.8 | 0.1 | 103.7 |
| | 647.86967 | 0% | 81% | | | | |
| | 3464.2032 | 0% | 93% | | | | |
| 48 hours | 39.663462 | 75% | 7% | 1 | | | |
| | 0.3677885 | 100% | 4% | 2 | 0.0 | 0.0 | na |
| | 0.3677885 | 100% | 4% | 3 | 1.0 | 0.0 | 88.2 |
| | 447.94721 | 25% | 70% | 4 | 2.8 | 0.1 | 103.7 |
| | 647.86967 | 0% | 81% | | | | |
| | 3464.2032 | 0% | 93% | | | | |

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 23.154 | 8.582 | 23.154 | 3.724 | 23.154 | 3.724 |
| average | 36.931 | 11.770 | 36.931 | 5.725 | 36.931 | 5.644 |
| stdev | 35.835 | 9.784 | 35.835 | 3.815 | 35.835 | 3.579 |
| p (t-test) | | 0.034 | | 0.028 | | 0.040 |
| min | 0.036 | 1.359 | 0.036 | 1.359 | 0.036 | 2.717 |
| max | 166.667 | 34.091 | 166.667 | 11.820 | 166.667 | 11.820 |

## FIG. 8 - 2

|          |    |    |    |   |    |   |
|----------|----|----|----|---|----|---|
| n (Samp) | 37 | 10 | 37 | 7 | 37 | 6 |
| n (Pat)  | 37 | 10 | 37 | 7 | 37 | 6 |

sCr only

|           | 0 hr prior toAKI stage | | 24 hr prior toAKI stage | | 48 hr prior toAKI stage | |
|-----------|----------|----------|----------|----------|----------|----------|
|           | Cohort 1 | Cohort 2 | Cohort 1 | Cohort 2 | Cohort 1 | Cohort 2 |
| median    | 21.739   | 8.582    | 21.739   | 8.582    | 21.739   | 7.772    |
| average   | 33.191   | 7.137    | 33.191   | 7.253    | 33.191   | 7.772    |
| stdev     | 32.494   | 3.959    | 32.494   | 5.356    | 32.494   | 5.725    |
| p (t-test)|          | 0.080    |          | 0.175    |          | 0.277    |
| min       | 0.036    | 1.359    | 0.036    | 1.359    | 0.036    | 3.724    |
| max       | 166.667  | 11.820   | 166.667  | 11.820   | 166.667  | 11.820   |
| n (Samp)  | 61       | 5        | 61       | 3        | 61       | 2        |
| n (Pat)   | 61       | 5        | 61       | 3        | 61       | 2        |

UO only

|           | 0 hr prior toAKI stage | | 24 hr prior toAKI stage | | 48 hr prior toAKI stage | |
|-----------|----------|----------|----------|----------|----------|----------|
|           | Cohort 1 | Cohort 2 | Cohort 1 | Cohort 2 | Cohort 1 | Cohort 2 |
| median    | 24.457   | 16.677   | 24.457   | 3.724    | 24.457   | 3.724    |
| average   | 41.340   | 16.404   | 41.340   | 4.579    | 41.340   | 4.579    |
| stdev     | 38.205   | 12.084   | 38.205   | 2.429    | 38.205   | 2.429    |
| p (t-test)|          | 0.163    |          | 0.068    |          | 0.068    |
| min       | 1.359    | 2.717    | 1.359    | 2.717    | 1.359    | 2.717    |
| max       | 166.667  | 34.091   | 166.667  | 8.152    | 166.667  | 8.152    |
| n (Samp)  | 27       | 5        | 27       | 4        | 27       | 4        |
| n (Pat)   | 27       | 5        | 27       | 4        | 27       | 4        |

sCr or UO

| Time prior AKI stage | AUC  | SE    | nCohort 1 | nCohort 2 | p     |
|----------------------|------|-------|-----------|-----------|-------|
| 0 hours              | 0.22 | 0.073 | 37        | 10        | 0.000 |
| 24 hours             | 0.13 | 0.058 | 37        | 7         | 0.000 |
| 48 hours             | 0.13 | 0.063 | 37        | 6         | 0.000 |

sCr only

| Time prior AKI stage | AUC  | SE    | nCohort 1 | nCohort 2 | p     |
|----------------------|------|-------|-----------|-----------|-------|
| 0 hours              | 0.17 | 0.072 | 61        | 5         | 0.000 |
| 24 hours             | 0.17 | 0.090 | 61        | 3         | 0.000 |
| 48 hours             | 0.18 | 0.115 | 61        | 2         | 0.006 |

UO only

| Time prior AKI stage | AUC  | SE    | nCohort 1 | nCohort 2 | p     |
|----------------------|------|-------|-----------|-----------|-------|
| 0 hours              | 0.25 | 0.105 | 27        | 5         | 0.018 |
| 24 hours             | 0.05 | 0.038 | 27        | 4         | 0.000 |
| 48 hours             | 0.05 | 0.038 | 27        | 4         | 0.000 |

**Tubulointerstitial nephritis antigen**

sCr or UO

|           | 0 hr prior to AKI stage | | 24 hr prior to AKI stage | | 48 hr prior to AKI stage | |
|-----------|----------|----------|----------|----------|----------|----------|
|           | Cohort 1 | Cohort 2 | Cohort 1 | Cohort 2 | Cohort 1 | Cohort 2 |
| median    | 0.532    | 0.618    | 0.532    | 0.618    | 0.532    | 0.618    |
| average   | 49.191   | 1.514    | 49.191   | 1.514    | 49.191   | 0.649    |
| stdev     | 117.958  | 2.240    | 117.958  | 2.240    | 117.958  | 0.447    |
| p (t-test)|          | 0.337    |          | 0.337    |          | 0.425    |
| min       | 0.087    | 0.136    | 0.087    | 0.136    | 0.087    | 0.136    |
| max       | 408.188  | 6.029    | 408.188  | 6.029    | 408.188  | 1.224    |
| n (Samp)  | 25       | 6        | 25       | 6        | 25       | 4        |
| n (Pat)   | 25       | 6        | 25       | 6        | 25       | 4        |

sCr only

|           | 0 hr prior toAKI stage | | 24 hr prior toAKI stage | | 48 hr prior toAKI stage | |
|-----------|----------|----------|----------|----------|----------|----------|
|           | Cohort 1 | Cohort 2 | Cohort 1 | Cohort 2 | Cohort 1 | Cohort 2 |
| median    | 0.754    | 0.457    | 0.754    | 0.457    | 0.754    | 0.358    |
| average   | 60.042   | 0.391    | 60.042   | 0.391    | 60.042   | 0.358    |
| stdev     | 131.942  | 0.230    | 131.942  | 0.230    | 131.942  | 0.315    |
| p (t-test)|          | 0.442    |          | 0.442    |          | 0.530    |
| min       | 0.087    | 0.136    | 0.087    | 0.136    | 0.087    | 0.136    |
| max       | 411.095  | 0.581    | 411.095  | 0.581    | 411.095  | 0.581    |
| n (Samp)  | 44       | 3        | 44       | 3        | 44       | 2        |

# FIG. 8 - 3

| n (Pat) | 44 | 3 | 44 | 3 | 44 | 2 |
|---|---|---|---|---|---|---|

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.804 | 0.939 | 0.804 | 0.939 | 0.804 | 0.655 |
| average | 49.793 | 2.122 | 49.793 | 2.122 | 49.793 | 0.820 |
| stdev | 118.251 | 2.620 | 118.251 | 2.620 | 118.251 | 0.352 |
| p (t-test) | | 0.434 | | 0.434 | | 0.487 |
| min | 0.087 | 0.581 | 0.087 | 0.581 | 0.087 | 0.581 |
| max | 408.188 | 6.029 | 408.188 | 6.029 | 408.188 | 1.224 |
| n (Samp) | 25 | 4 | 25 | 4 | 25 | 3 |
| n (Pat) | 25 | 4 | 25 | 4 | 25 | 3 |

sCr or UO

| Time prior AKI stage | AUC | SE | nCohort 1 | nCohort 2 | p |
|---|---|---|---|---|---|
| 0 hours | 0.49 | 0.133 | 25 | 6 | 0.940 |
| 24 hours | 0.49 | 0.133 | 25 | 6 | 0.940 |
| 48 hours | 0.44 | 0.152 | 25 | 4 | 0.694 |

sCr only

| Time prior AKI stage | AUC | SE | nCohort 1 | nCohort 2 | p |
|---|---|---|---|---|---|
| 0 hours | 0.31 | 0.139 | 44 | 3 | 0.163 |
| 24 hours | 0.31 | 0.139 | 44 | 3 | 0.163 |
| 48 hours | 0.27 | 0.155 | 44 | 2 | 0.142 |

UO only

| Time prior AKI stage | AUC | SE | nCohort 1 | nCohort 2 | p |
|---|---|---|---|---|---|
| 0 hours | 0.57 | 0.161 | 25 | 4 | 0.664 |
| 24 hours | 0.57 | 0.161 | 25 | 4 | 0.664 |
| 48 hours | 0.52 | 0.181 | 25 | 3 | 0.912 |

sCr or UO

| Time prior AKI stage | Cutoff value | sens | spec | Quartile | OR | 95% CI of OR | |
|---|---|---|---|---|---|---|---|
| 0 hours | 0.4079312 | 83% | 40% | 1 | | | |
| | 0.4079312 | 83% | 40% | 2 | 4.2 | 0.2 | 112.2 |
| | 0.0865309 | 100% | 4% | 3 | 1.0 | 0.0 | 88.2 |
| | 1.9259983 | 17% | 72% | 4 | 1.2 | 0.0 | 107.9 |
| | 22.125912 | 0% | 80% | | | | |
| | 209.06008 | 0% | 92% | | | | |
| 24 hours | 0.4079312 | 83% | 40% | 1 | | | |
| | 0.4079312 | 83% | 40% | 2 | 4.2 | 0.2 | 112.2 |
| | 0.0865309 | 100% | 4% | 3 | 1.0 | 0.0 | 88.2 |
| | 1.9259983 | 17% | 72% | 4 | 1.2 | 0.0 | 107.9 |
| | 22.125912 | 0% | 80% | | | | |
| | 209.06008 | 0% | 92% | | | | |
| 48 hours | 0.5315467 | 75% | 52% | 1 | | | |
| | 0.0865309 | 100% | 4% | 2 | na | na | na |
| | 0.0865309 | 100% | 4% | 3 | na | na | na |
| | 1.9259983 | 0% | 72% | 4 | na | na | na |
| | 22.125912 | 0% | 80% | | | | |
| | 209.06008 | 0% | 92% | | | | |

# FIG. 8 - 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20080090304 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- **ABD El LATIF M M S.** *Journal of Medical Sciences,* vol. 7 (7), 1171-1176
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- Merck Manual of Diagnosis and Therapy. Merck Research Laboratories, 1999
- **WIJEYSUNDERA et al.** *JAMA,* 2007, vol. 297, 1801-9
- **HANLEY, J. A. ; MCNEIL, B.J.** The meaning and use of the area under a receiver operating characteristic (ROC) curve. *Radiology,* 1982, vol. 143, 29-36